# EUROPEAN PATENT SPECIFICATION

(11) **EP 3 535 409 B1**
(45) Date of publication and mention of the grant of the patent: **20.03.2024**
(21) Application number: 17805333.6
(22) Date of filing: 03.11.2017
(51) Int. Cl.: C12Q 1/04, G01N 33/569

(54) **DIRECT DETECTION OF MICROORGANISMS IN PATIENT SAMPLES BY IMMUNOASSAY**
DIREKTE DETEKTION VON MIKROORGANISMEN IN PATIENTENPROBEN DURCH IMMUNTEST
DÉTECTION DIRECTE DE MICRO-ORGANISMES DANS DES ÉCHANTILLONS DE PATIENT PAR DOSAGE IMMUNOLOGIQUE

(30) Priority: 04.11.2016 US 201662417559 P; 04.11.2016 US 201662417778 P
(43) Date of publication of application: 11.09.2019
(73) Proprietor: CD Diagnostics, Inc., Claymont, Delaware 19703 (US)
(72) Inventor: STAVE, James W., Bear, Delaware 19701 (US); CARPENTER, Megan, Claymont, Delaware 19703 (US); ARNONE, Marc, Claymont, Delaware 19703 (US); JOAQUIM, Tony, Mount Laurel, New Jersey 08054 (US); GOULD, Martin Raymond, Mullica Hill, New Jersey 08062 (US); MIAMIDIAN, John Leon, Philadelphia, Pennsylvania 19129 (US)
(74) Representative: Handley, Matthew Edward
(86) International application number: PCT/US2017/059966
(87) International publication number: WO 2018/085679

(56) References cited:
- EP-A1- 3 085 769
- EP-A1- 3 085 770
- EP-A2- 0 286 434
- US-A1- 2006 134 729
- US-A1- 2015 011 412

## Description

### CLAIM OF PRIORITY

This application claims the benefit of U.S. Provisional Patent Application Serial No. 62/417,559, and U.S. Provisional Patent Application Serial No. 62/417,778, each filed on November 4.

### TECHNICAL FIELD

The document pertains generally, but not by way of limitation, to medicine and more particularly to direct detection of microorganisms in a patient tissue sample

### BACKGROUND

This section provides background information related to the present disclosure, but such background information is not admitted to be prior art.

Joint infection, such as native j oint infection and periprosthetic joint infection ("PJ1"), sepsis, bacterial meningitis, and other forms of microbial (e.g., bacterial, fungal, viral, protozoal) infection can be debilitating and difficult to diagnose It is important to diagnose microbial infection and determine the identity of the infecting microorganism in order to inform treatment, which can include selection of an appropriate pharmacological intervention for the specific microorganism causing the infection. The classic means or "gold standard" for determining whether a microbial infection is present in a patient tissue or fluid sample, is to culture the sample of tissue and observe the culture for the growth of microorganisms. However, culture methods have long time-to-results (requiring several days to weeks for culture growth), and also have high false negative rates because not all infected samples will be evidenced by culture growth, e.g.. only approximately 50% of actual PJI infections are evidenced in sample culture. This inability to directly detect microbes in patient tissue samples has left physicians without means for routine diagnosis of microbial infections, including PJI. Therefore, physicians often must rely on indirect criteria to diagnose microbial infection For example, in 2011, the Musculoskeletal Infection Society ("MSIS") proposed the following clinical criteria on which to base a diagnosis of PJI (see Parvizi, j. and Gehrke. T. (2014) Definition of Periprosthetic Infection. J. Arthroplasty 29:133 1).

International Consensus Group Adaptation of the Musculoskeletal Infection Society Definition of PJI:

| | |
|---|---|
| Major Criteria | Two positive periprosthetic cultures with phenotypically identical organisms, OR |
| | A sinus tract communicating with the joint OR |
| Minor Criteria | 1) Elevated serum C-reactive protein (CRP) AND erythrocyte sedimentation rate (ESR) |
| | 2) Elevated synovial fluid white blood cell (WBC) count OR ++ change on leukocyte esterase test strip |
| | 3) Elevated synovial fluid polymorphonuclear neutrophil percentage (PMN%) |
| | 4) Positive histological analysis of periprosthetic tissue |
| | 5) A single positive culture |

In the absence of Major Criteria for PJI. an infection is considered if the sample meets at least three of the Minor Criteria. A limitation of this indirect approach to diagnosis of microbial infection is that a number of the MSIS criteria rely on results only obtained after surgery.

Immunoassay and other binding assays, polymerase chain reaction ("PCR"), enzyme activity assays, and instrument-based methods such as mass spectrometry have been evaluated for the ability to detect microorganisms in patient samples. These methods are generally coupled with a wide variety of sample preparation techniques that may separate, purify or concentrate the bacterial antigen that is the target of the test method The sample preparation protocol is a critical component of the overall method and frequently makes the difference between success and failure. To date, researchers have had limited success in developing rapid methods that directly detect the presence of microorganisms in patient tissue samples with improved sensitivity, including immunoassay, PCR, and mass spectrometry. The current methods of direct detection of microorganisms in patient tissue samples by immunoassay and PCR frequently yield false negative results, and have poor clinical sensitivity and specificity, rendering them unsuitable for use in diagnosing infection.

Recently, a test has been developed to measure the concentration of human alpha defensin ("α-defensin" or "AD"), in synovial fluid samples as a biomarker of native joint infection or PJI. Neutrophils are a type of white blood cell that represent a first line of defence against invading microorganisms. Neutrophils secrete AD, which binds to bacterial cells and directly kills them. Elevated AD concentration is a biomarker of infection and has been shown to have excellent agreement (98% sensitivity. 98% specificity) with the diagnosis of infection using the MSIS criteria. While AD as a biomarker of infection is rapidly gaining acceptance in the medical community, approximately 50% of all AD positive samples are culture negative. The validation of AD versus the MSIS criteria and the preponderance of data from third party clinical studies confirm the accuracy of the diagnosis of PJI by AD (i.e., a sample that is AD positive indicates infection). However, many physicians, who are unaware of the poor sensitivity of culture methods and/or the excellent performance of AD, may view an AD positive, culture negative result as a false positive. A method to directly detect bacteria in patient synovial fluid is needed to increase the positive predictive value of the diagnosis when AD is positive and no infectious microorganism has been recovered using culture-based methods. In addition, a bacterial detection method that provides information regarding the type or identity of the infecting microorganism has further value to inform antibiotic treatment. A method for detecting bacteria and fungi in a sample is described in EP 0 286 434.

A method to directly detect microorganisms, e.g , bacteria, virus, fungi, in patient samples, including in synovial fluid samples, is needed to increase the positive predictive value of the infection diagnosis from the AD test, particularly when infectious microorganisms have not been recovered using culture-based methods. In addition, it would be useful for a microorganism detection method that provides information regarding the type or identity of the infecting microorganism to better inform the decision on therapeutic and pharmacological treatment. Furthermore, there is a need for a routine, rapid, and cost-effective means for diagnosing infection, in particular in native joint infection and PJI. in a way that informs treatment prior to surgery or other therapeutic intervention.

The present invention provides biological sample preparation methods and immunoassay methods using the prepared biological samples, and compositions and systems that enable direct detection and identification of infecting microorganisms in biological samples, including in patient samples that initially test as culture-negative. The sample preparation and immunoassay techniques of the present patent document enable the use of rapid immunoassay methods for detecting and identifying microorganisms and diagnosing infection with greater sensitivity and in a shorter period of time than the existing "gold-standard" culture methods. The unique combination of reagents and methods of the present invention enables direct detection of microorganisms in biological samples, including synovial fluid, from patients that have previously tested culture-negative. Diagnosis of infection in culture-negative biological samples has not been previously described and, therefore, the methods, compositions and systems of the present patent document represent a significant breakthrough in test sensitivity and diagnostic capability

### OVERVIEW

The claimed invention is defined in the attached independent claims and preferred embodiments are set out in the sub-claims. This overview is intended to provide an overview of subject matter of the present patent application. It is not intended to provide an exclusive or exhaustive explanation of the invention or its full scope or all of its features. The detailed description is included to provide further information about the present patent application.

Developing an effective test to detect native joint infection and periprosthetic joint infection ("PJI") begins by understanding which microorganisms are responsible for infection. The present inventors have isolated microorganisms from 1060 synovial fluid samples that contained over a hundred different species of bacteria. Eighty-six percent of the samples contained Gram-positive bacteria, 10 percent contained Gram-negative bacteria, and the remainder contained fungi (overwhelmingly *Candida* species). Two different genera of bacteria, *Staphylococcus* and *Streptococcus,* accounted for three-quarters of all organisms isolated, and two species of bacteria, *Staphylococcus epidermidis* and *Staphylococcus aureus,* accounted for half of all the organisms isolated. From this data it is clear that the preponderance of samples contain Gram-positive organisms, especially *Staphylococcus* and *Streptococcus.*

The "gold standard" method for determining the presence of infection is to incubate a sample of tissue in culture media for a period of time under conditions that enable replication of the infectious agent to an extent at which growth can be observed. The large number of different infecting organisms and the limited culture conditions contribute to the high failure rate of culture for diagnosing native joint infection and PJI (i.e., false negative culture results).

Another factor contributing to the high failure rate of culture is that bacteria present in a sample may be damaged to an extent that they do not readily grow. It is well established that antibiotic therapy can lead to culture failure and false negative results due to damaged bacteria. Similarly, antimicrobial substances produced by the patient's immune system during the course of infection, like α-defensin, can damage bacteria in a way that could prevent them from growing in culture, causing false negative culture results.

The present inventors have recognized, among other things, that a problem to be solved can include the frequently false negative results from current methods of detection of microorganisms in patient samples. e.g.. by sample culture, and direct detection by immunoassay, and PCR, and the poor clinical sensitivity and specificity of such methods, rendering them unsuitable for use in diagnosing native joint infection and PJI. The present subject matter can help provide a solution to this problem, such as by treating a patient sample with a combination of enzymes, detergents and heat to increase the amount of microorganism antigen available for detection and identification by immunoassay of the patient sample

### DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document The drawings described herein are for illustrative purposes only of selected examples and not all possible examples or implementations, and these drawings are not intended to limit the scope of the present disclosure.

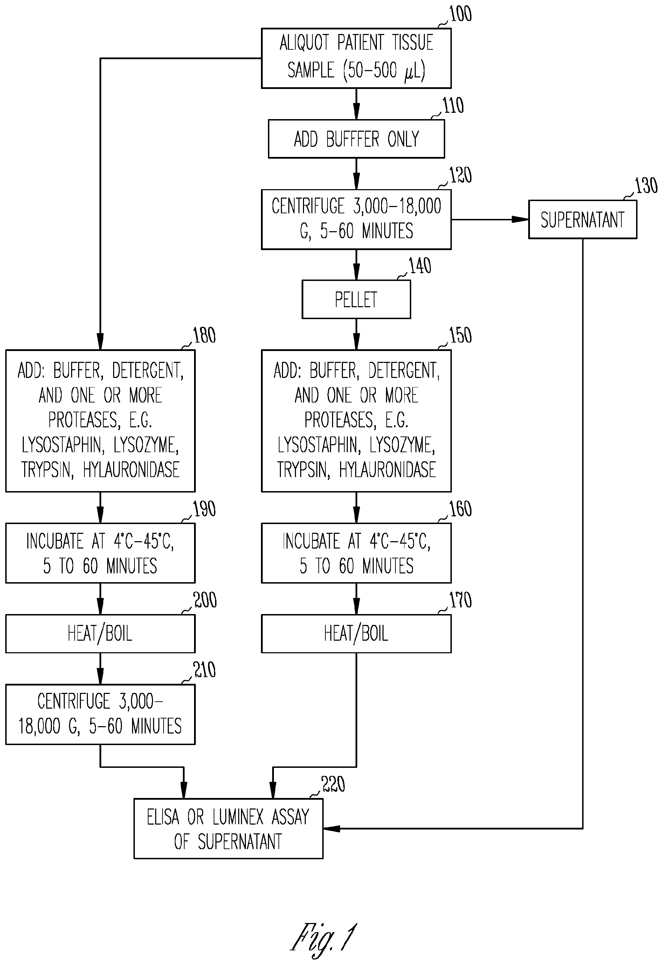

**FIG. 1** illustrates methods of preparing a patient biological sample for use in immunoassays for the direct detection and identification of microorganisms in a patient biological sample.

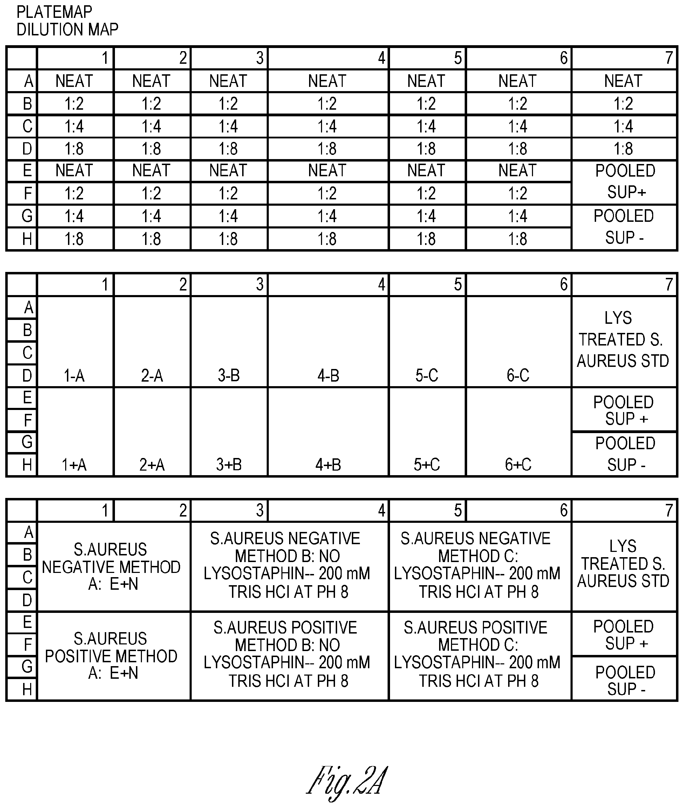

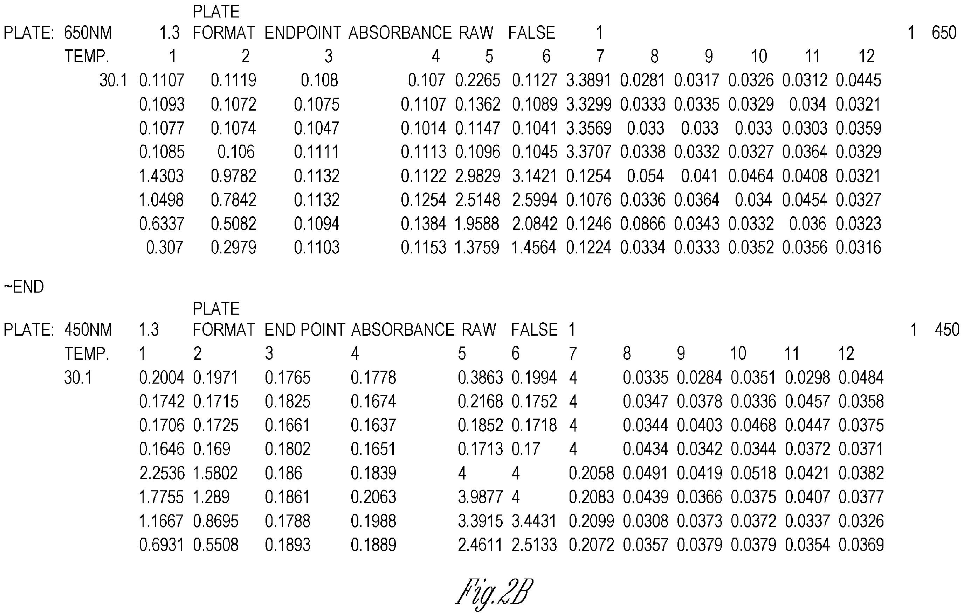

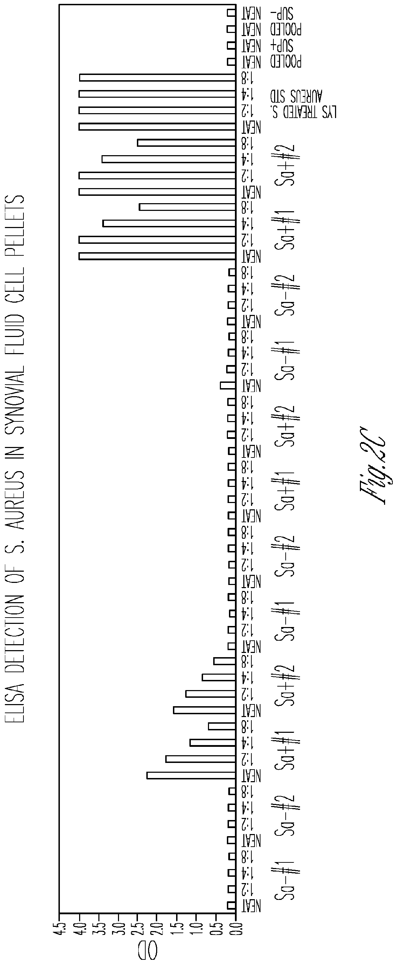

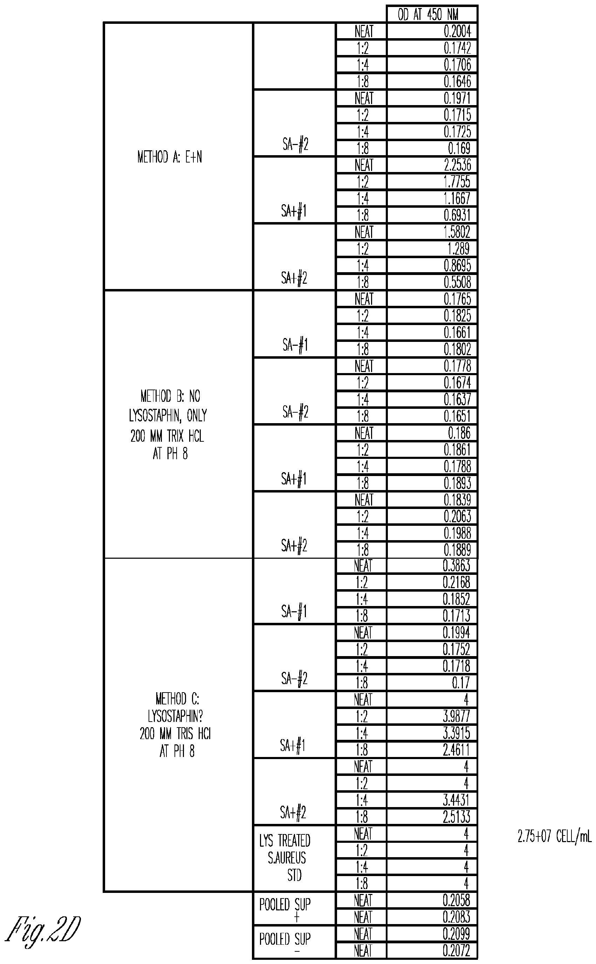

FIGS. 2A-2D illustrate data of an ELISA microorganism detection and identification immunoassays ("MID immunoassay(s)") to directly detect *Staphylococcus* species of bacteria in patient synovial fluid samples prepared by an example of the method according to the present patent document **FIG. 2A** is a plate map and dilution map for the MID immunoassay. **FIG. 2B** is a table of the results of the immunoassay. **FIG. 2C** is a bar graph of the results of the immunoassay. **FIG. 2D** is a table of the results of the immunoassay

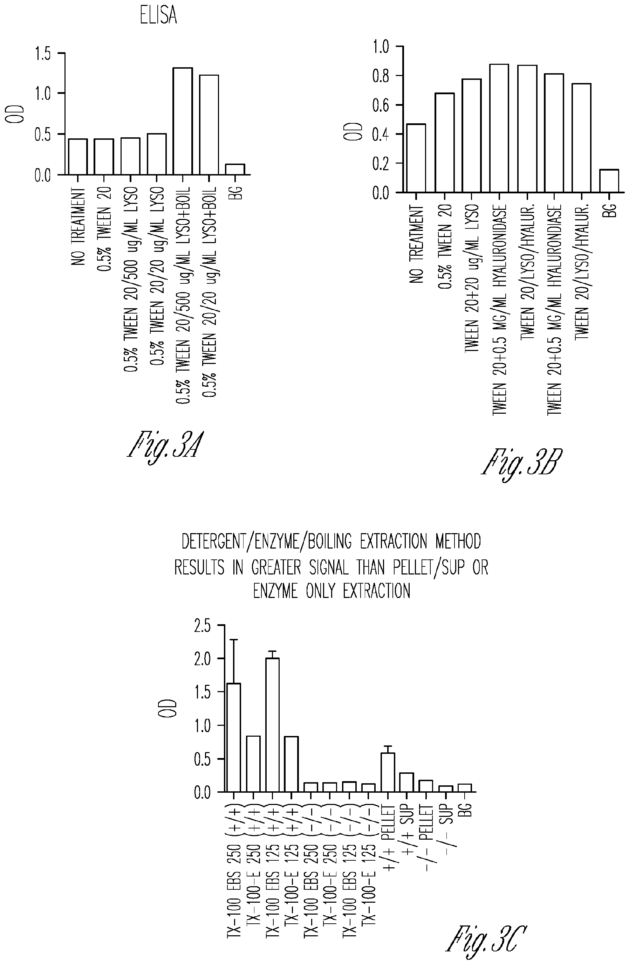

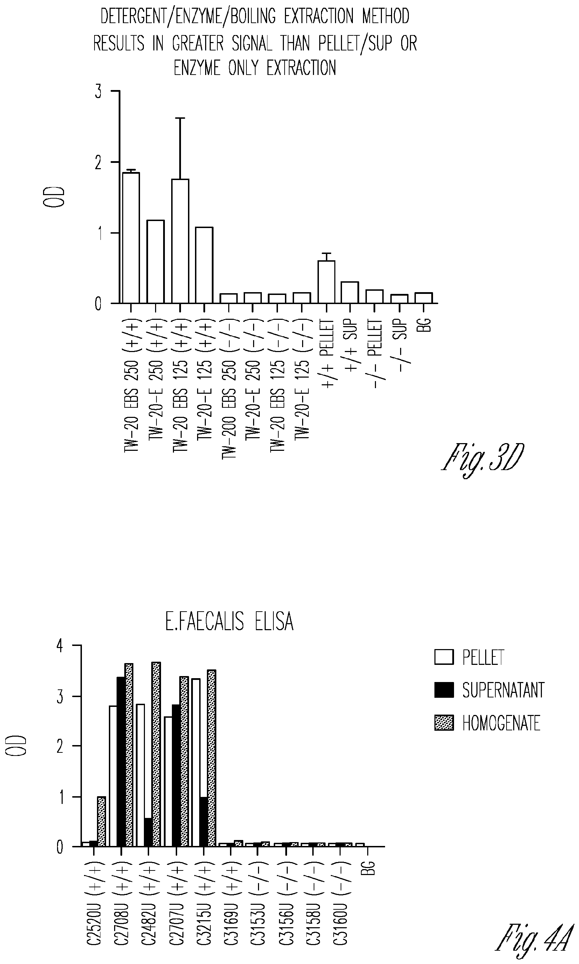

**FIGS. 3A-3D** illustrate bar graphs of the results of a MID immunoassay to directly detect *Staphylococcus* species of bacteria in patient synovial fluid samples prepared by various methods according to the present patent document. **FIG. 3A** illustrates direct detection of *Staphylococcus* species in synovial fluid samples prepared using various concentrations of lysostaphin and detergent (Tween 20), boiling for 5 minutes, and centrifugation at 18,000 g for 10 minutes (as more fully described in Experimental Example 2). **FIG. 3B** illustrates direct detection of *Staphylococcus* species in synovial fluid samples prepared using various concentrations of lysostaphin, hyaluronidase and detergent (Tween 20), boiling for 5 minutes, and centrifugation at 18,000 g for 10 minutes. **FIG. 3C** illustrates direct detection of *Staphylococcus* species in synovial fluid samples comparing sample preparation methods of Example 1 and Example 2, using detergent (Triton-X 100). **FIG. 3D** illustrates direct detection of *Staphylococcus* species in synovial fluid samples comparing sample preparation methods of Example 1 and Example 2, using detergent (Tween 20). EBS = samples prepared using enzymes (20 µg/mL lysostaphin and 1 mg/mL hyaluronidase), with boiling for 5 minutes, and centrifugation at 18,000 g for 10 minutes obtain supernatant; E = lysostaphin and hyaluronidase only; TX = 0.5% Triton-X 100; TW = 0.5% Tween 20, pellet = pellet from the original sample preparation method (Experimental Example 1); sup = supernatant from the original sample preparation method (Experimental Example 1), 125 or 250 = sample extraction volume (µL), 1:2 dilution, BG = background. Detergent/enzyme/boiling extraction results in greater signal than pellets/sup or enzyme only extraction

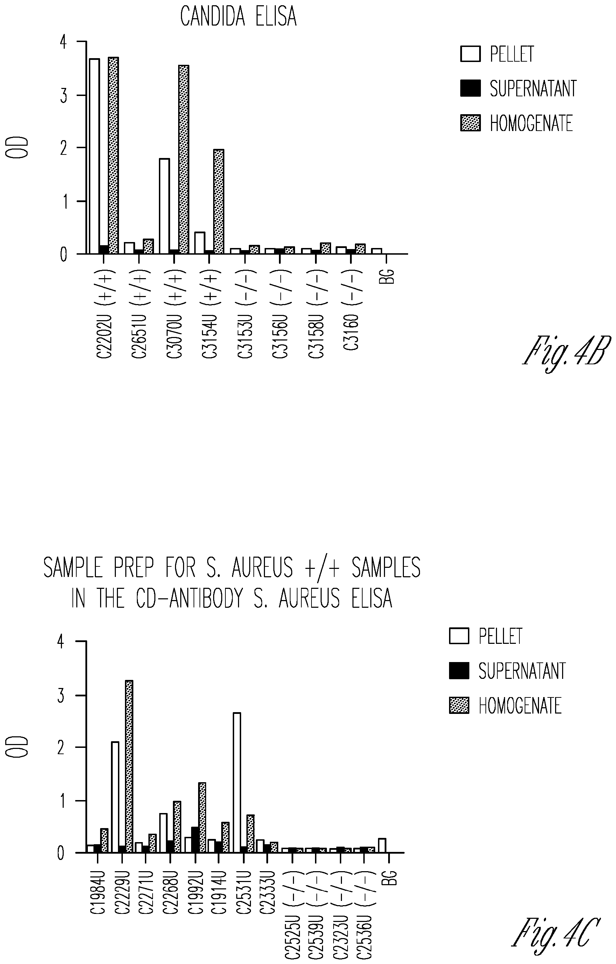

**FIGS. 4A** - **4C** illustrate bar graphs of MID (ELIS A) immunoassays detecting microorganisms in synovial fluid samples prepared according to the methods of Experimental Example 1 (pellet/supernatant) and Experimental Example 2 (homogenate). **FIG. 4A** illustrates MID immunoassays detecting *Enterococcus faecalis.* **FIG. 4B** illustrates MID immunoassays detecting *Candida albicans* **FIG. 4C** illustrates MID immunoassays detecting *Staphylococcus aureus* Samples C1984U, C2229U, C2268U, C194 U, C2531U and C2333U testing α-defensin positive and culture positive (+/+). The remainder of the samples testing α-defensin negative and culture negative (-/-).

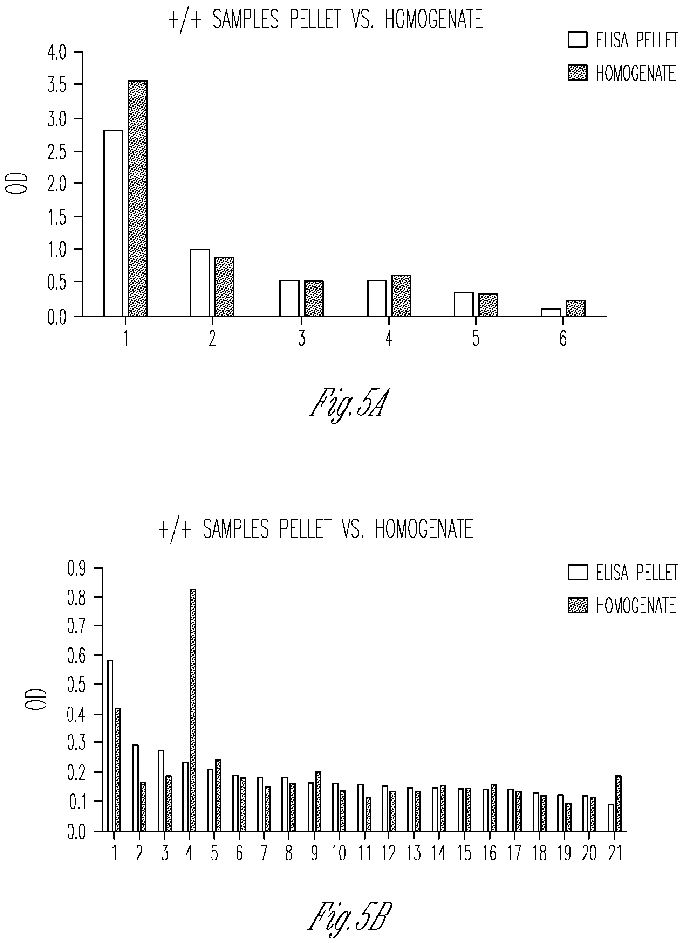

**FIGS. 5A-5B** illustrate bar graphs of MID (ELISA) immunoassays detecting *Staphylococcus epidermidis* in synovial fluid samples prepared according to Experimental Example 1 (pellet/sup) or Experimental Example 2 (homogenate) **FIG. 5A** illustrates samples testing α-defensin positive and culture positive (+/+). **FIG. 5B** illustrates samples testing α-defensin positive and culture negative (+/-) These results indicate the Experimental Example 2 sample preparation method is minimally similar to the pellet/sup method of Experimental Example 1

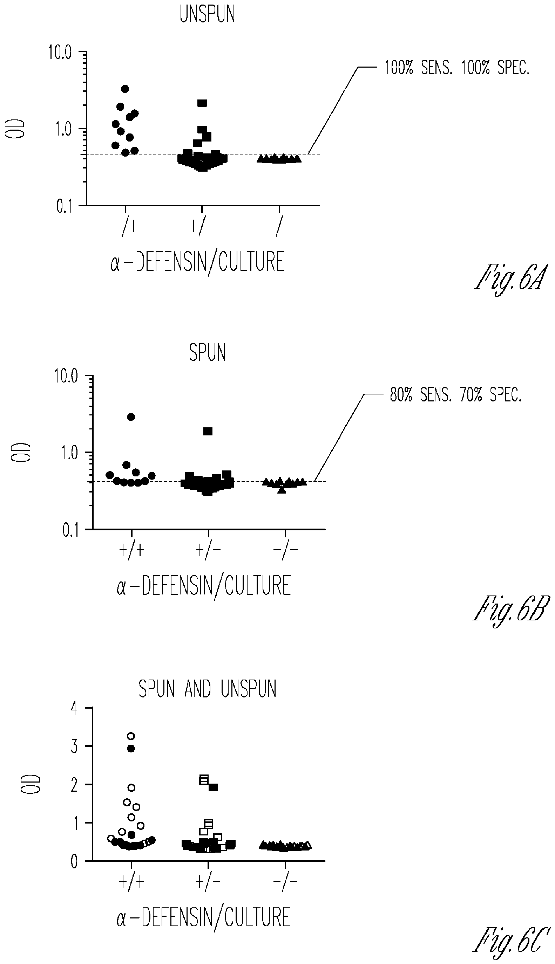

**FIGS. 6A-6C** illustrate dot plots of MID (ELISA) immunoassays detecting *Staphylococcus* species in synovial fluid samples testing α-defensin positive and culture positive (+/+), α-defensin positive and culture negative (+/-), or α-defensin negative and culture negative (-/-). **FIG. 6A** illustrates synovial fluid samples prepared according to Experimental Example 1 Of the (+/-) 6148, or 12.5% are above the cut-off for sensitivity/specificity **FIG. 6B** illustrates synovial fluid samples prepared according to Experimental Example 1 with an initial centrifugation at <3,000 g before processing according to Experimental Example 1. Of the (+/-) samples, 6/48 or 12.5% are above the cut-off for sensitivity/specificity. **FIG. 6C** illustrates the combined results of **FIGS. 6A** and **6B****.** Unspun samples result in higher (+/+) and (+/-) signals, and sensitivity and specificity are improved in unspun samples

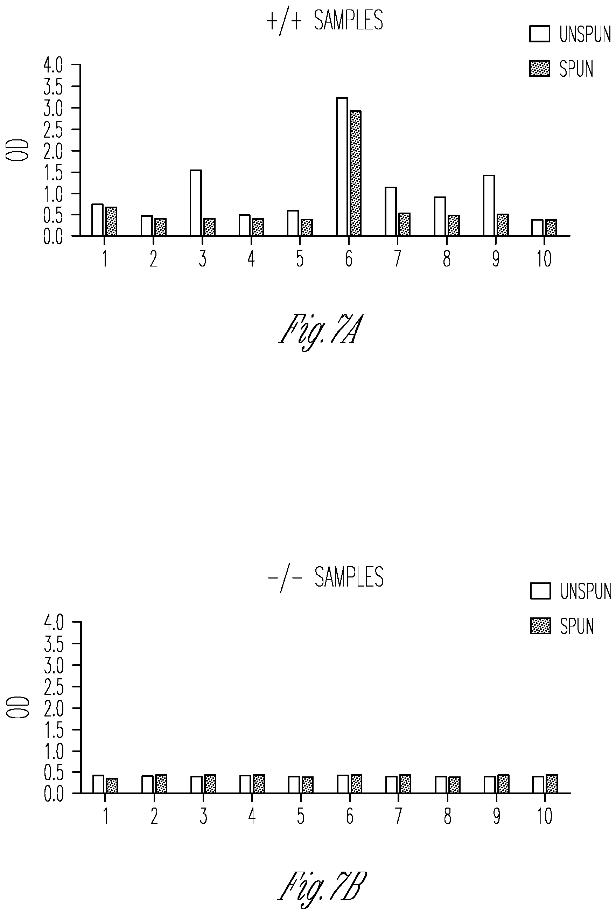

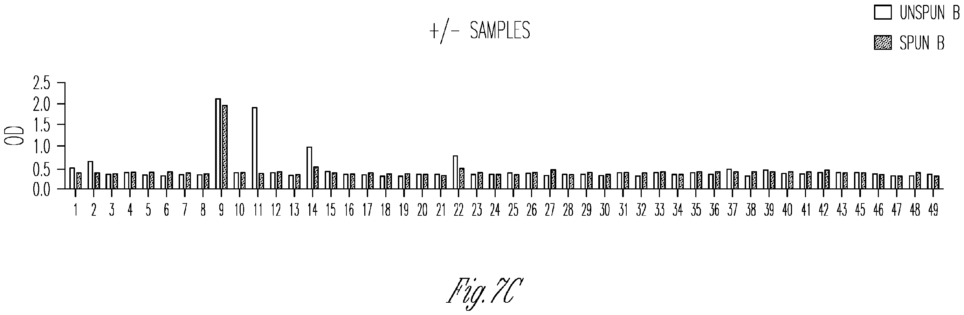

**FIGS. 7A-7C** illustrate bar graphs of ELISA MID immunoassays detecting *Staphylococcus* species in synovial fluid samples prepared as described above for **FIGS. 6A-6C** **FIG. 7A** illustrates synovial fluid samples testing α-defensin positive and culture positive (+/+). **FIG. 7B** illustrates synovial fluid samples testing α-defensin negative and culture negative (-/-). **FIG. 7C** illustrates synovial fluid samples testing α-defensin positive and culture negative (+/-).

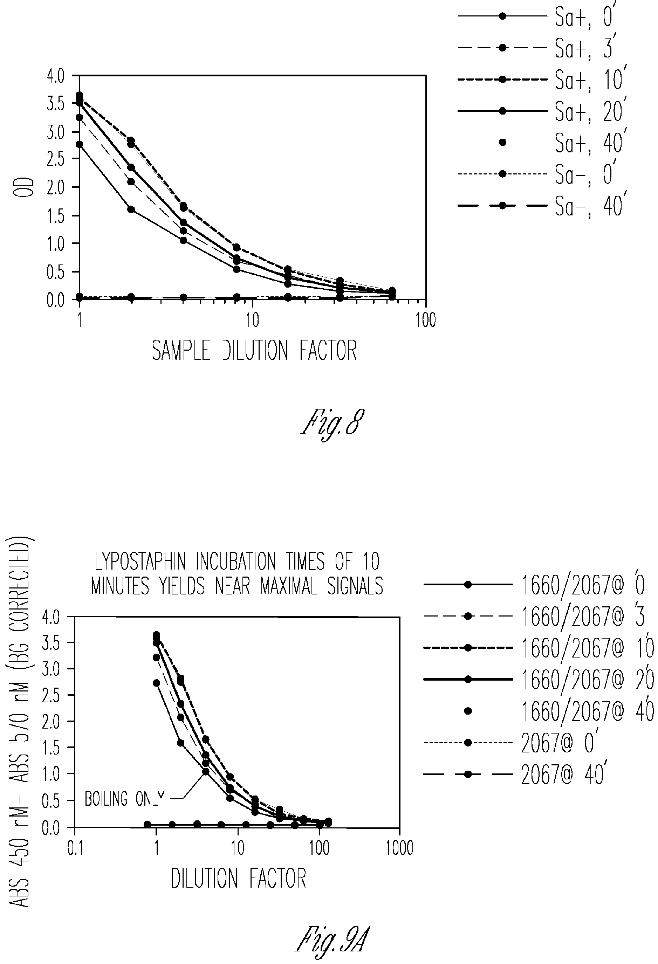

**FIG. 8** illustrates a graph of MID immunoassay for detection of *Staphylococcus aureus* using lysostaphin digestion for various times. Sa+ = *Staphylococcus aureus* positive sample Sa- = *Staphylococcus aureus* negative sample. Samples prepared according to Experimental Example 1.

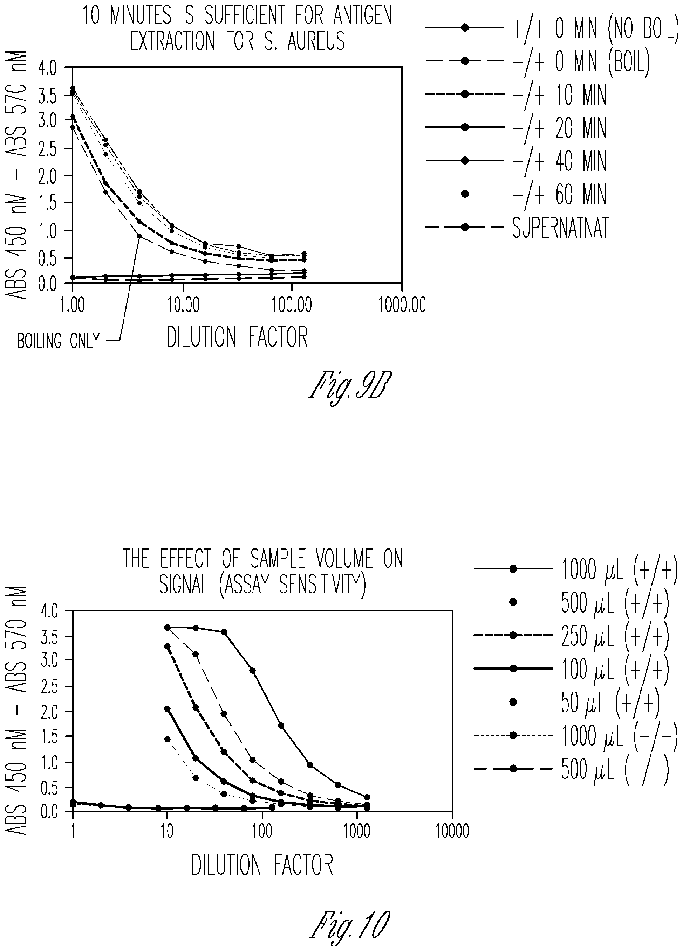

**FIGS. 9A-9B** illustrate graphs MID immunoassay of *Staphylococcus aureus* in synovial fluid samples prepared using various lysostaphin digestion times. Maximal antigen extraction occurs at 10 minutes lysostaphin digestion for synovial fluid samples testing α-defensin positive and culture positive (+/+) Samples prepared according to Experimental Example 1.

**FIG. 10** illustrates a graph of sample volume effect on assay sensitivity in MID immunoassay of samples testing α-defensin positive and culture positive (+/+), or α-defensin negative and culture negative (-/-). Larger sample volumes produce samples having greater antigen content. Samples prepared according to Experimental Example 1

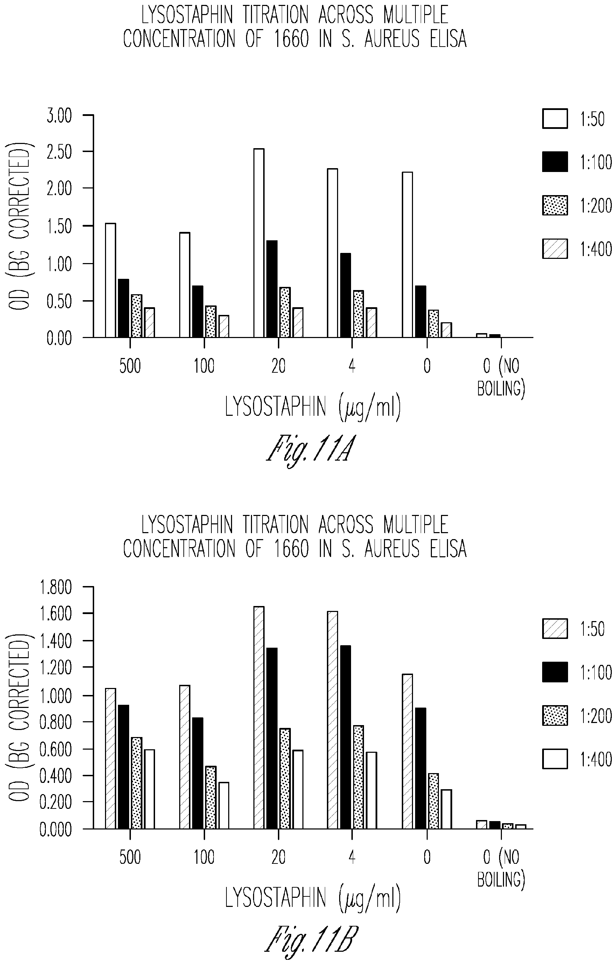

**FIGS. 11A-11B** illustrate bar graphs of lysostaphin titration using various dilutions of a synovial fluid sample, in an ELISA MID immunoassay for detecting *Staphylococcus aureus.*

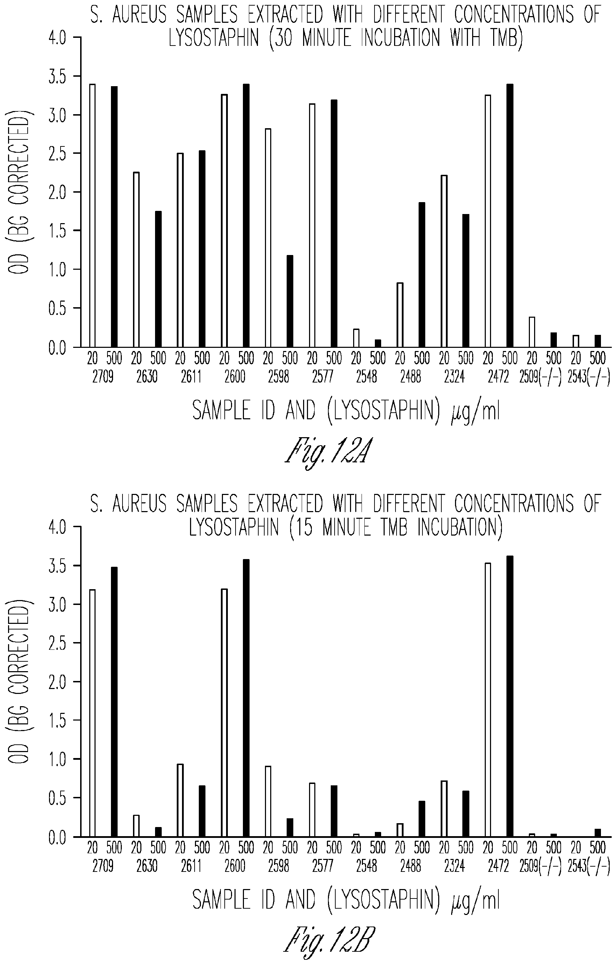

**FIGS. 12A-12B** illustrate bar graphs of MID immunoassay for *Staphylococcus aureus* antigen from synovial fluid samples prepared using lysostaphin extraction of 20 µg/mL and 500 µg/mL. **FIG. 12A** illustrates extraction for 30 minutes. **FIG. 12B** illustrates extraction for 15 minutes These graphs establish that 500 µg/mL lysostaphin is no better than 20 µg/mL lysostaphin. All samples tested α-defensin positive and culture positive (+/+), unless otherwise indicated to be α-defensin negative and culture negative (-/-). Samples prepared according to Experimental Example 1.

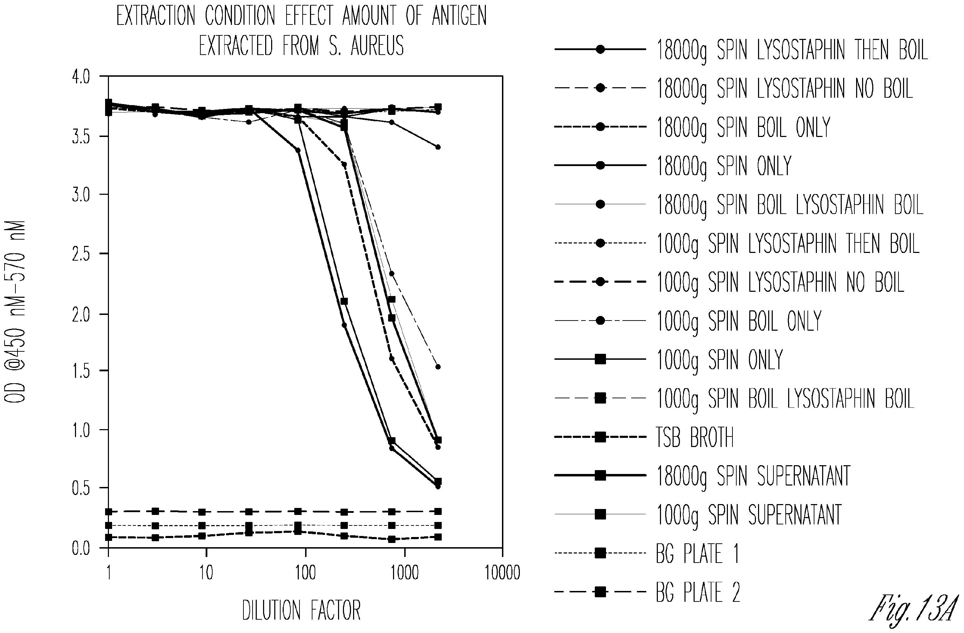

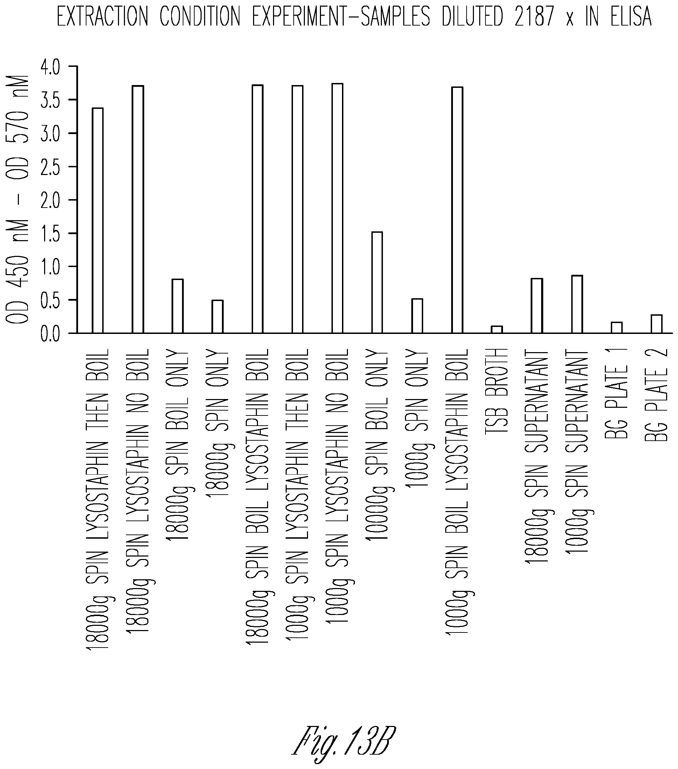

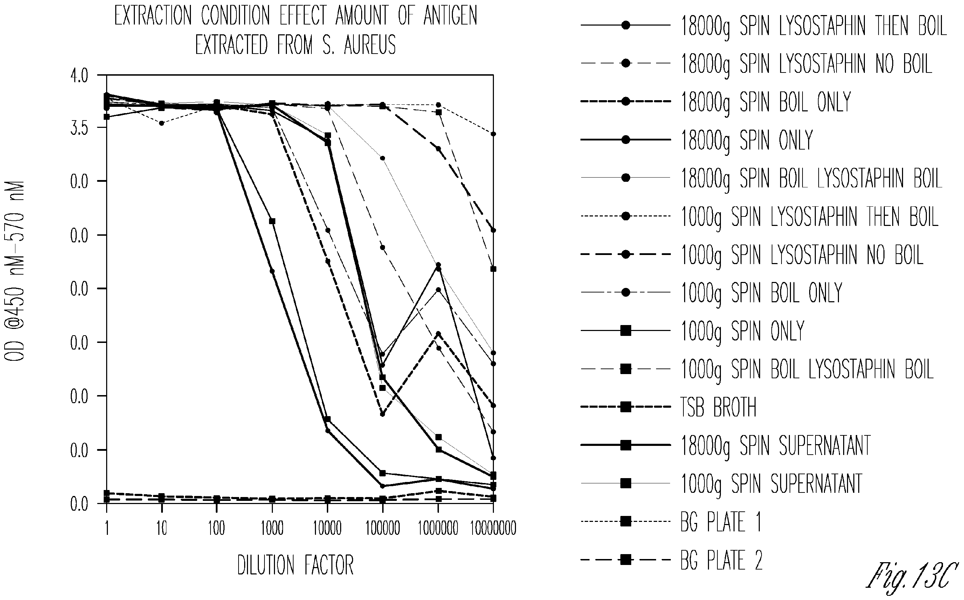

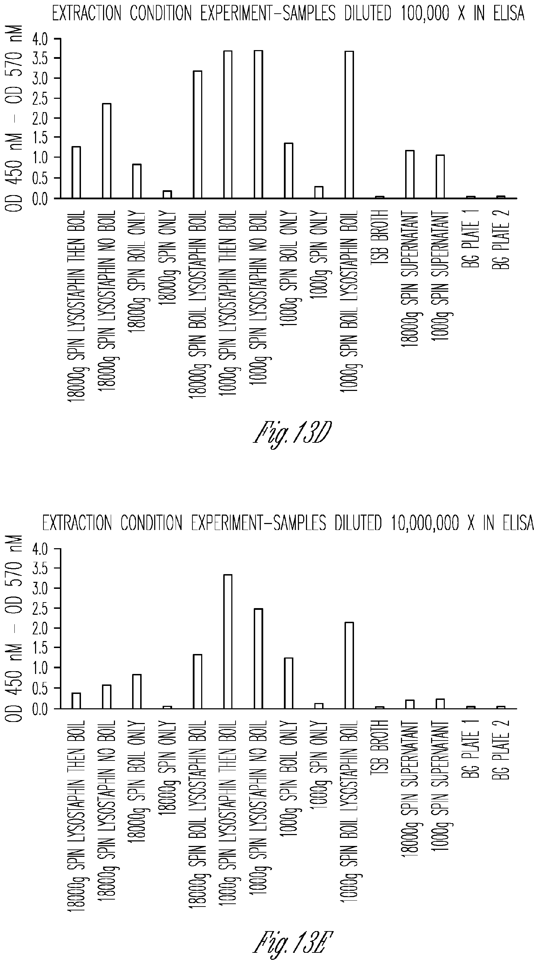

**FIGS. 13A-13D** illustrate graphs of MID immunoassay *Staphylococcus aureus* antigen from fresh culture prepared using various extraction conditions. Lysostaphin treatment exposes more antigen than boiling alone.

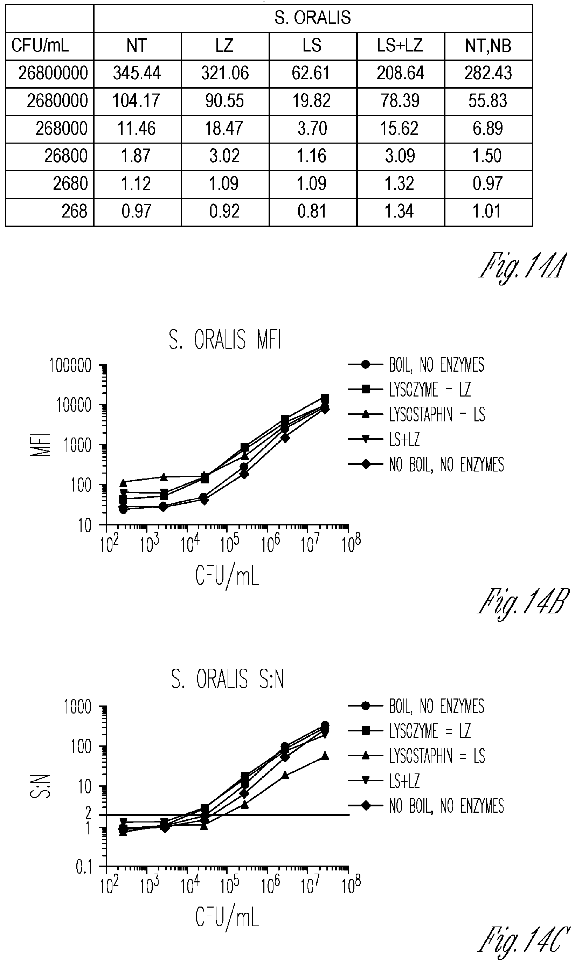

**FIGS. 14A-14C** illustrate data of the effect of lysozyme and/or lysostaphin treatment and boiling on extraction of antigen of *Streptococcus oralis* cultures **FIG. 14A** is a table of calculated signal.noise for various extraction conditions. NT = [boiling; no enzymes]. LZ = lysozyme treatment. LS = lysostaphin treatment. NTINB = [no enzymes; no boiling]. **FIG. 14B** and **14C** are graphs of Luminex ^{®} data for the different extraction conditions.

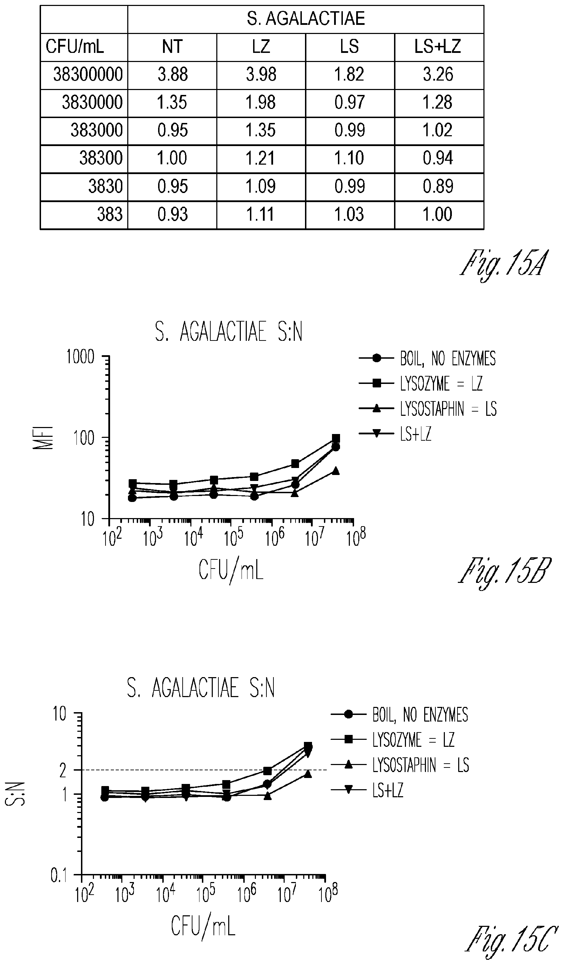

**FIGS. 15A-15C** illustrate data of the effect of lysozyme and/or lysostaphin treatment and boiling on extraction of antigen of *Streptococcus agalactiae* cultures **FIG. 15A** is a table calculated signal -noise(background) for various extraction conditions NT = [boiling; no enzymes?]. LZ = lysozyme treatment LS = lysostaphin treatment. NT/NB = [no enzymes; no boiling]. **FIG. 15B** and **15C** are graphs of Luminex data for the different extraction conditions

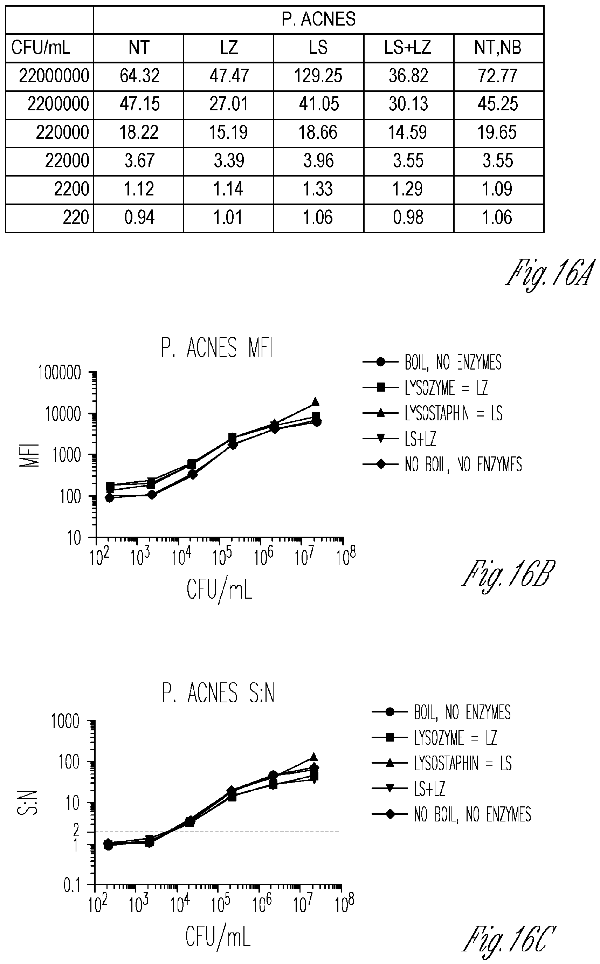

**FIGS. 16A-16C** illustrate data of the effect of lysozyme and/or lysostaphin treatment and boiling on extraction of antigen of *Propionibacterium acnes* cultures. **FIG. 16A** is a table of calculated signal noise for various extraction conditions. NT = [boiling, no enzymes?] LZ = lysozyme treatment LS = lysostaphin treatment. NT/NB = [no enzymes; no boiling]. **FIG. 16B** and 16C are graphs of Luminex data for the different extraction conditions.

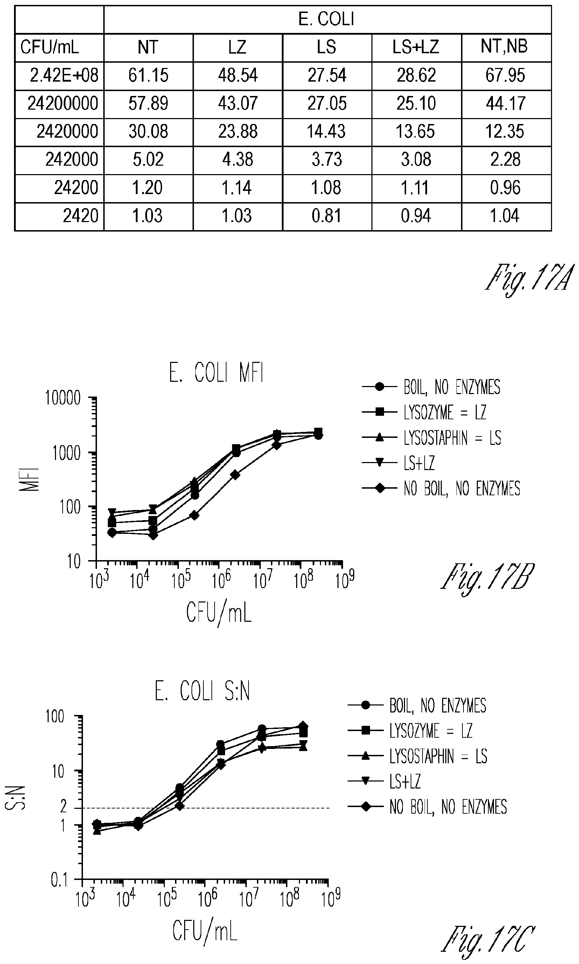

**FIGS. 17A-17C** illustrate data of the effect of lysozyme and/or lysostaphin treatment and boiling on extraction of antigen of *Escherichia coli* cultures. **FIG. 17A** is a table of calculated signal:noise for various extraction conditions. NT = [boiling: no enzymes?] LZ = lysozyme treatment LS = lysostaphin treatment NT/NB = [no enzymes; no boiling]. **FIG. 17B** and **17C** are graphs of Luminex data for the different extraction conditions.

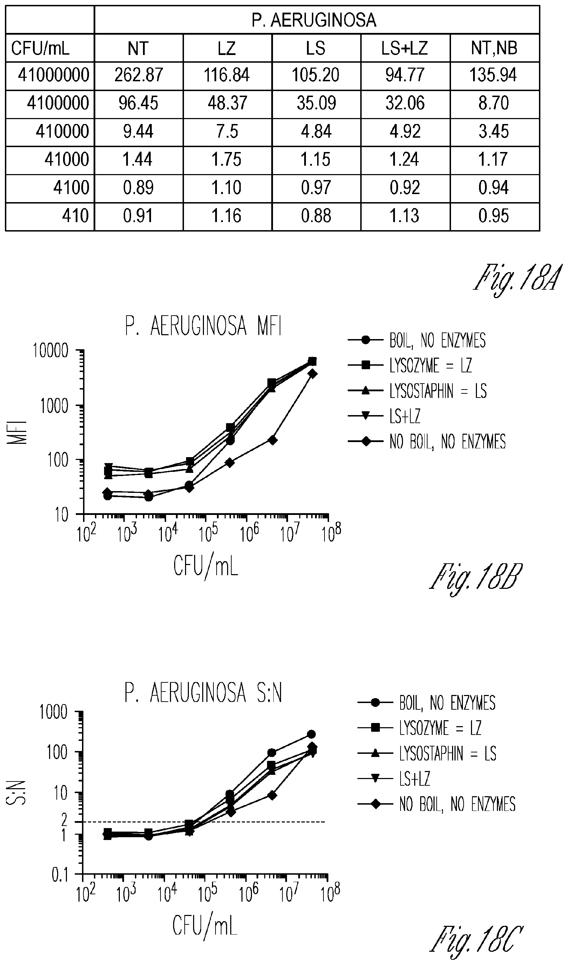

**FIGS. 18A-18C** illustrate data of the effect of lysozyme and/or lysostaphin treatment and boiling on extraction of antigen of *Pseudomonas aeruginosa* cultures. **FIG. 18A** is a table of signal:noise for various extraction conditions. NT = [boiling; no enzymes?]. LZ = lysozyme treatment. LS = lysostaphin treatment NT/NB = [no enzymes; no boiling] **FIG. 18B** and **18C** are graphs of Luminex data for the different extraction conditions.

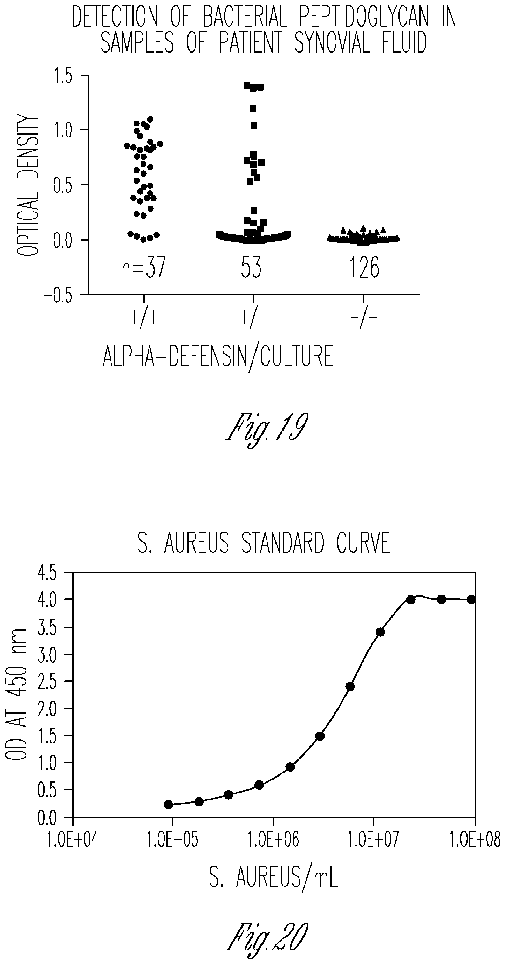

**FIG. 19** illustrates a dot plot of the MID immunoassay detection of peptidoglycan in synovial fluid samples testing α-defensin positive and culture positive (+/+), α-defensin positive and culture negative (+/-), and α-defensin negative and culture negative (-/-)

FIGS. 20A-20B illustrate graphs of MID immunoassay of 5 different *Staphylococcus aureus* samples detected based on the known concentration of bacteria in the sample.

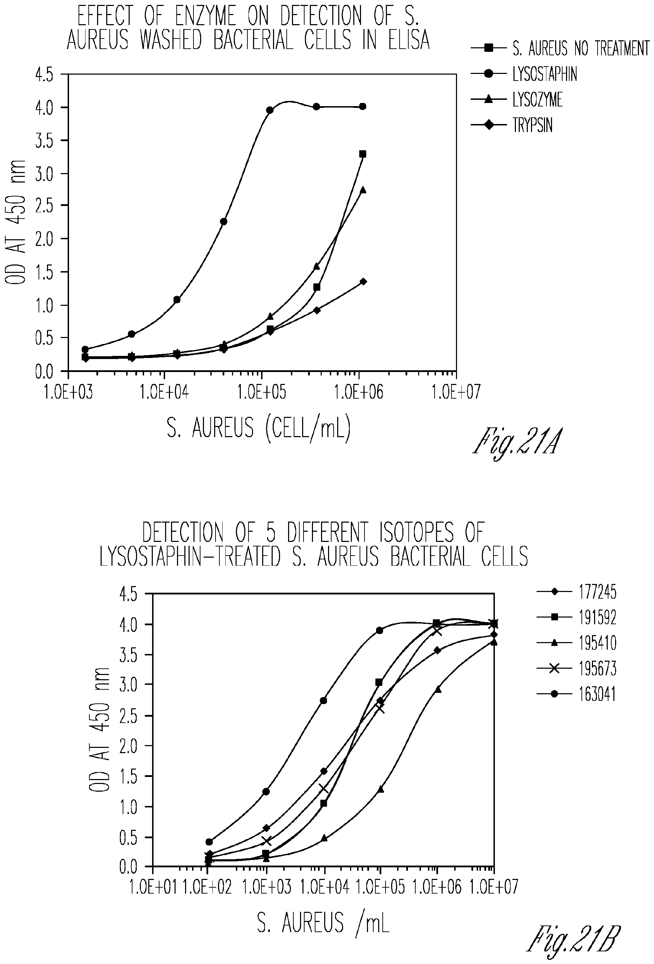

**FIG. 21A-21B** illustrate a graphs and ELISA MID immunoassay for *Staphylococcus aureus* from samples prepared using various enzyme treatments **FIG. 21A** illustrates different enzyme treatment methods **FIG. 21B** illustrates different Staphylococcus aureus strains tested using lysostaphin.

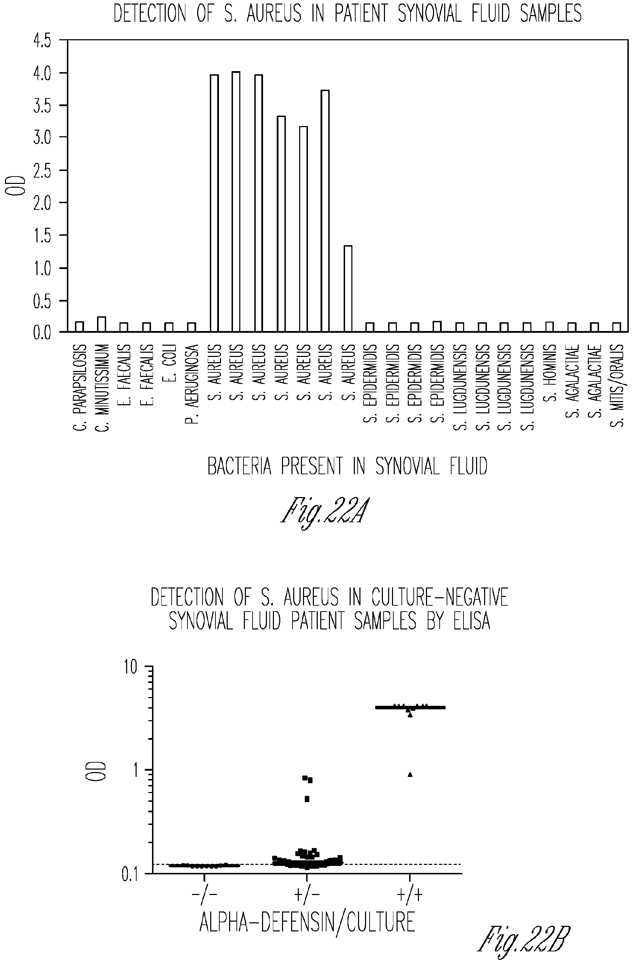

**FIGS. 22A-22B** illustrate results of MID immunoassay for *Staphylococcus aureus* in synovial fluid samples. **FIG. 22A** illustrates a bar graph of the detection of *Staphylococcus aureus* in various synovial fluid samples having known infection by various microorganisms. **FIG. 22B** illustrates the results of an ELISA MID immunoassay of synovial fluid samples testing α-defensin positive and culture positive (+/+), α-defensin positive and culture negative (+/-), and α-defensin negative and culture negative (-/-).

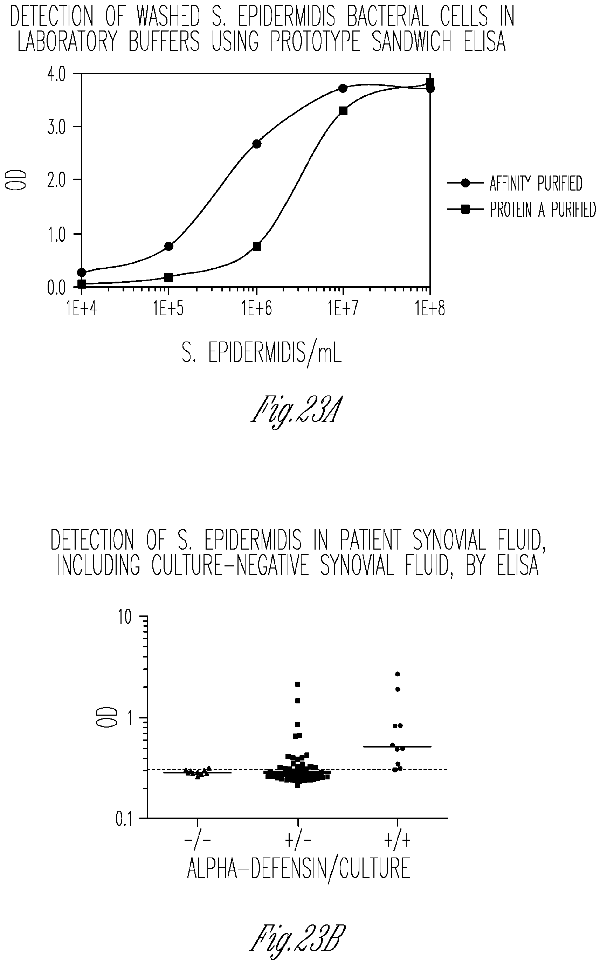

**FIGS. 23A-23B** illustrate results of MID immunoassay for *Staphylococcus epidermidis.* **FIG. 23A** illustrates a graph of the results of an ELISA assay using *anti-Staphylococcus epidermidis* antibody purified by protein A or affinity chromatography. **FIG. 23B** illustrates a dot plot of the results of an ELISA assay of synovial fluid samples testing α-defensin positive and culture positive (+/+), α-defensin positive and culture negative e (+/-), and α-defensin negative and culture- (-/-).

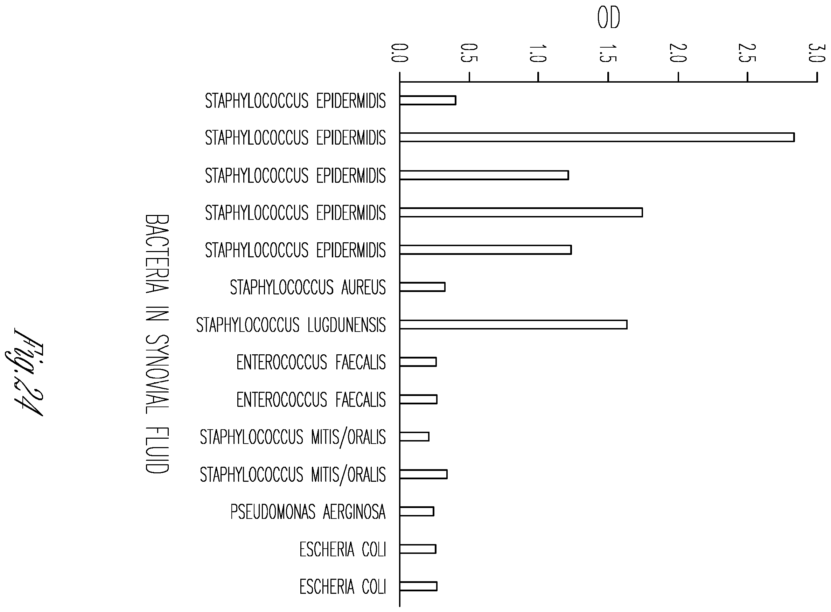

**FIG. 24** illustrates a bar graph of bacteria identified in synovial fluid samples.

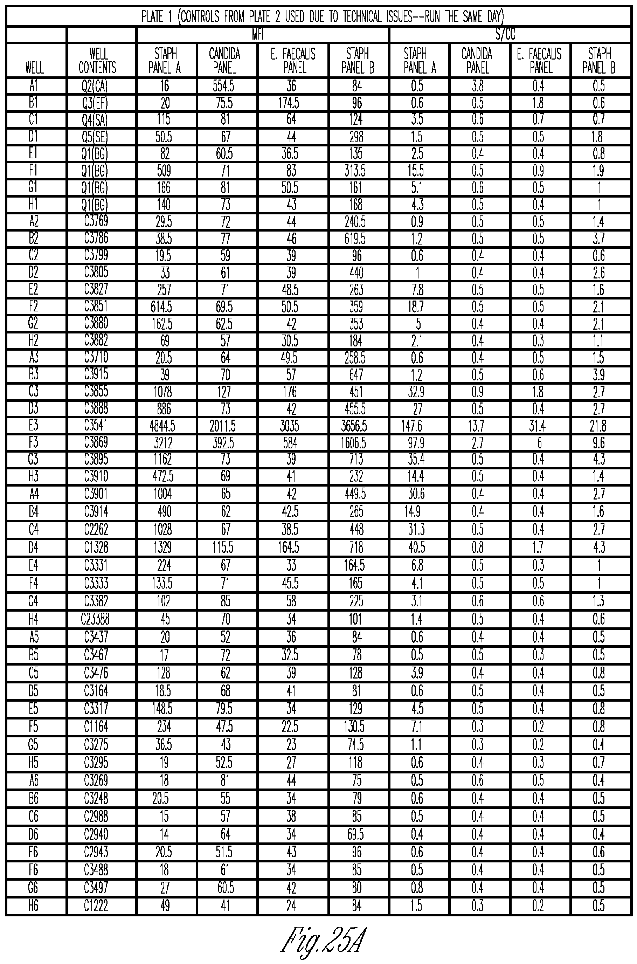

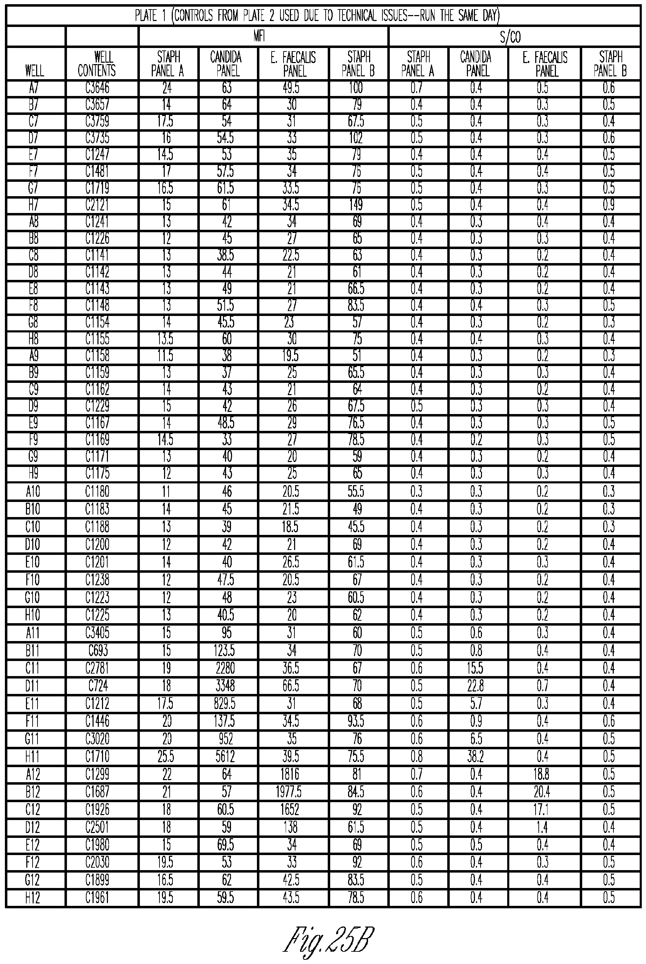

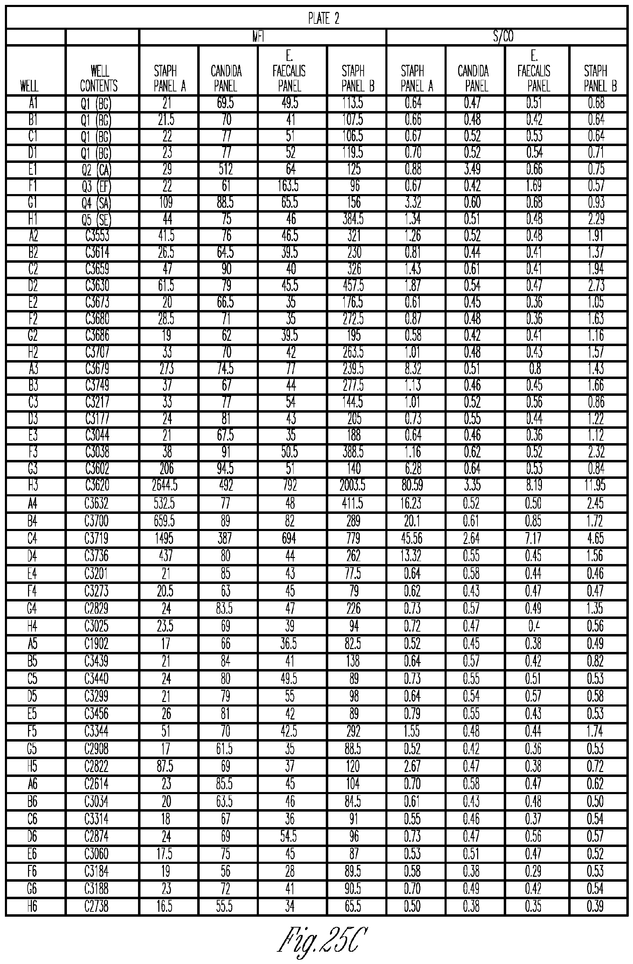

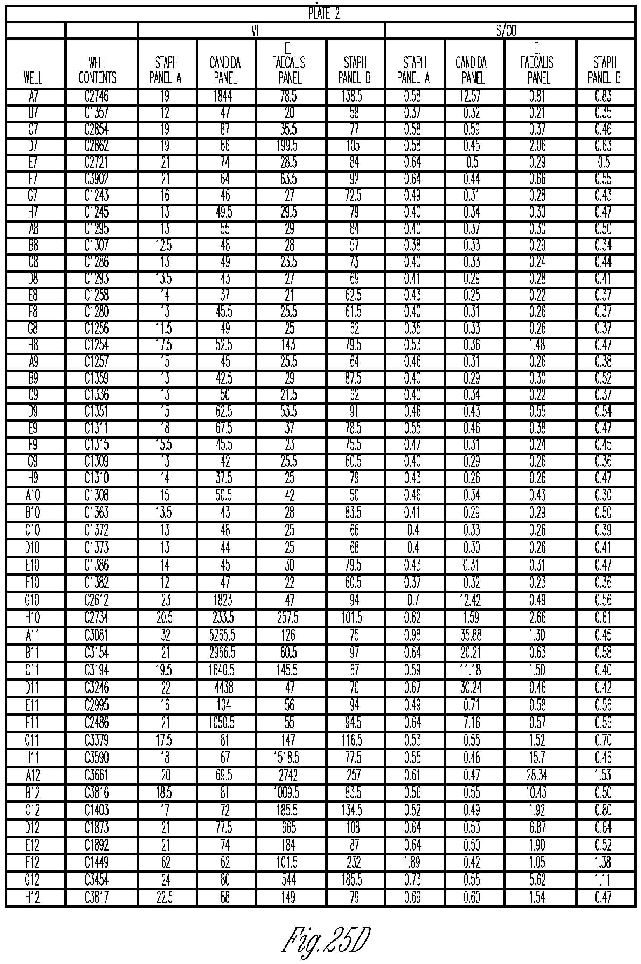

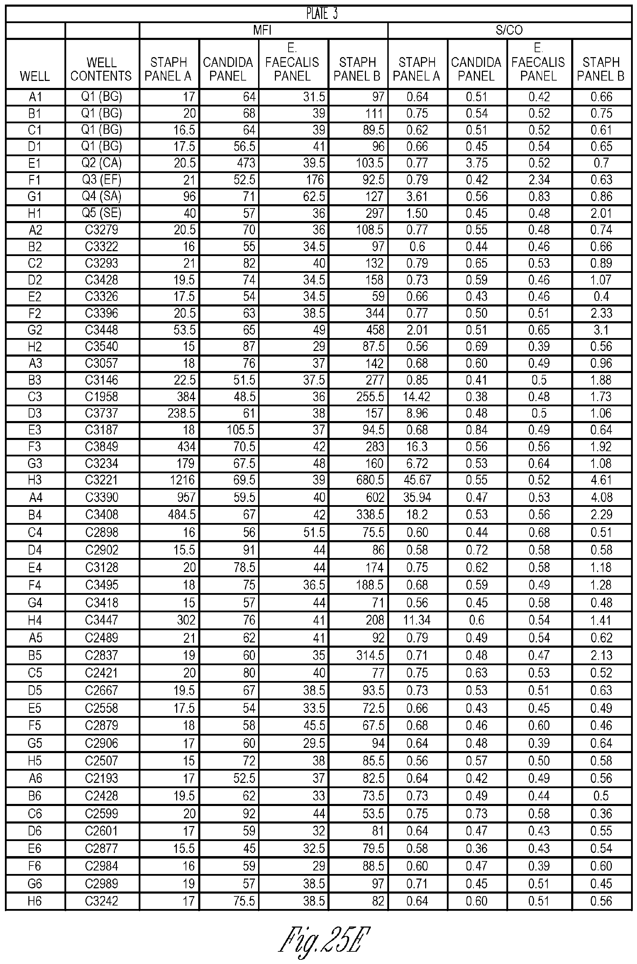

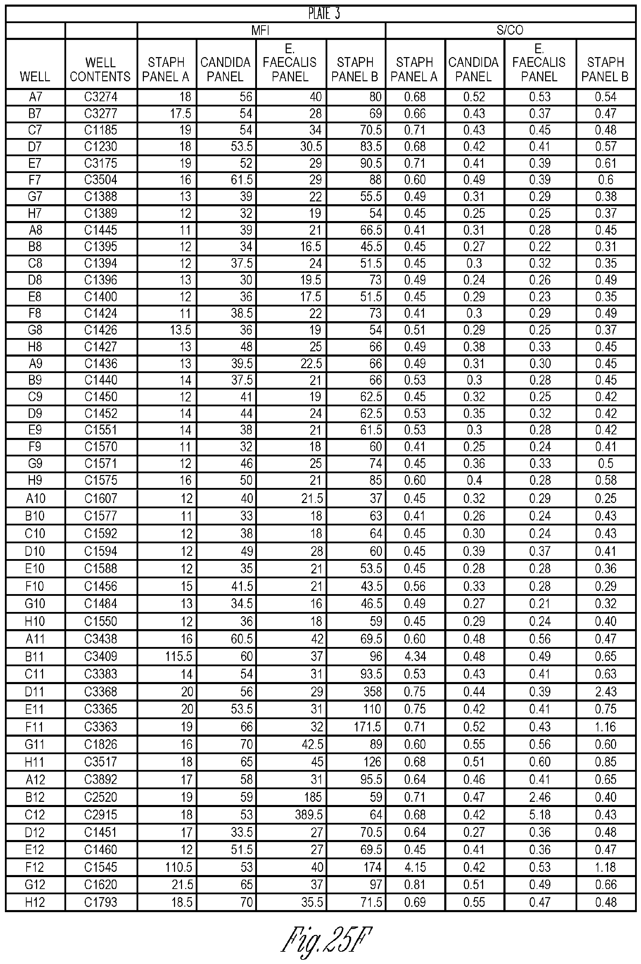

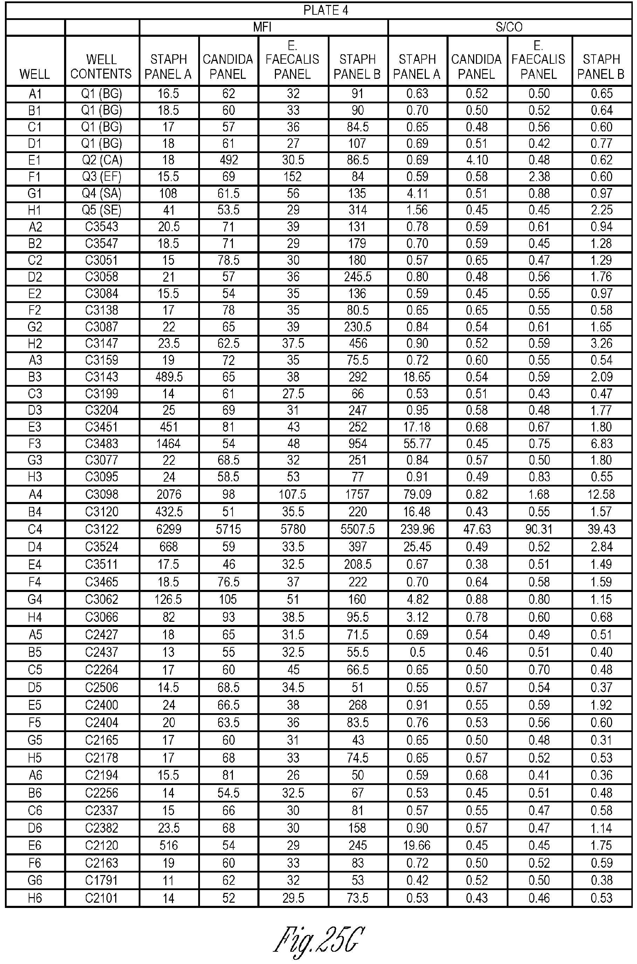

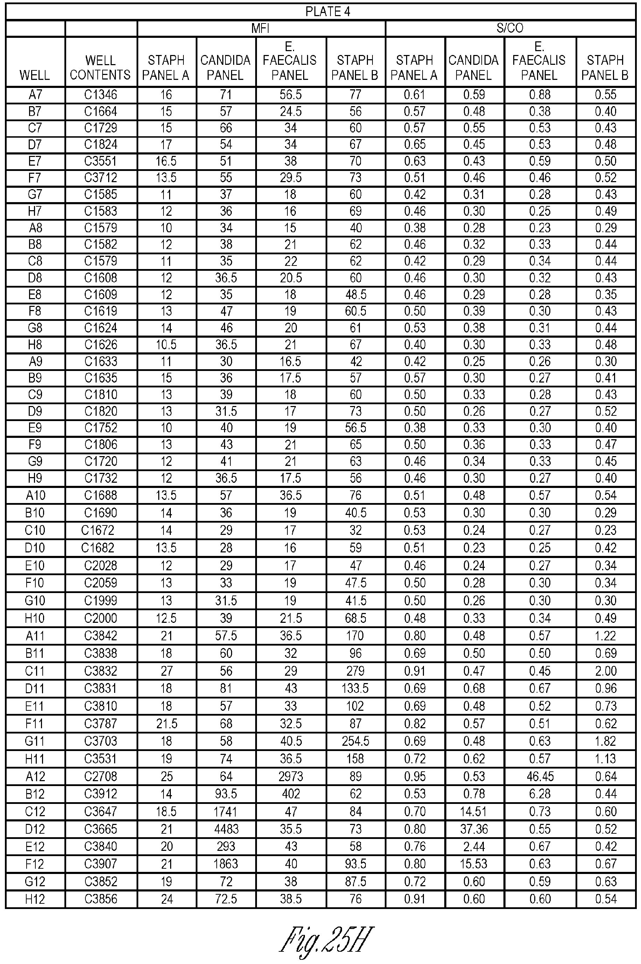

**FIGS. 25A-25H** illustrate a series of tables of immunoassay plate data from a Luminex^{®} assay for *Staphylococcus* aureus (Panel A). *Staphylococcus* epidermidis (Panel B), *Candida albicans,* and *Enterococcus faecalis.* Raw data of median fluorescent intensity ("MFI") and signal, cut-off ("S.C/O" or "S/CO") for each plate Well contents represent the controls or patient sample tested. Note that the controls for plate 1 were erroneous due to a technical error therefore the controls from plate 2 (run at the same time) were used for the assay.

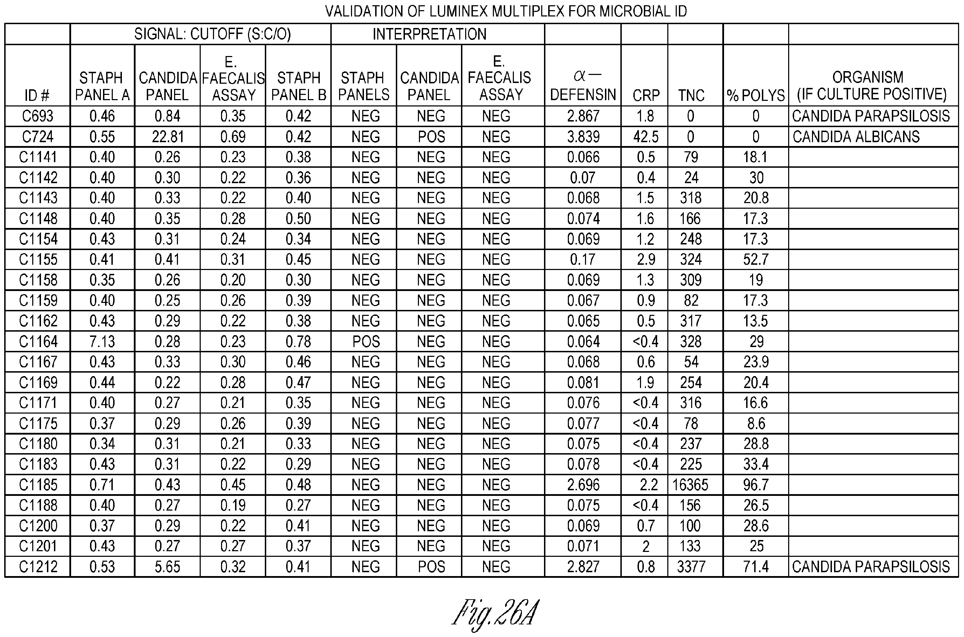

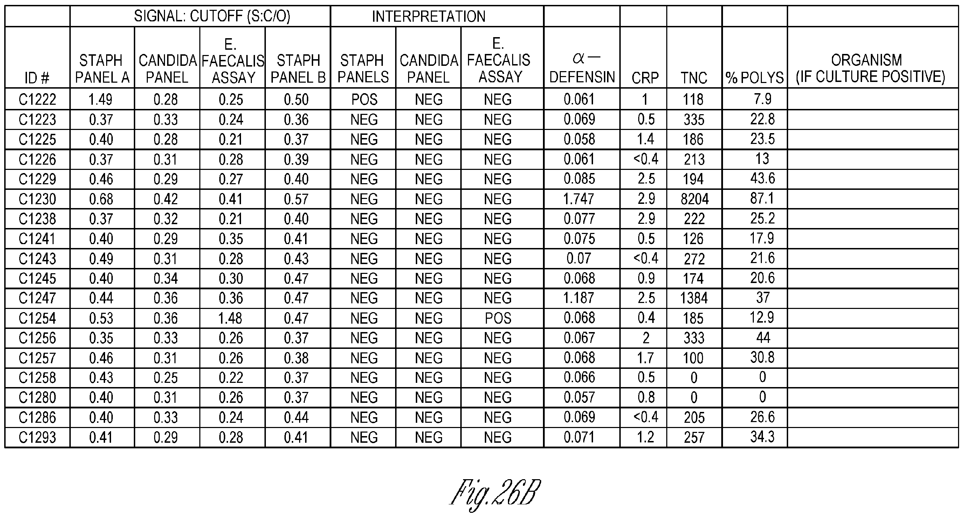

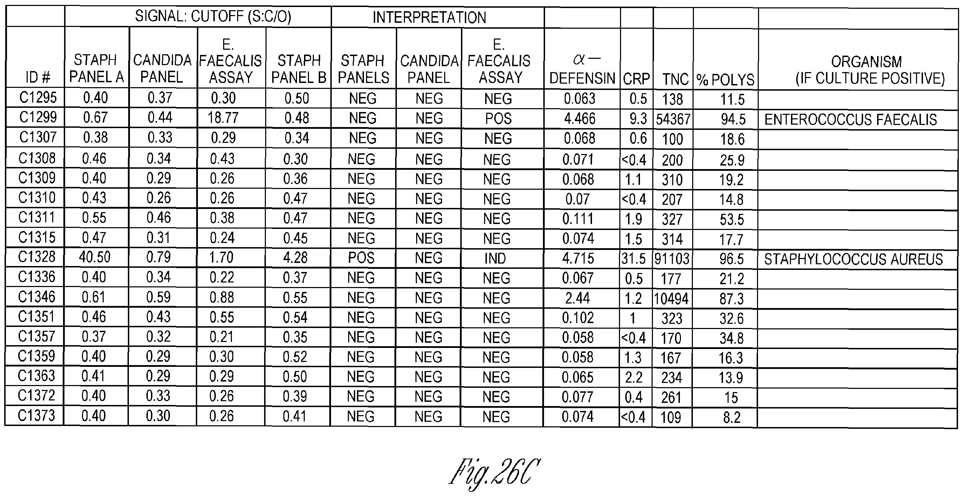

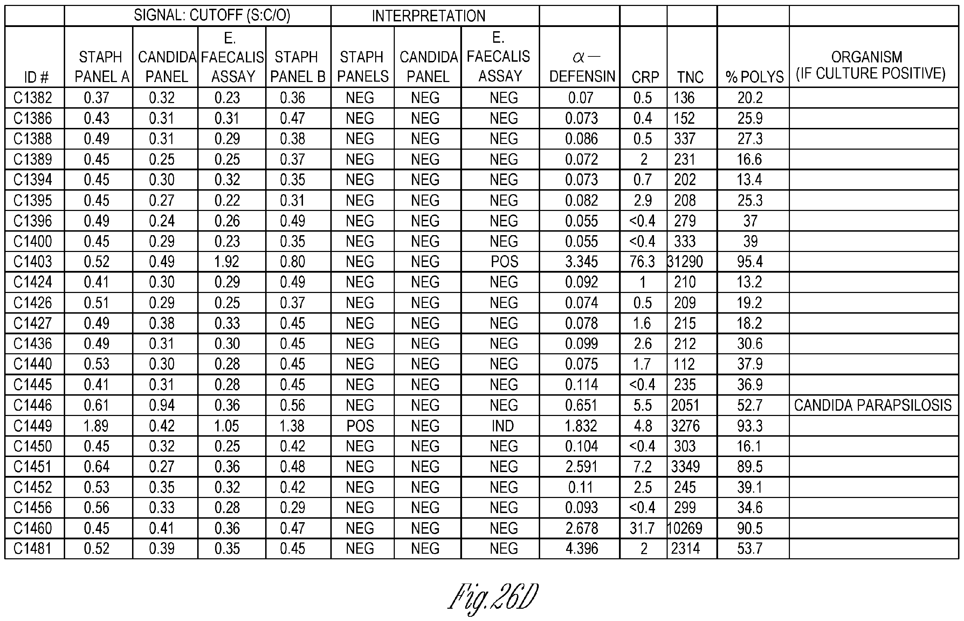

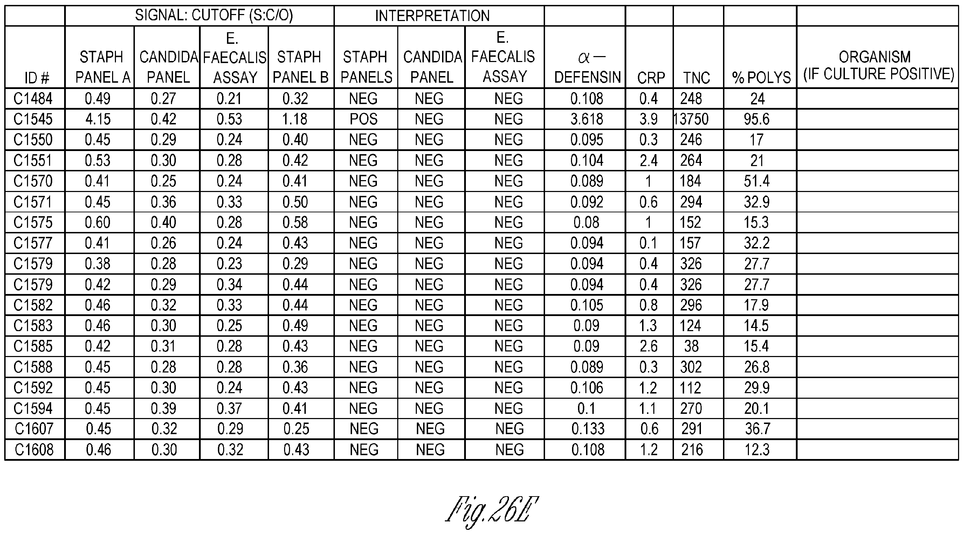

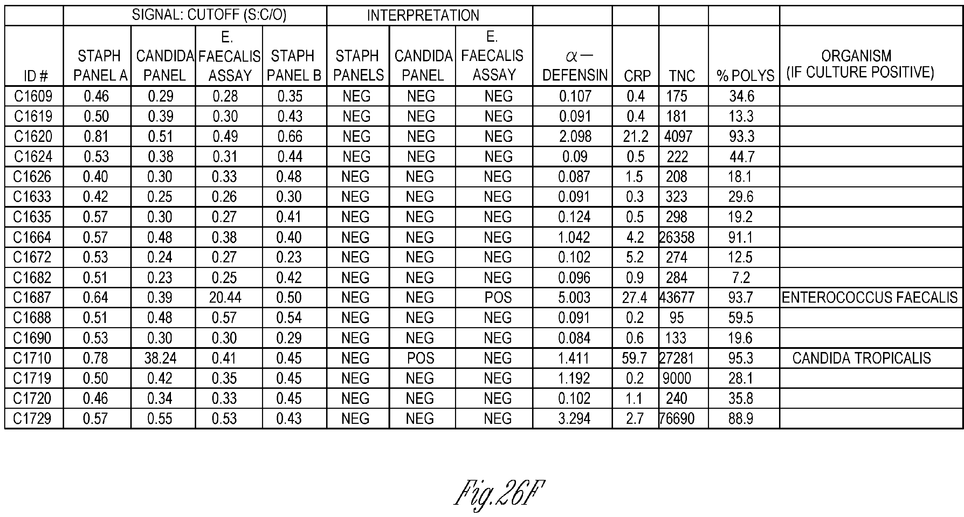

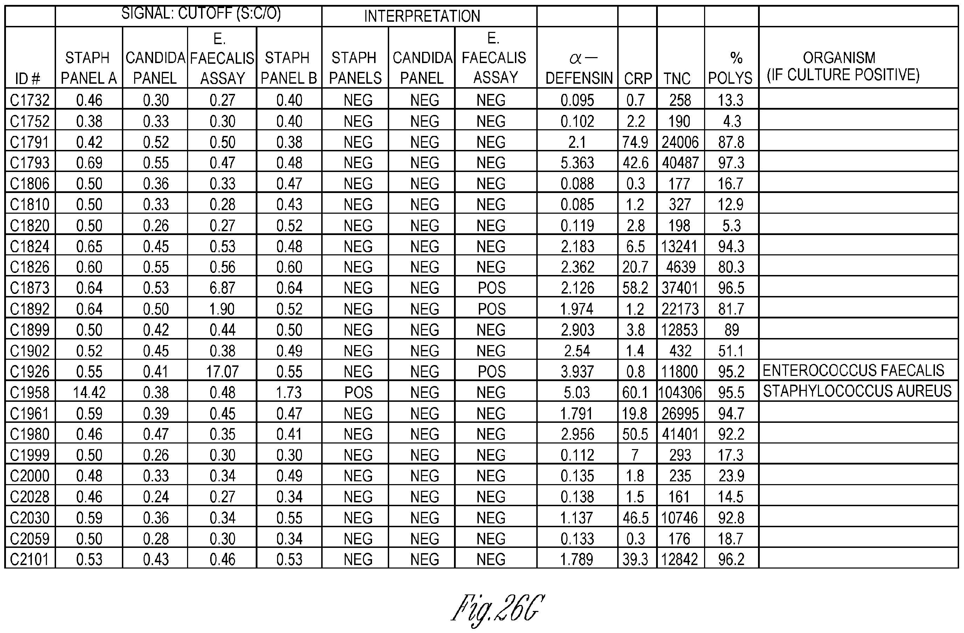

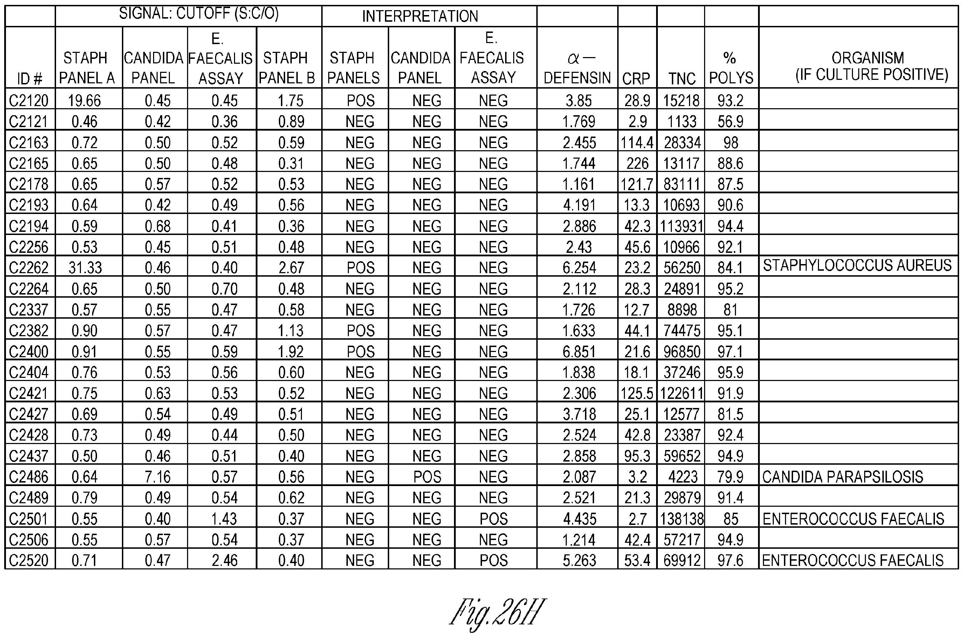

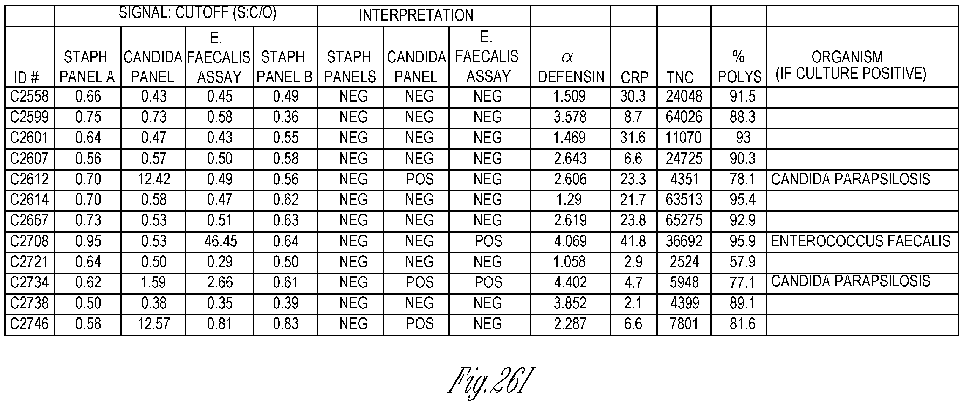

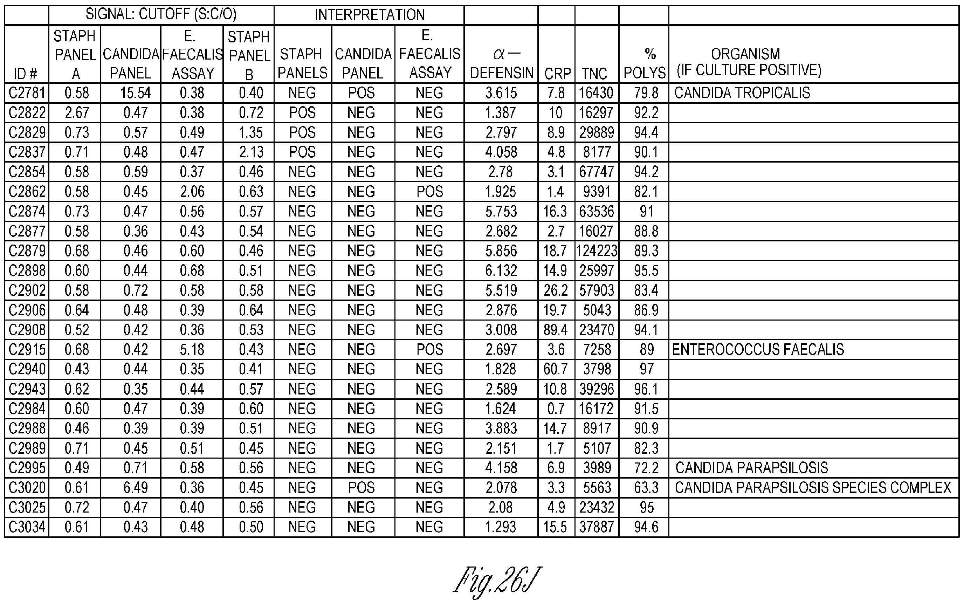

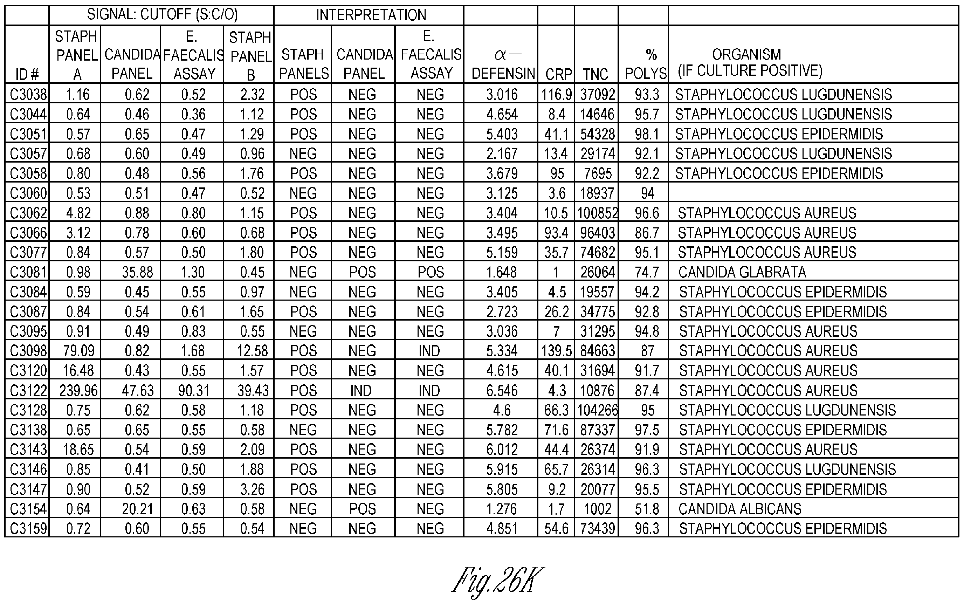

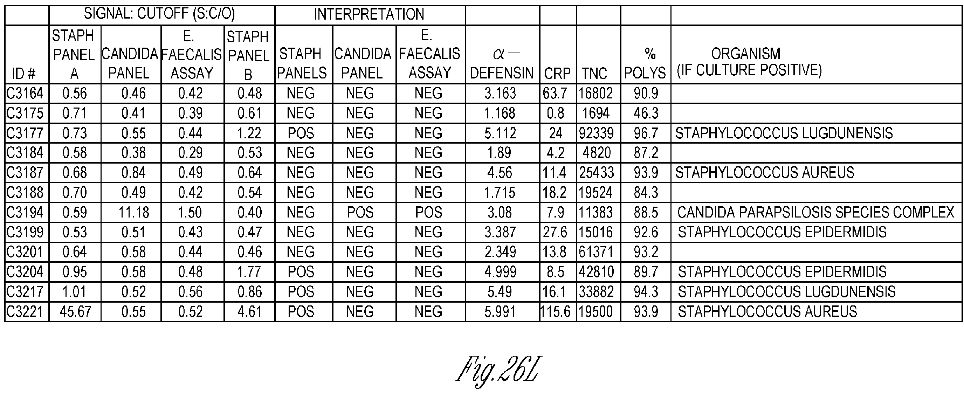

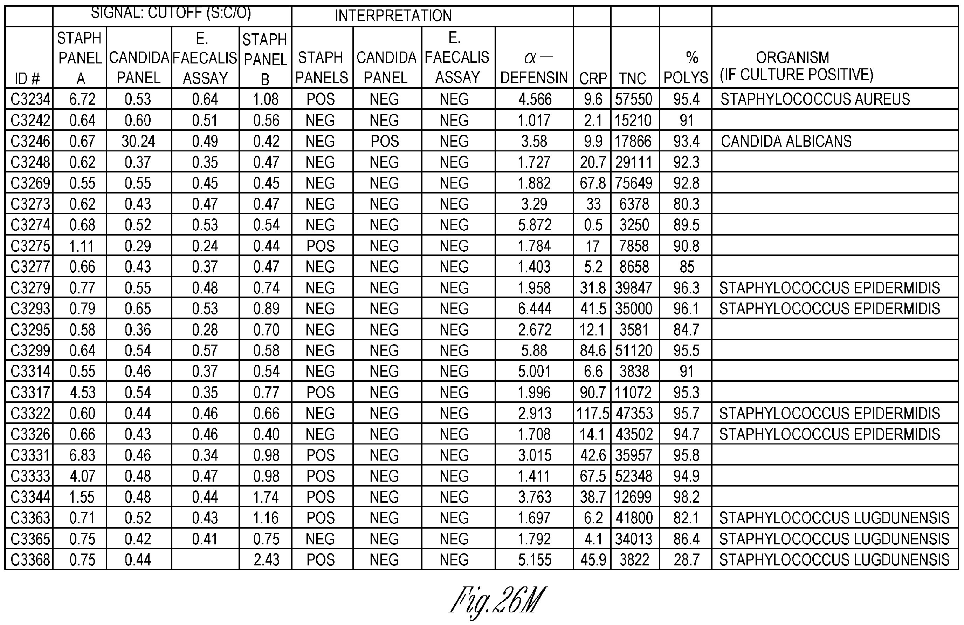

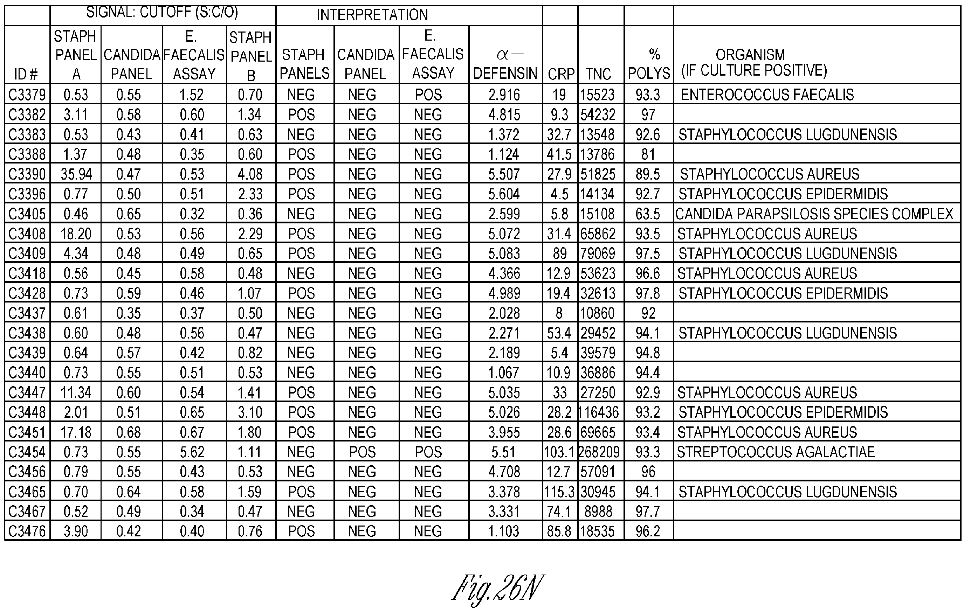

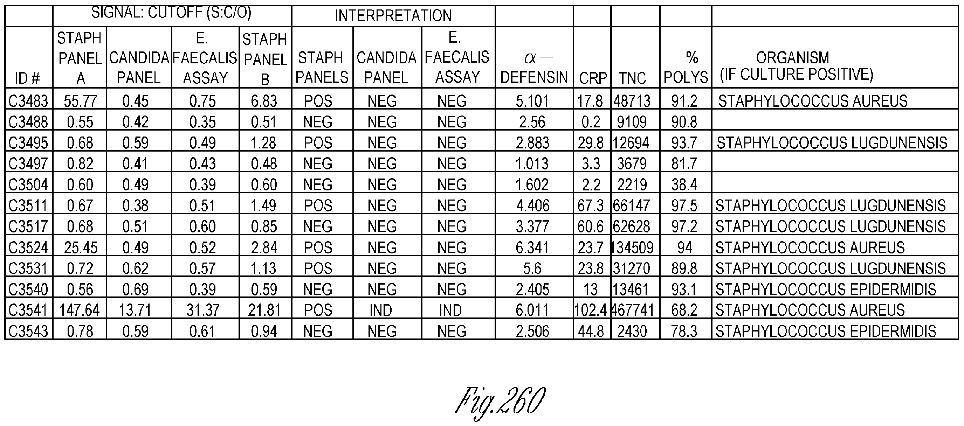

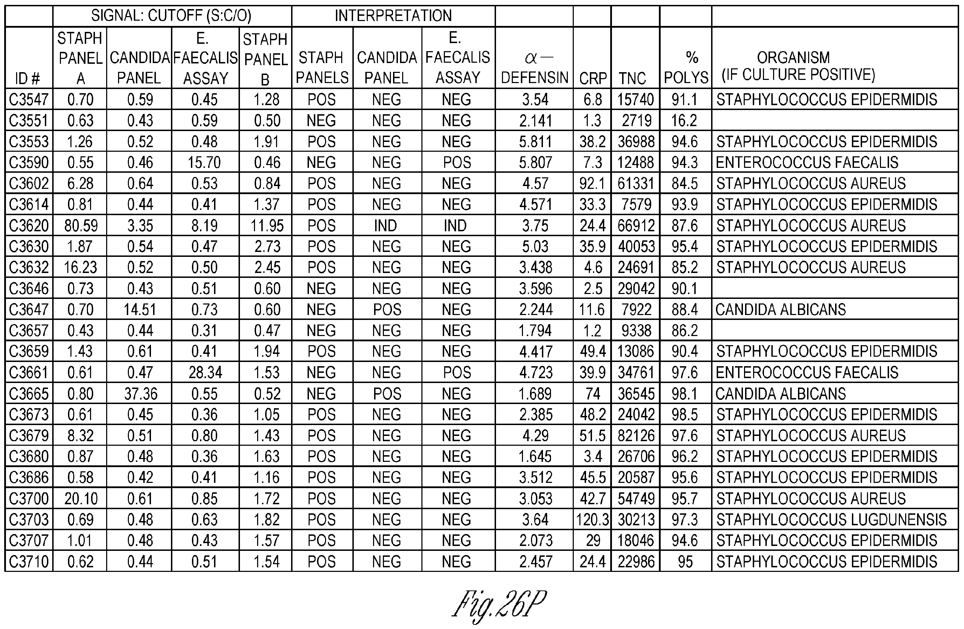

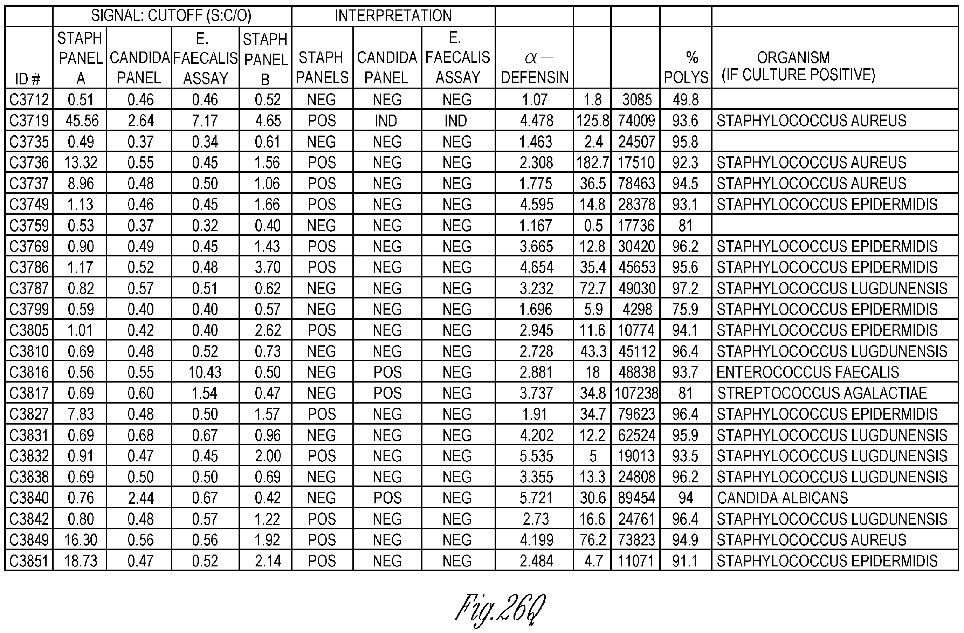

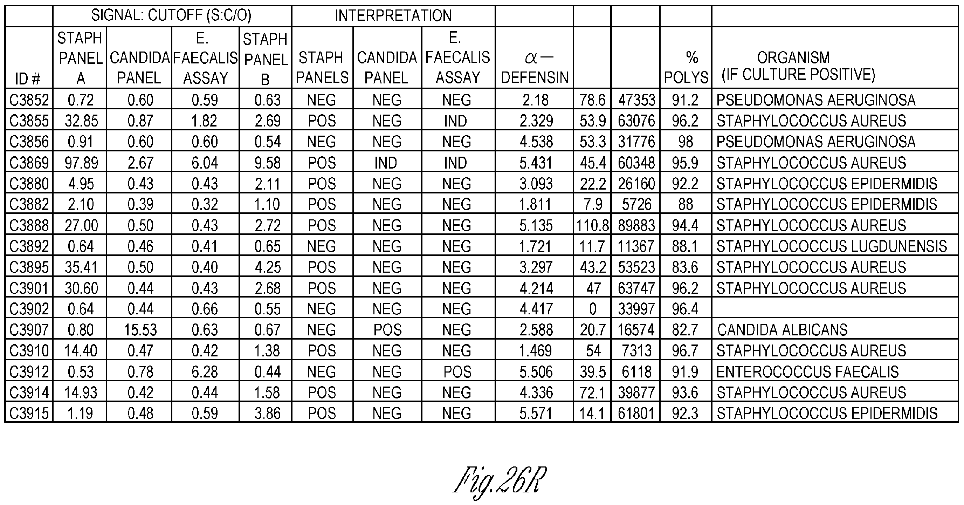

**FIGS. 26A-26R** illustrate a table of individual sample cell and culture data of **FIGS. 25A-25H****,** listed in sample order, S/CO and MID immunoassay interpretation, α-defensin >1 is positive, C-reactive protein (CRP) >3 is positive, total nucleated cells (TNC >3000 is positive). Organism = organism identified through culture at a maximum of 7 days; most samples (>95%) culture positive within 2 days

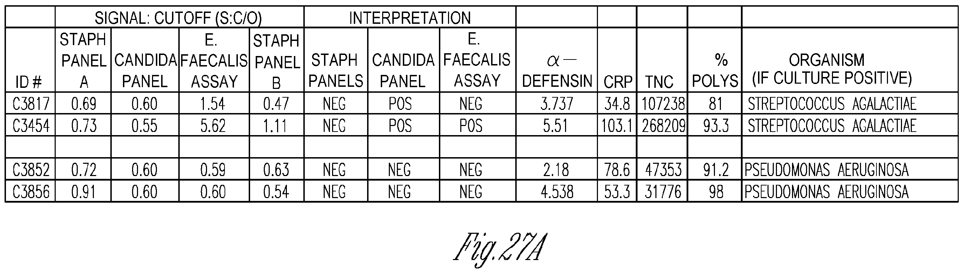

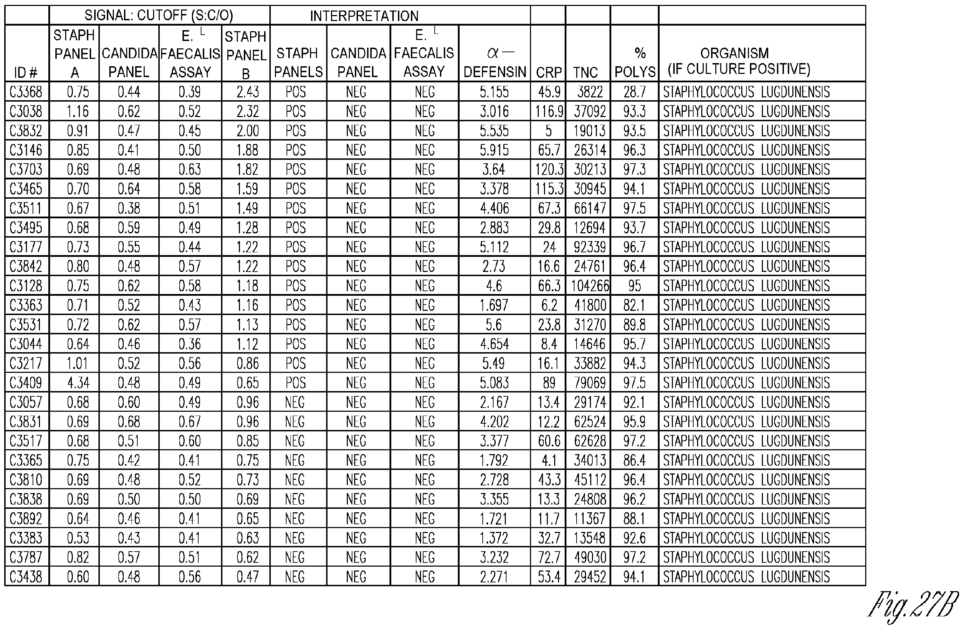

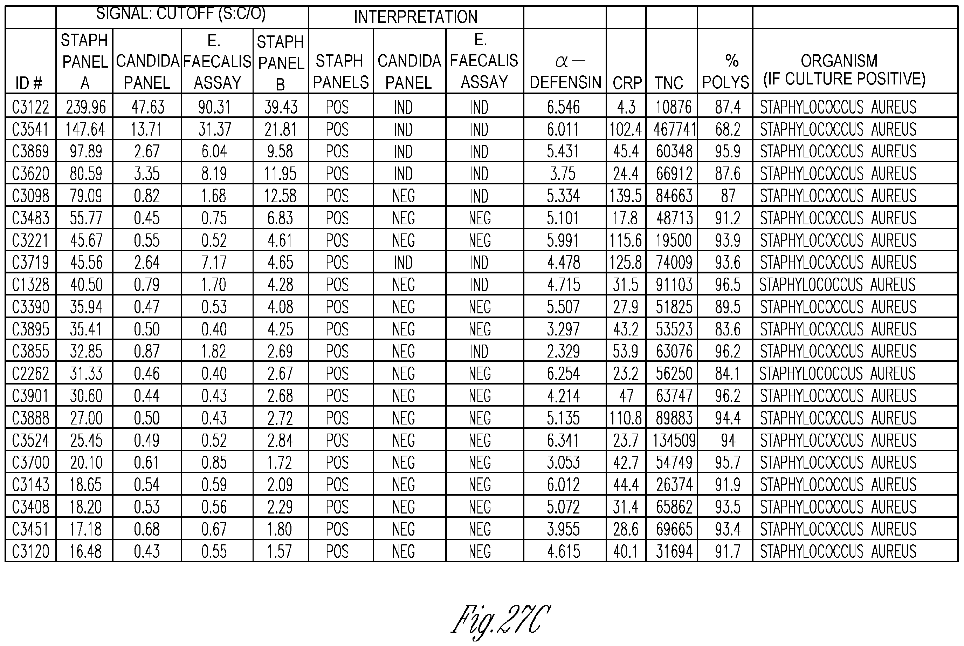

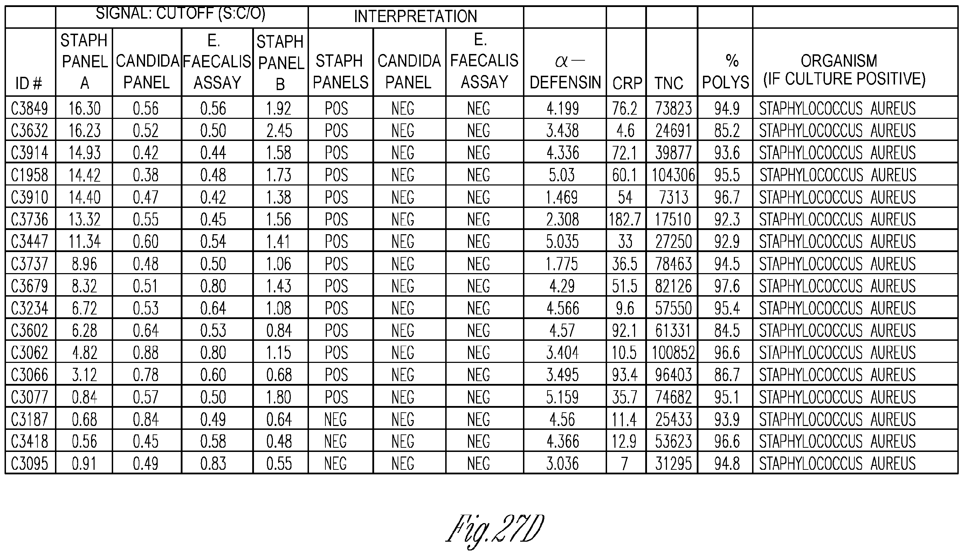

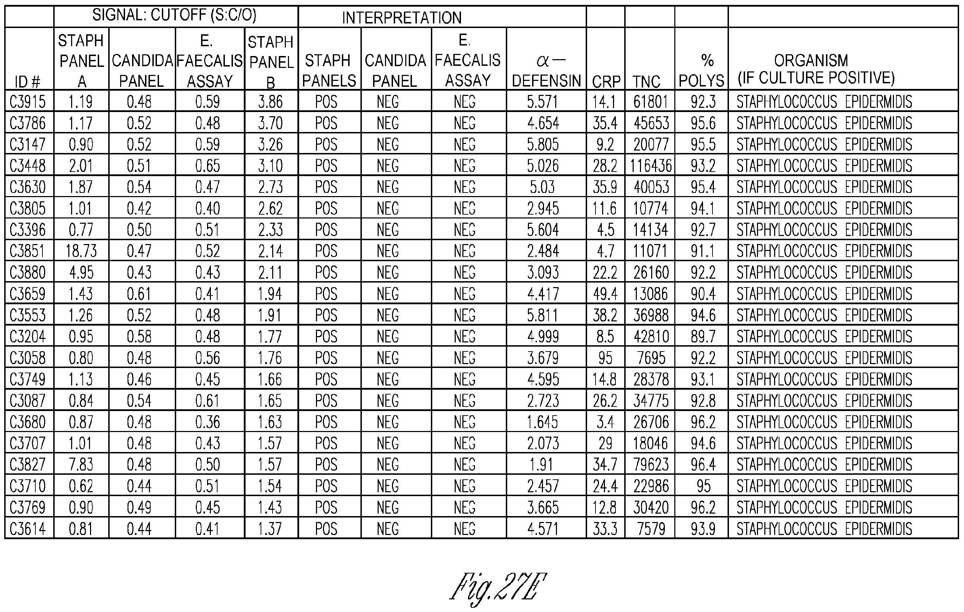

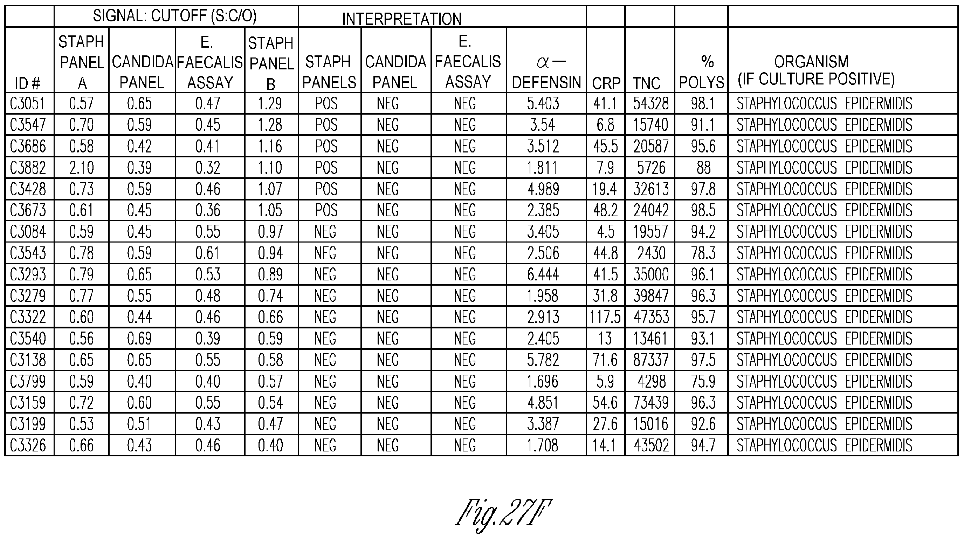

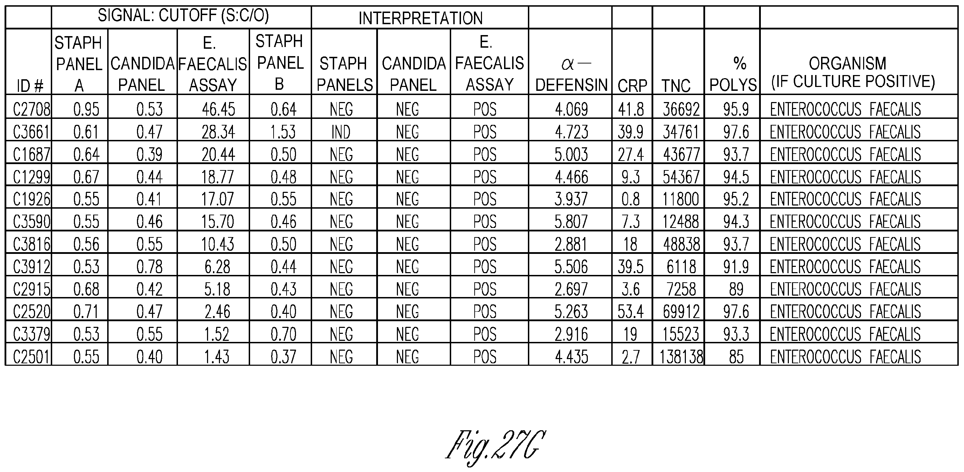

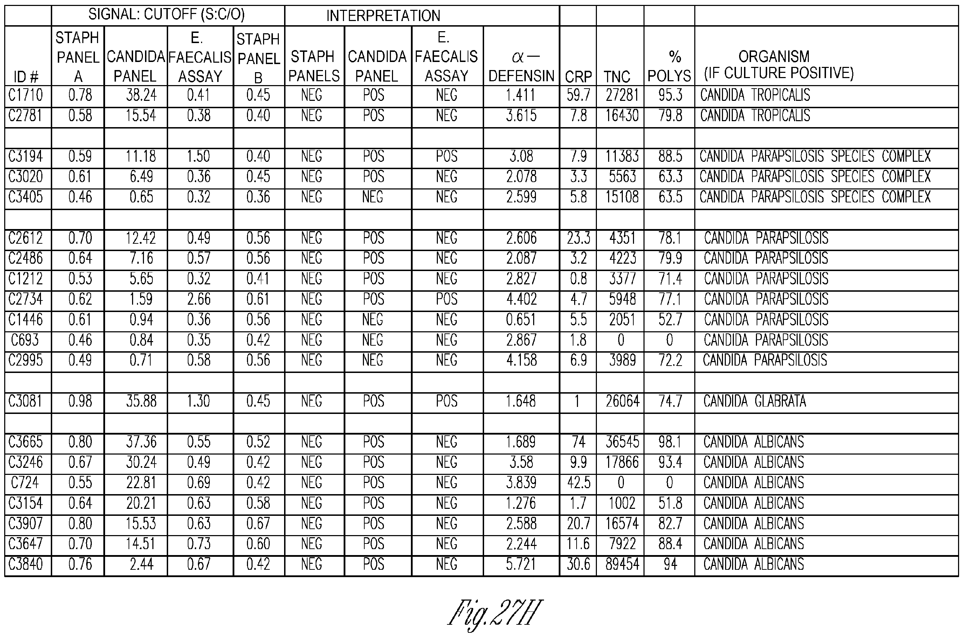

**FIGS. 27A-27H** illustrate a series of tables of individual sample cells and culture data for samples testing positive for various microorganisms according to an example of the MID immunoassay. **FIG. 27A** is a table of synovial fluid samples testing culture positive for *Streptococcus agalactiae* or *Pseudomonas aeruginosa.* **FIG. 27B** is a table of synovial fluid samples testing positive for *Staphylococcus lugdunensis.* **FIG. 27C-27D** is a table of synovial fluid samples testing culture positive for *Staphylococcus aureus.* **FIG. 27E-27F** is a table of synovial fluid samples testing culture positive *Staphylococcus epidermidis* **FIG. 27G** is a table of synovial fluid samples testing culture positive for *Enterococcus faecalis.* **FIG. 27H** is a table of synovial fluid samples testing culture positive for *Candida albicans, candidaparapsilosis, Candida glabrata, or Candida tropicalis.*

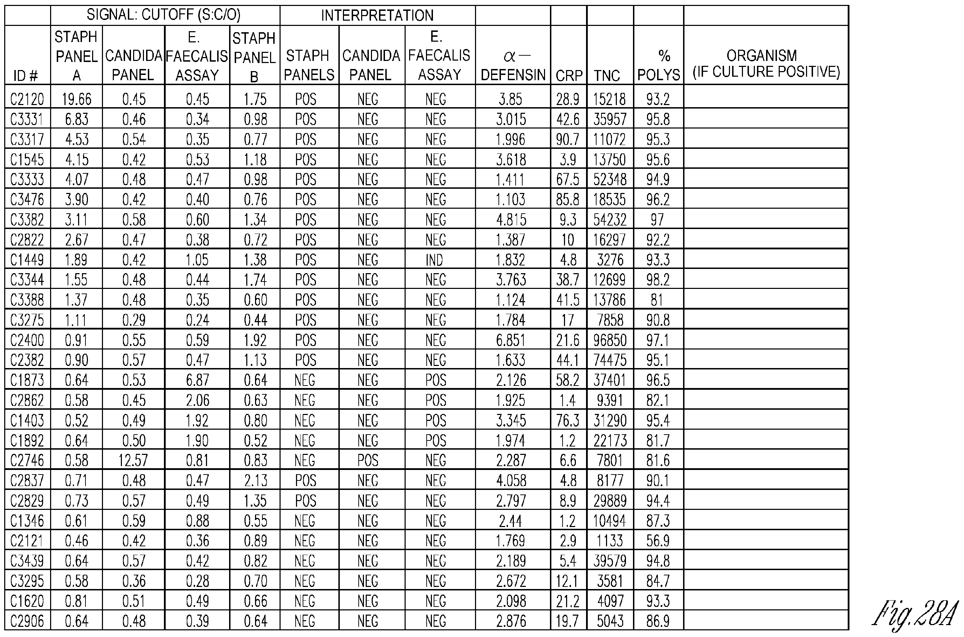

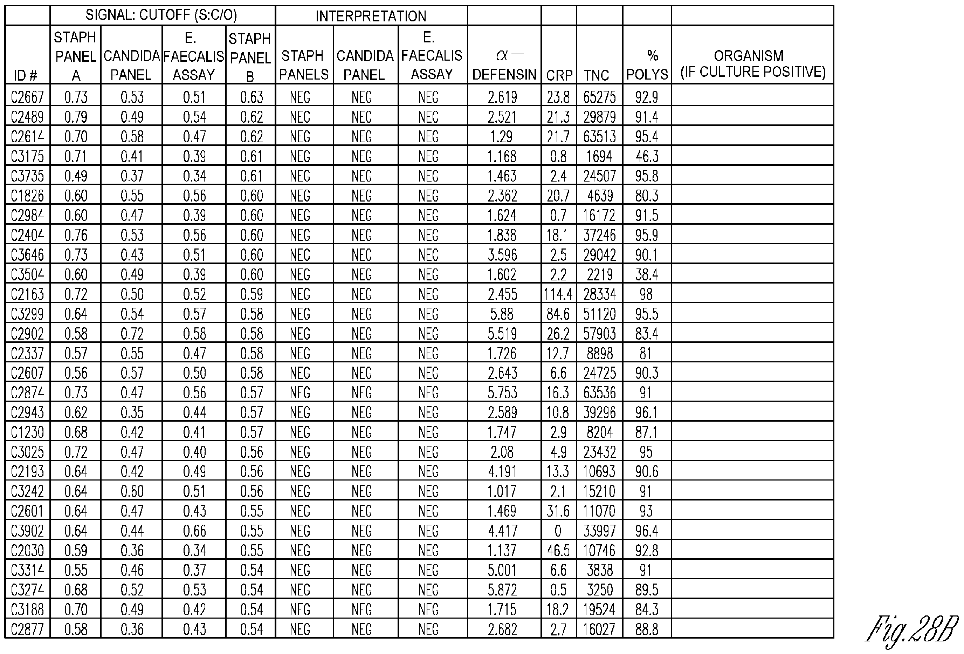

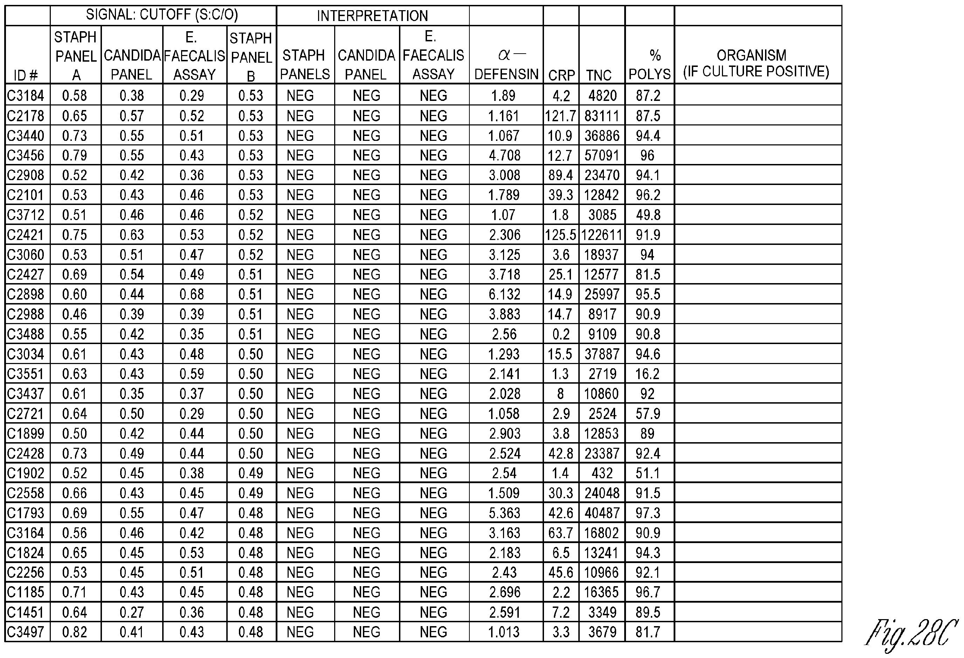

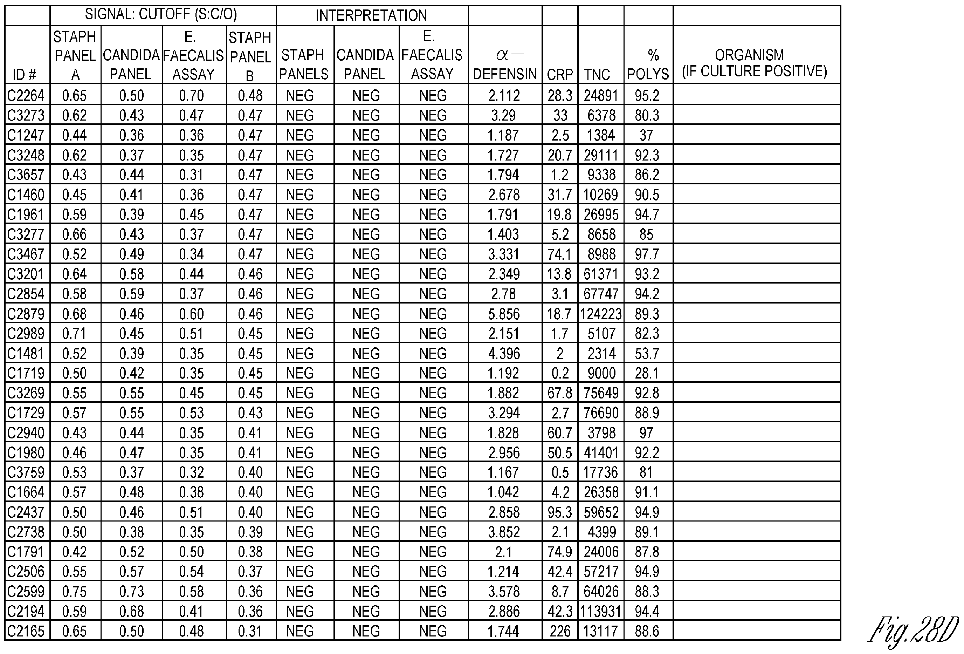

**FIGS. 28A-28D** illustrate table of the results of an example of the MID immunoassay for individual synovial fluid samples testing α-defensin positive and culture negative (+/-)

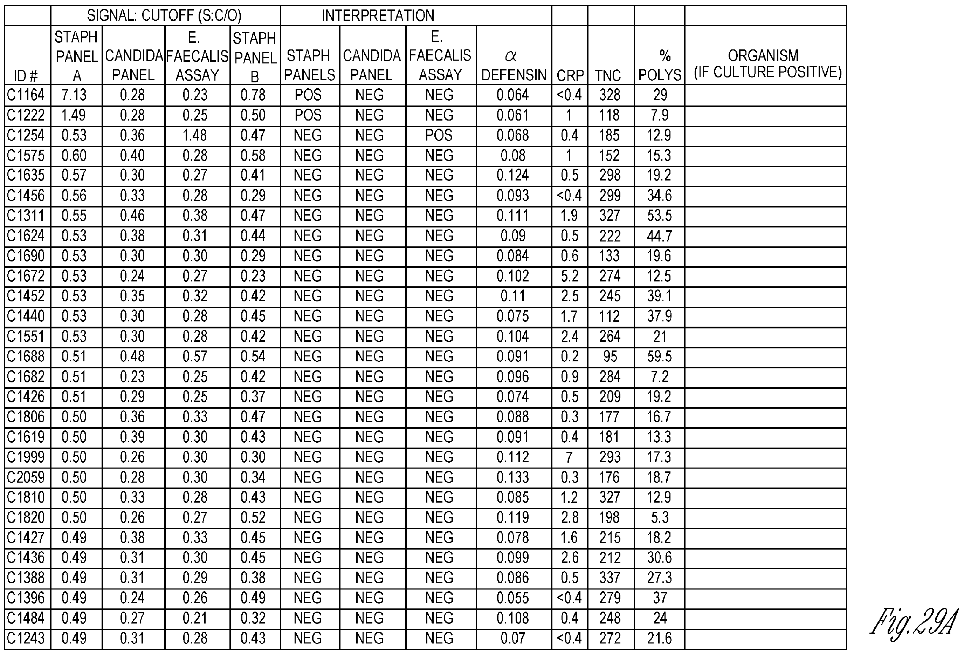

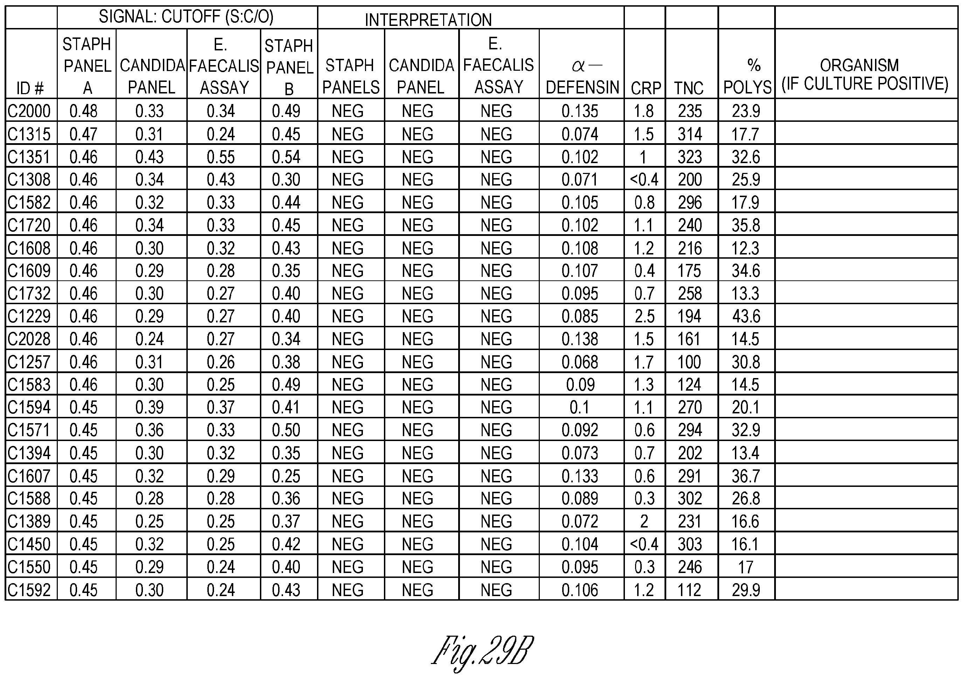

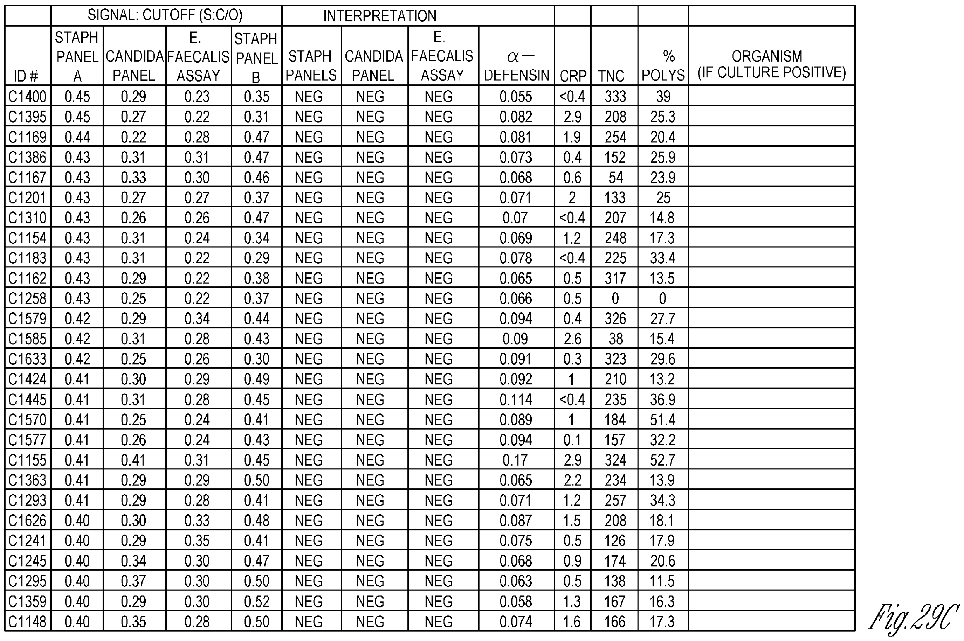

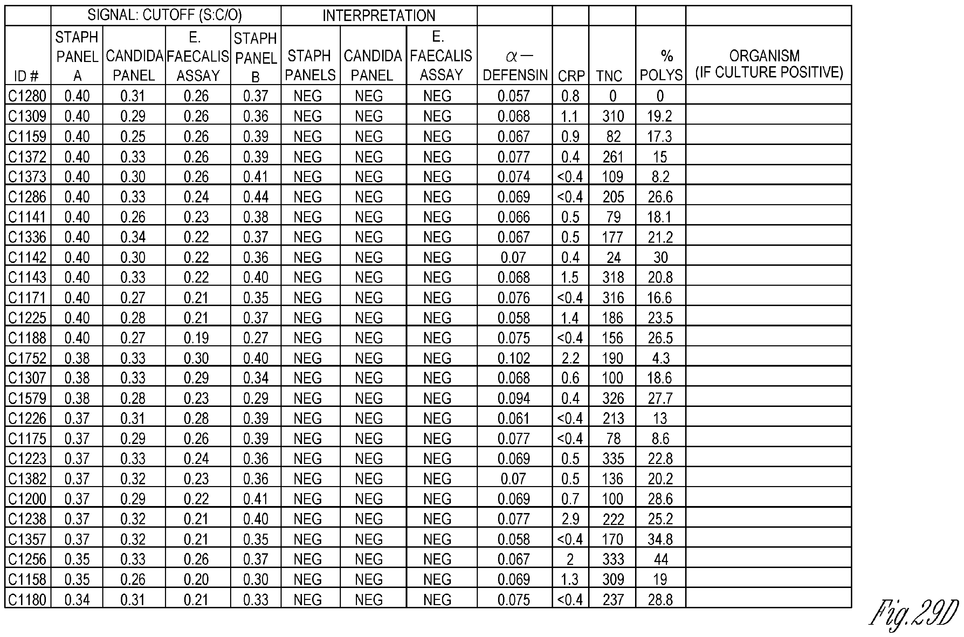

**FIGS. 29A-29D** illustrate a table of an example of the MID immunoassay for individual synovial fluid samples testing α-defensin negative and culture negative (-/-).

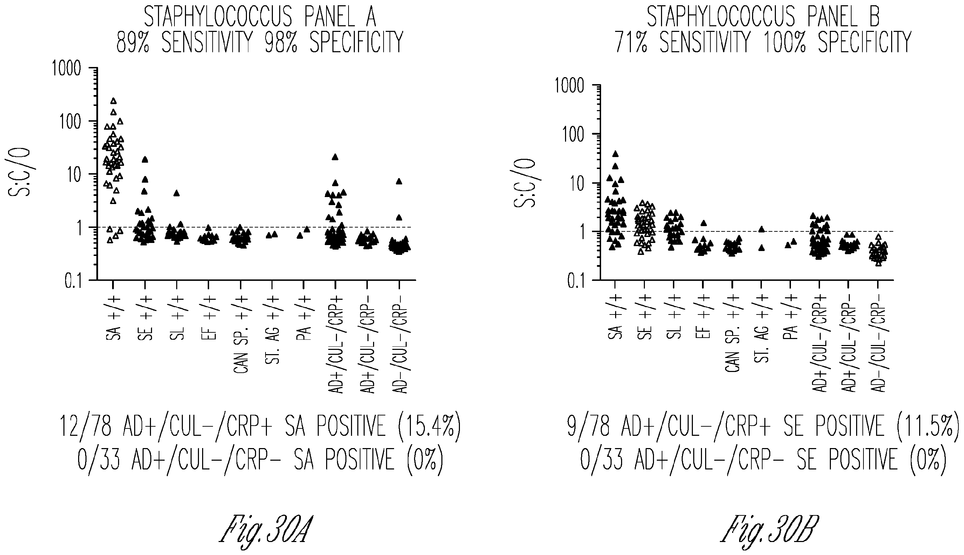

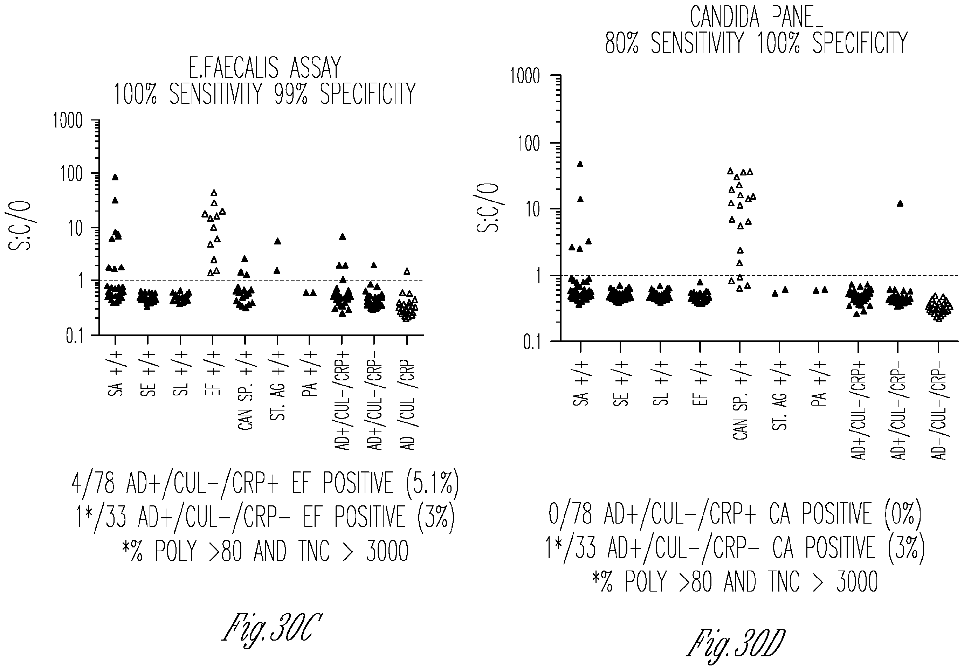

**FIG. 30A-30D** illustrate dot plots of the results of an example of the MID immunoassay. **FIG. 30A** is a dot plot of the results of immunoassay for *Staphylococcus aureus* (Panel A). FIG. 30B is a dot plot of the results of immunoassay for *Staphylocriccus epidermidis* (Panel B). **FIG. 30C** is a dot plot of the results of an immunoassay for *Enterococcus faecalis.* **FIG. 30D** is a dot plot of the results of an immunoassay for *Candida* panel (*Candida albicans* and *Candida parapsilosis*). SA(+/+) = *Staphylococcus aureus* α-defensin positive and culture positive SE(+/+) = *Staphylococcus epidermidis* α-defensin positive and culture positive. SL(+/+) = *Staphylococcus lugdunensis* α-defensin + and culture positive EF(t/+) = *Enterococcus faecalis* α-defensin positive and culture positive. Candida(+/+) = *Candida* species α-defensin positive and culture positive. StAg(+/+) *= Streptococcus agalactiae* α-defensin positive and culture positive. PA(+/+) = *Pseudomonas aeruginosa* α-defensin positive and culture positive AD + = samples testing α-defensin positive. AD - = samples testing α-defensin negative. Cul - = samples testing culture negative. CRP + = samples testing C-reactive protein positive. CRP- = samples testing C-reactive protein negative. Poly = percentage of polymorphonuclear neutrophil cells. TNC = total nucleated cell count.

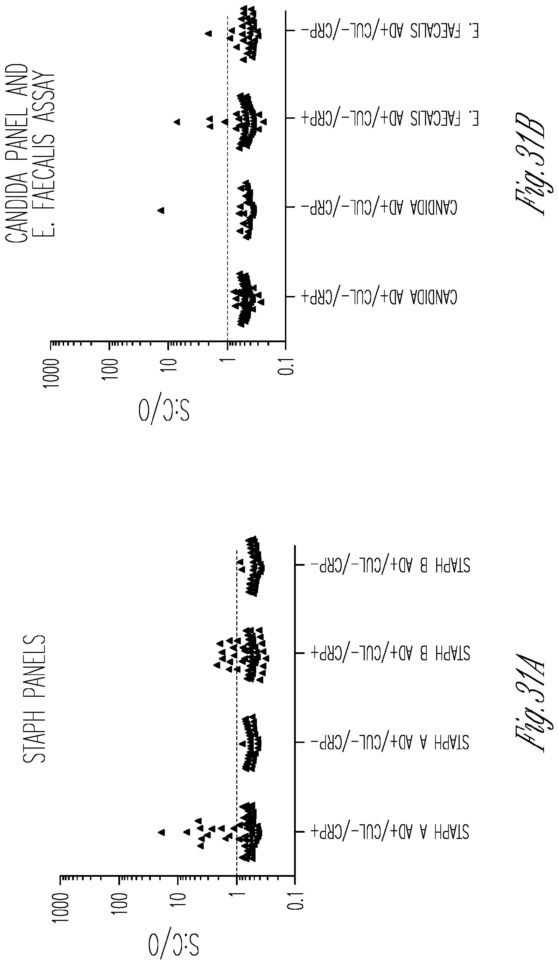

**FIG.31A-31** **B** illustrates dot plots of the results of an example of the MID immunoassay. FIG. 31is a dot plot of the results of immunoassay for *Staphylococcus aureus* (Panel A) and *Staphylococcus epidermidis* (Panel B) for synovial fluid cultures previously testing α-defensin positive and culture negative (+/-), and also testing either positive for C-reactive protein (CRP +) or negative for C-reactive protein (CRP-). **FIG. 31B** is a dot plot of the results of immunoassay for *Candida* Panel (*Candida albicans* and *Candida parapsilosis*) and *Enterococcus faecalis* for synovial fluid cultures previously testing α-defensin positive and culture negative (+/-), and also testing either positive for C-reactive protein (CRP +) or negative for C-reactive protein (CRP-).

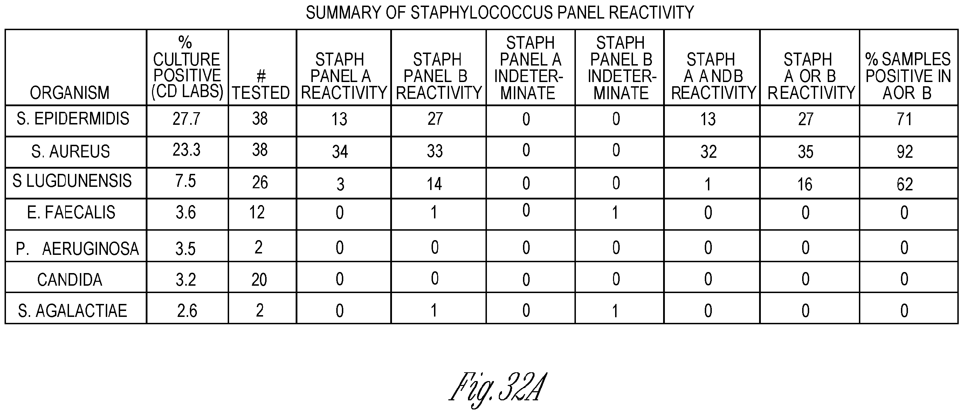

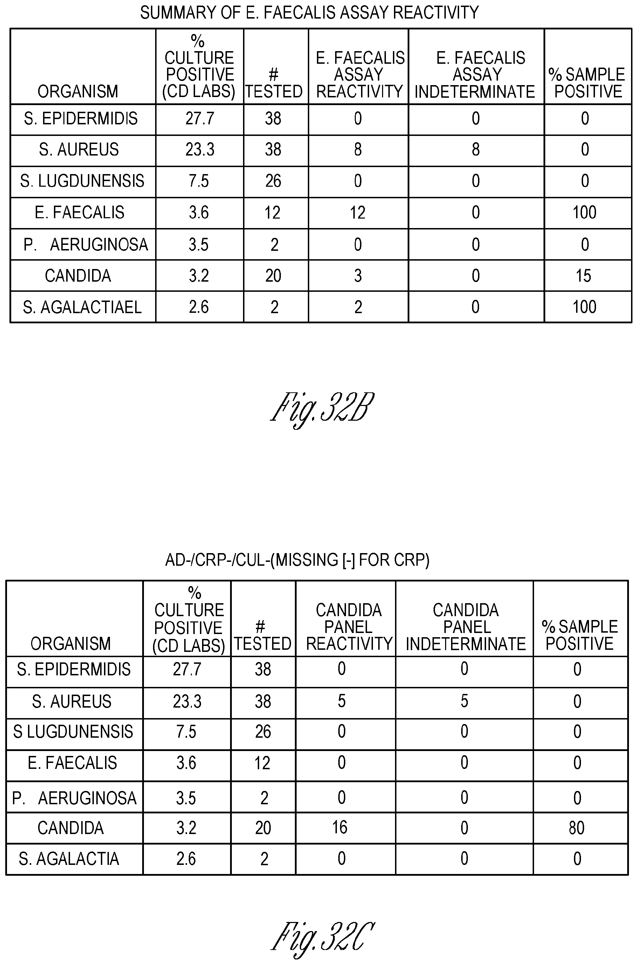

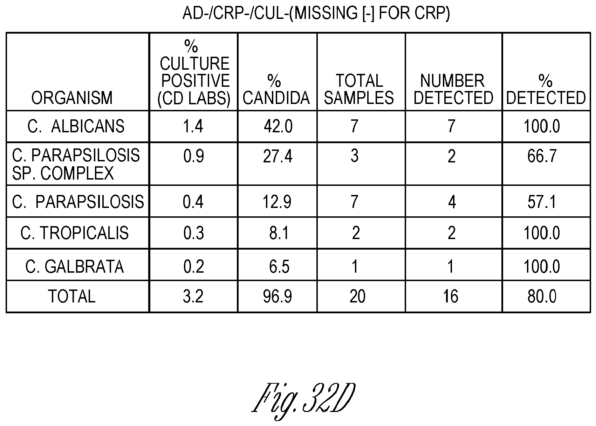

**FIG. 32A-32D** illustrate a series of tables of the summary of the results of the MID immunoassay of **FIGS. 25A-25H** and **26A-26R.** **FIG. 32A** is a summary table of the results of synovial fluid reactivity in the *Staphylococcus aureus* (Panel A) assay and *Staphylococcus epidermidis* (Panel B) assay. **FIG. 32B** is a summary table of the results of synovial fluid sample reactivity in the *Enterococcus* faecalis assay **FIG. 32C** is a summary table of the results of synovial fluid reactivity in the *Candia* Panel assay. **FIG. 32D** is a summary table of the results of synovial fluid sample reactivity of the *Candida* Panel across several species of *Candida.* AD- = α-defensin negative Cul- = culture negative CRP- = C-reactive protein negative. Staphylococcus represent 65% of all culture positive *(S. aureus, S. epidermidis* and S. *lugdenensis* account for 90% of all Staphylococcus PJI. Protein G in *Streptococcus agalactiae* is known to result in higher signal Staph Panel A Specificity = 98.1% (101/103 AD-/Cul-/CRP-); 98.6% (137/139 AD-/Cul-/CRP-, +non-Staph species). Staph Panel B Specificity = 100% (103/103 AD-/Cul-/CRP-); 100% (139/139 AD-/Cul-/CRP-, + non-Staph species). Staph Panel A + Staph Panel B Specificity: 99% (204/206 AD-/Cul-/CRP-), 98 9% (275/278 AD-/Cul-/CRP-, + non-Staph species). *Enterococcus faecalis* Specificity = 99.0% (102/103 AD-/Cul-/CRP-); 96.4% (134/139 AD-/Cul-/CRP-, + non-E. faecalis species). Candida Panel Specificity = 100% (103/103 AD-/Cul-/CRP-); 100% (139/139 AD-/Cul-/CRP-, + non-Candida species).

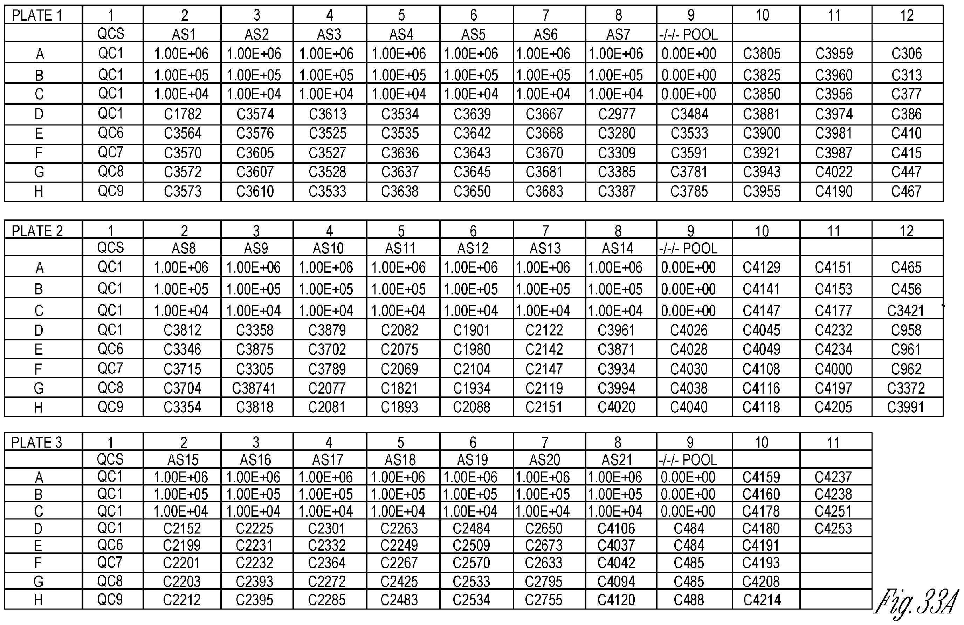

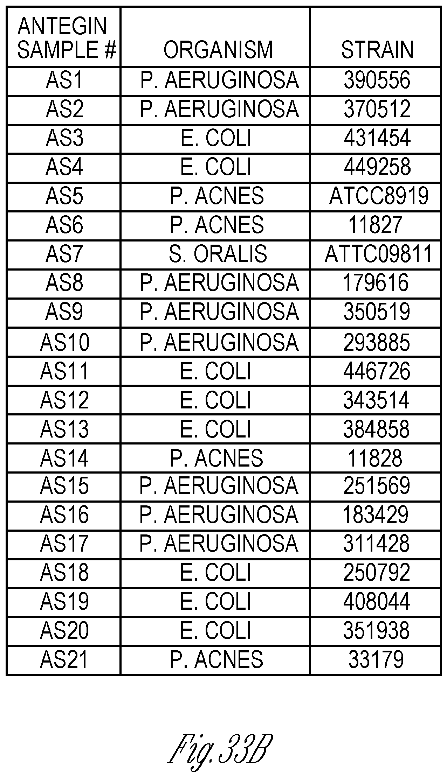

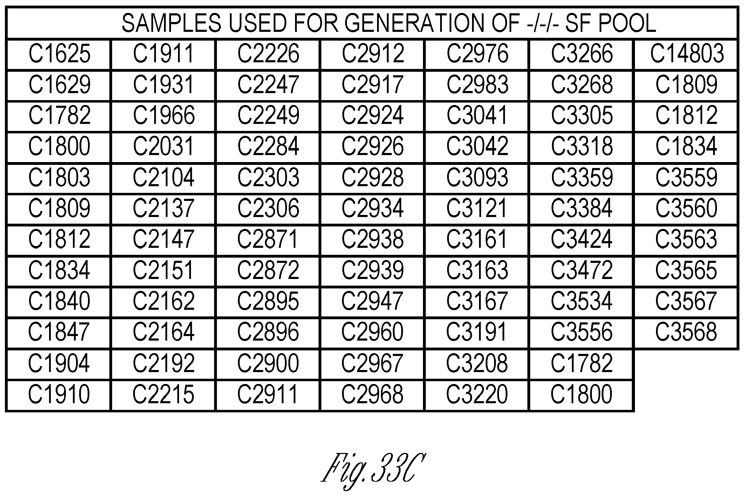

**FIG. 33A-33C** illustrate data of an example of a MID immunoassay for *Pseudomonas aeruginosa, Escherichia coli, Propionibacterium acnes,* and *Streptococcus mitis.* FIG. 33A is a plate map of synovial fluid samples. **FIG. 33B** is a table of microorganism strains used to spike synovial fluid samples in the immunoassay **FIG. 33C** illustrates a table of individual synovial fluid samples previously testing α-defensin negative, culture negative and C-reactive protein negative; (-/-/-) samples are pooled for use in the immunoassay testing.

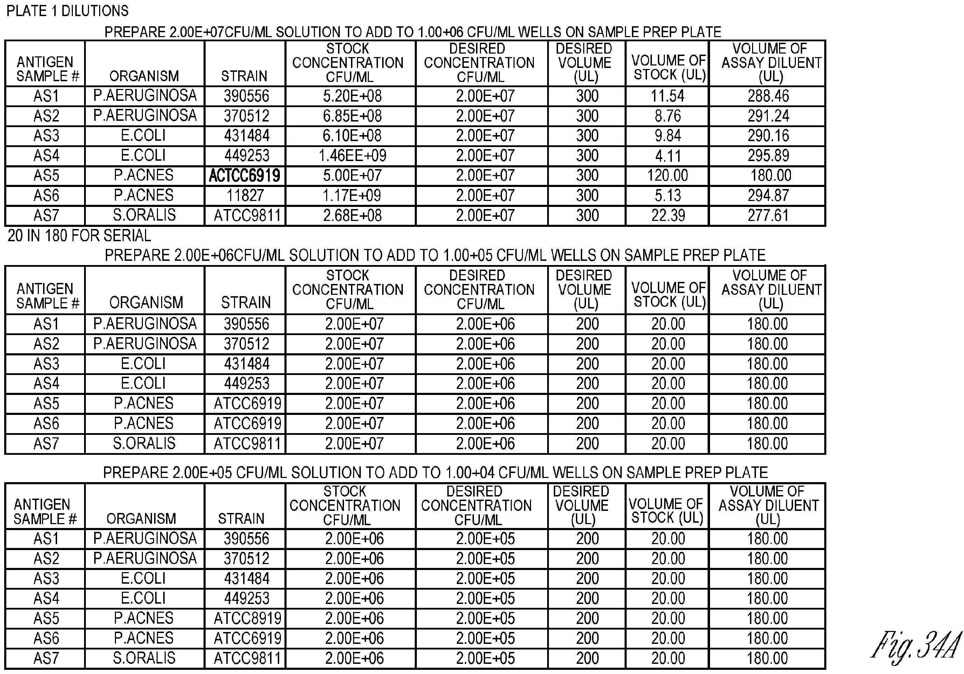

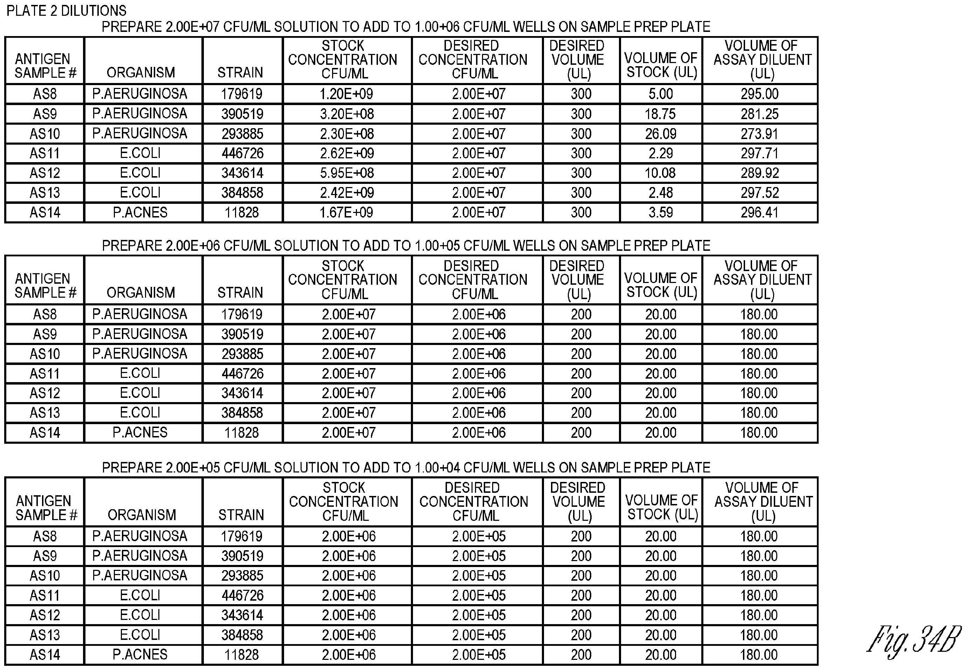

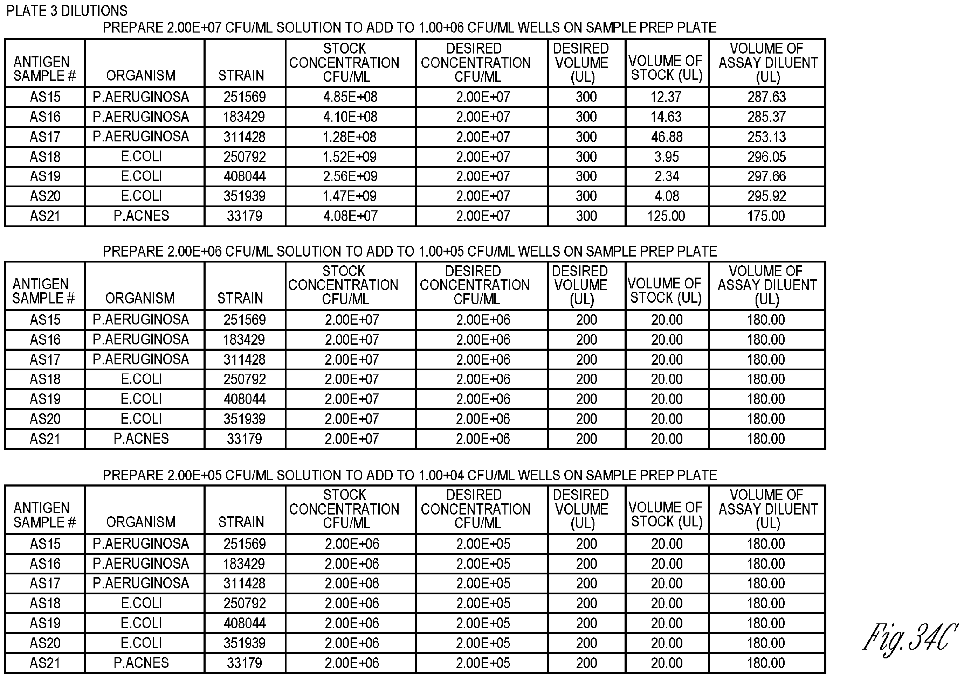

**FIG. 34A-34C** illustrate a series of tables of microorganisms from **FIG. 33B** used to spike the (-/-/-) pool of synovial fluid samples of **FIG. 33C****.** **FIG. 34A** is a table of microorganism dilutions for Plate 1 of **FIG. 33A****.** **FIG. 34B** is a table of microorganism dilutions for Plate 2 of **FIG. 33B****.** **FIG. 34C** is a table of microorganism dilutions for Plate 3 of **FIG. 33C****.**

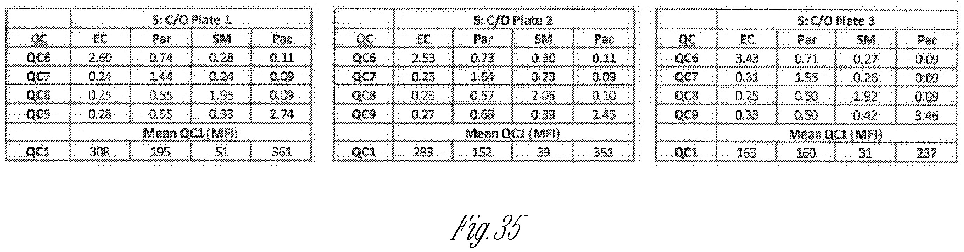

**FIG. 35** illustrates a table of the results of the MID immunoassay of **FIGS. 33A** for control samples of Plates 1, 2 and 3.

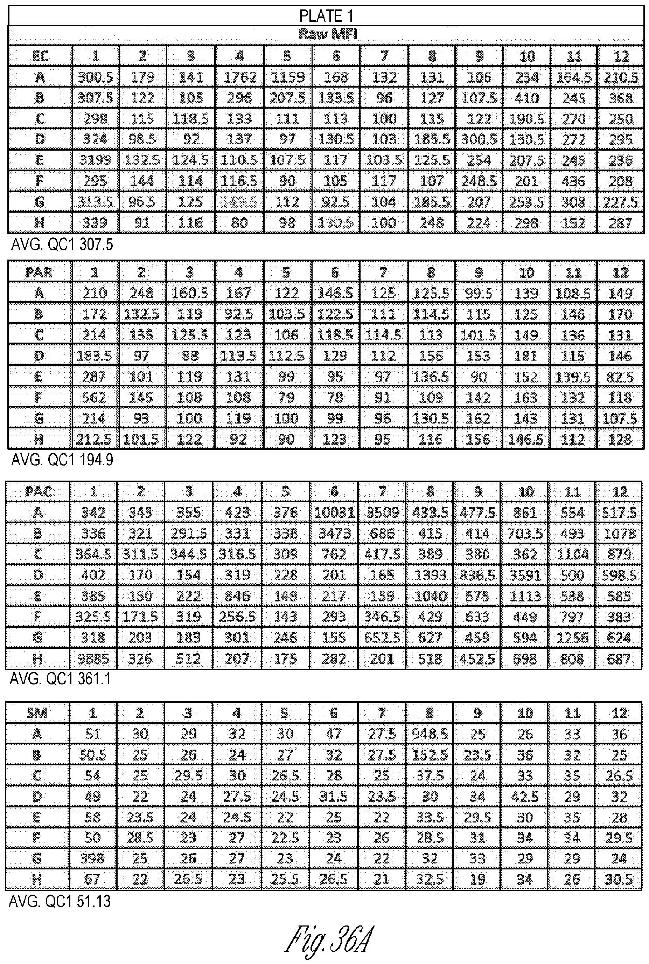

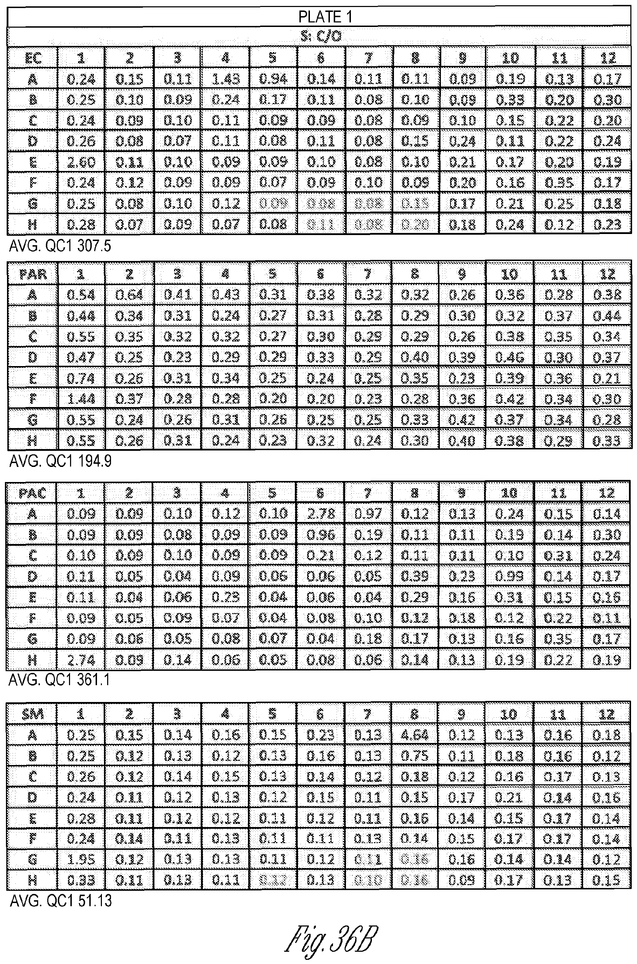

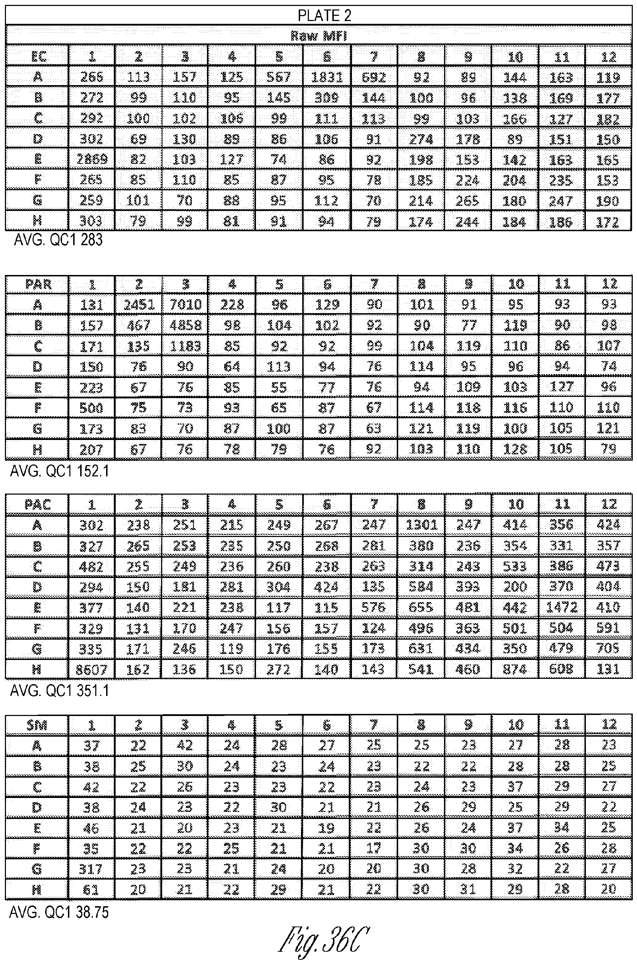

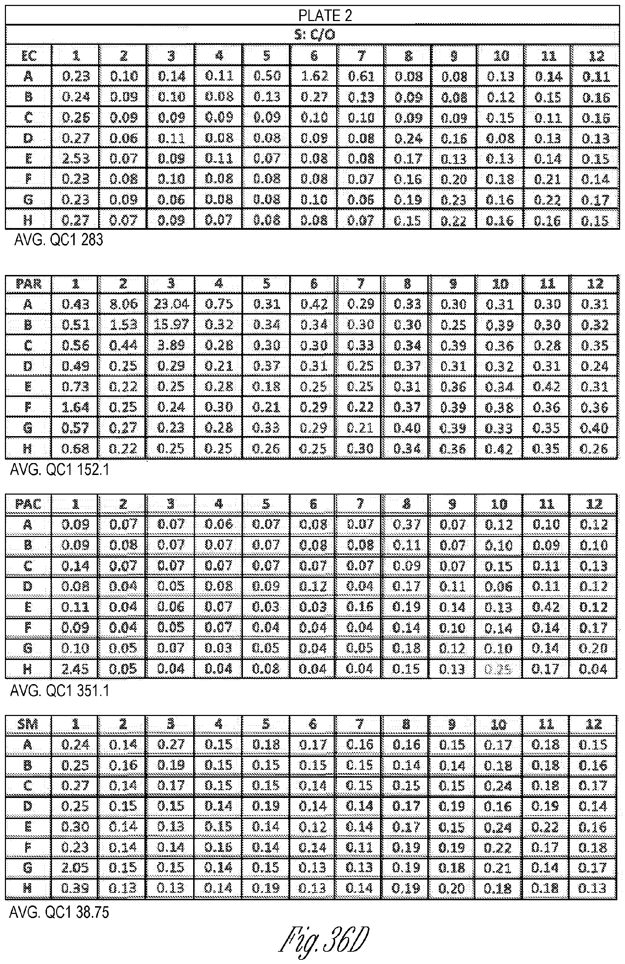

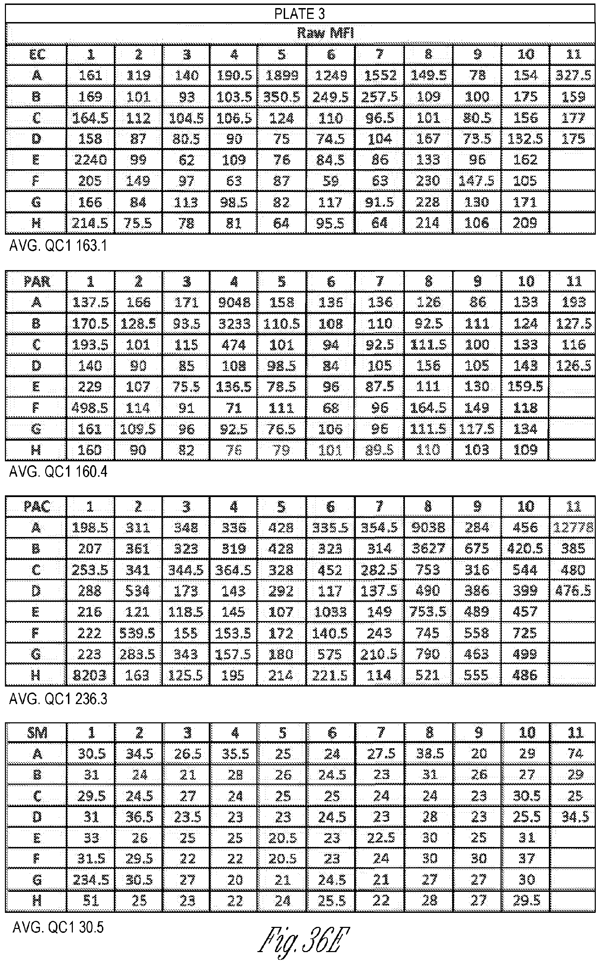

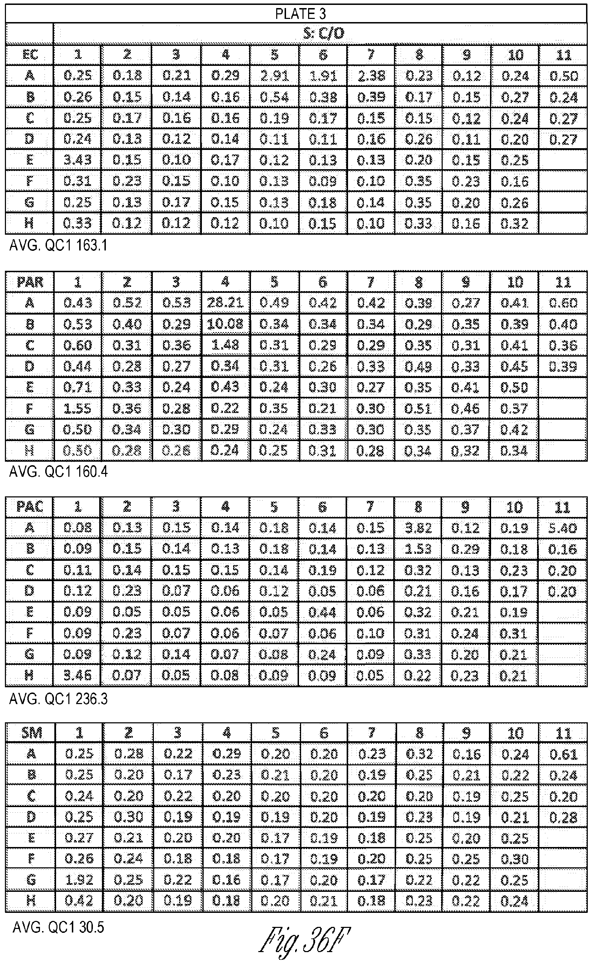

**FIG. 36A-36C** illustrate a series of tables of results from the MID immunoassay of **FIGS. 33A****.** MFI = median fluorescent intensity. S/CO or S:/CO = signal:cut-off. EC = *Escherichia coli.* PAR = *Pseudomonas aeruginosa* PAC = *Propionibacterium acnes* SM = *Streptococcus mitis.*

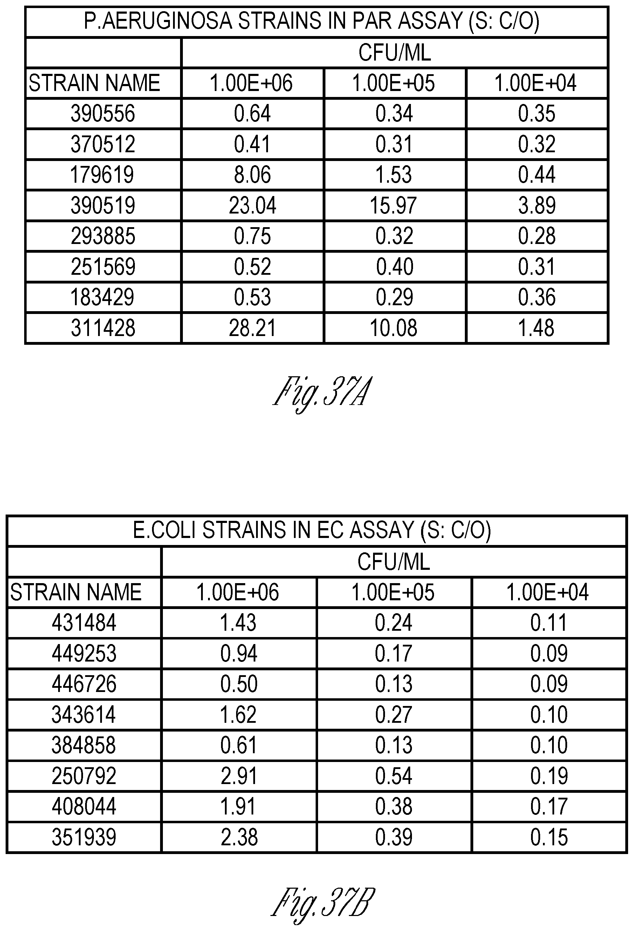

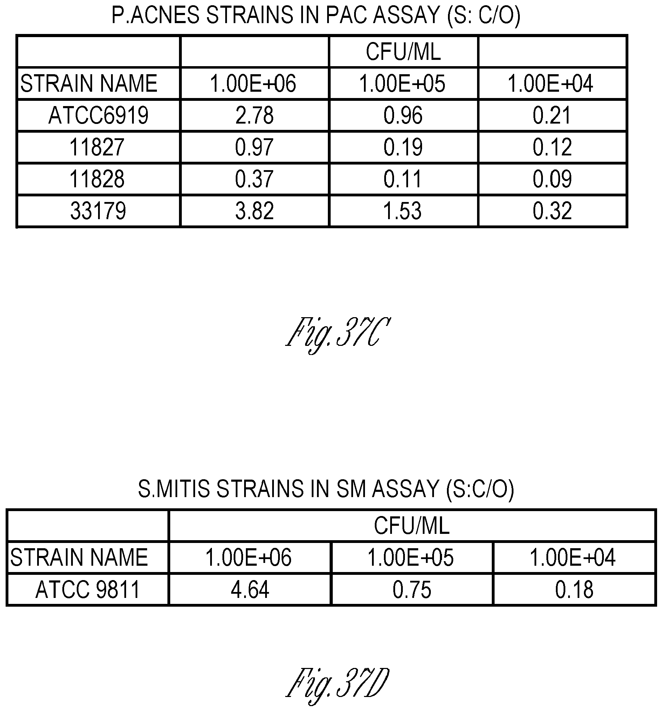

**FIGS. 37A-37D** illustrate a series of table of the results of the MID immunoassay of **FIGS. 36A-36D** **FIG. 37A** is a table of the results of the immunoassay for *Pseudomonas aeruginosa* **FIG. 37B** is a table of the results of the immunoassay for *Escherichia coli* **FIG. 37C** is a table of the results of the immunoassay for *Propionibacterium acnes.* **FIG. 37D** is a table summarizing the results of the immunoassay for *Streptococcus mitis.* S/CO or S:C/O = signal.cut-off. S:C/O > 1 is a positive result.

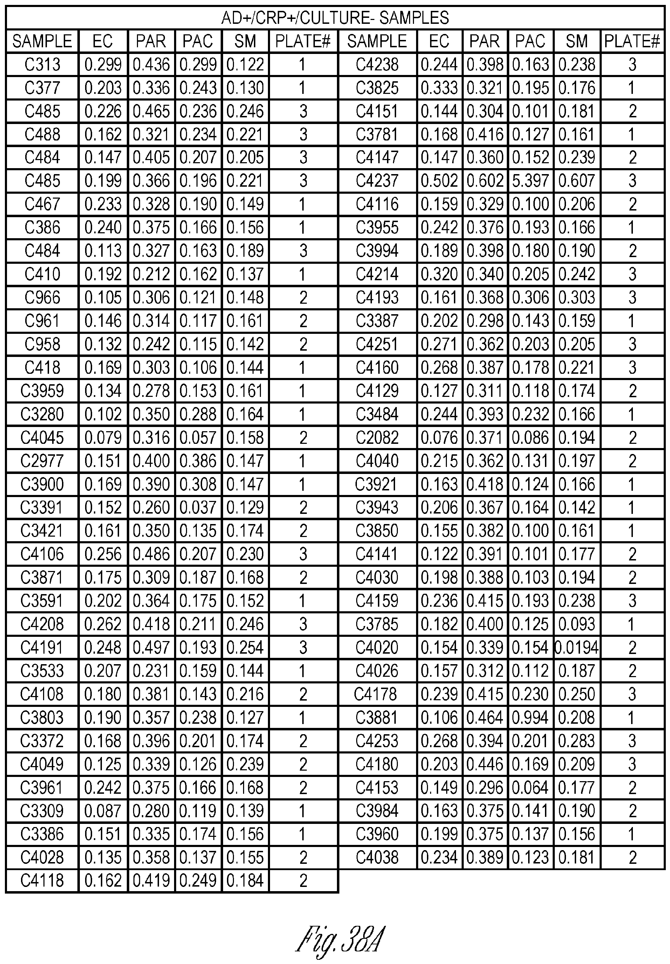

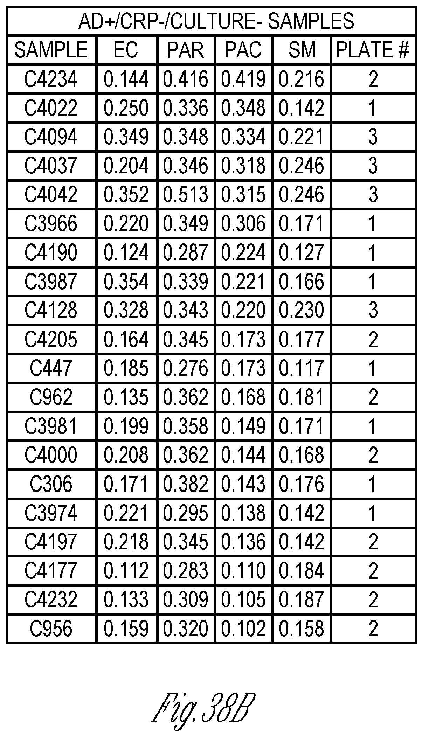

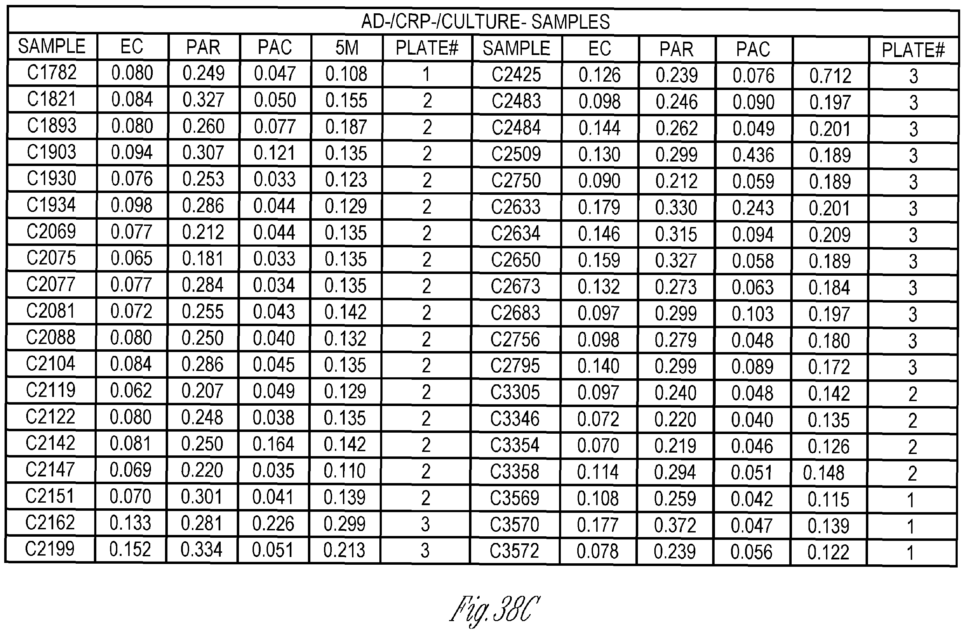

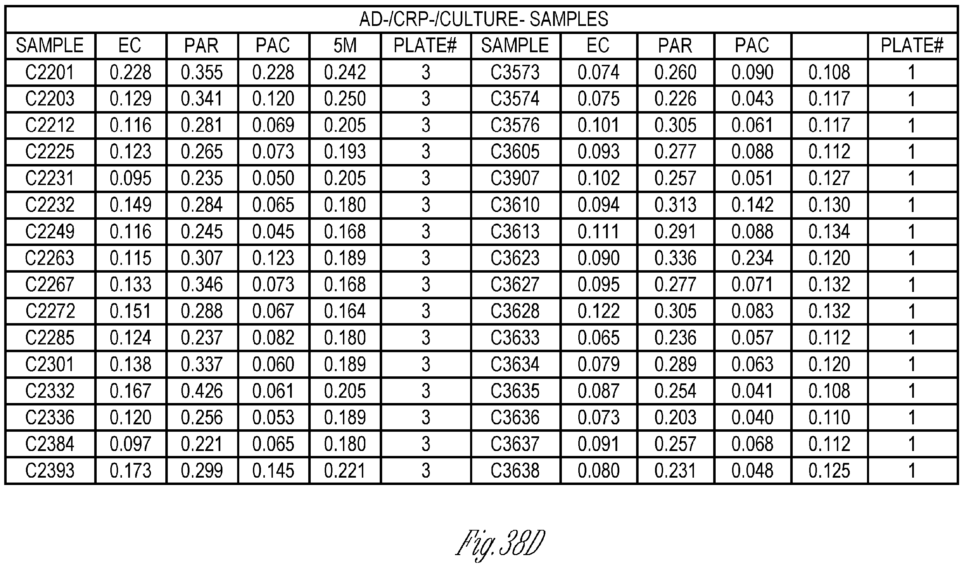

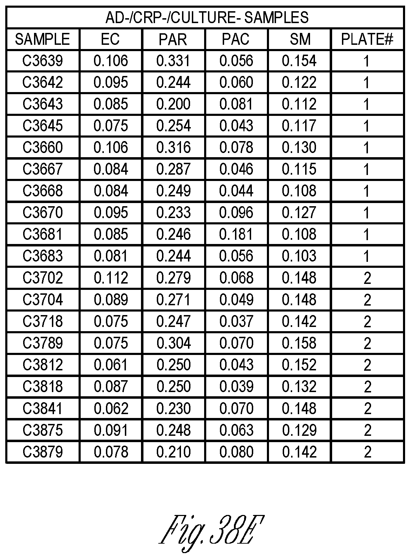

**FIGS. 38A-38E** illustrate a series of tables summarizing the results of a MID immunoassay of various synovial fluid samples. **FIG. 38A** is a table of immunoassay results for synovial fluid samples testing α-defensin positive, CRP positive, culture negative (AD+/ CRP+/ Cul-). **FIG. 38B** is a table of immunoassay results for synovial fluid samples testing α-defensin positive. CRP negative, culture negative (AD+/ CRP-/Cul-) **FIGS. 38C-38E** is a table of immunoassay results for synovial fluid samples testing α-defensin negative, CRP negative, culture negative (AD-/ CRP-/ Cui-).

### DETAILED DESCRIPTION

The present technology generally provides validated, simple and effective methods and compositions for preparing biological samples for direct detection and identification of one or more microorganisms in a biological sample The present technology also provides for compositions. methods, and systems for direct detection and identification of microorganisms in biological samples using immunoassay techniques. The microorganism can be a bacteria, fungus, virus, protozoa, or other unicellular organism; microorganisms in general may be referred to herein as "microbes" or "microbial"

In one example, there is provided a method of preparing patient sample for use in microorganism detection ("MID") immunoassay methods The patient sample can comprise a synovial fluid sample. In an example, the patient sample can include a synovial fluid sample from a patient suspected of having a native joint infection or PJI. In another example, the patient sample can include a synovial fluid sample from a patient that has previously has a synovial fluid sample cultured and the culture has tested negative for microbial growth ("Cul - "). In still another example, the patient sample can include a synovial fluid sample from a patient that tests human α-defensin positive ("AD +")

In an example, the patient sample can be from about 10µL to about 1 mL **(****FIG. 10****).** In another example, the patient synovial fluid sample can be from about 50 µL to about 500 µL. In yet another example the patient sample can be from about 100µL to about 250 µL. In a preferred example the patient sample is about 200 µL. The patient sample preparation protocol can employ either a 96 well plate format for high through-put screening of samples or can employ individual sample tubes

In one example, the patient sample can be mixed with a bacteriolytic enzyme at a concentration in the mixture from about 0. 1 pg/mL to about 10,000 µg/mL. In another example, the bacteriolytic enzyme can have a concentration of about 1 µg/mL to about 1,000 µg/mL in the mixture. In yet another example, the bacteriolytic enzyme can have a concentration of about 4 µg/mL to about 500 µg/mL in the mixture. In still another example, the bacteriolytic enzyme can have a concentration of about 20 µg/mL to about 400 µg/mL in the mixture. In a preferred example, the bacteriolytic enzyme can have a concentration of about 40 µg/mL to about 200 µg/mL in the mixture The bacteriolytic enzyme is configured to cleave a protein of a bacterial cell wall. Bacteriolytic enzymes can include, without limitation, any enzyme that can cleave a protein of a bacterial cell wall. Examples of bacteriolytic enzymes include glycosidases (lysozymes or muramidases and glucosaminadases), endopeptidases, amidases, autolysins, endolysins (e.g, endo β-N-acetylglucosaminidase or N-acetylmuramidase, endopeptidase, amidase, LysK), achromopeptidase. lysyl-endopeptidase. labiase, and lysostaphin

A major component of bacterial cells wall is peptidoglycan. Technology to detect peptidoglycan I lmmunetics, Boston, MA) in 217 synovial fluid samples established that almost all culture positive samples contain PGN and many AD positive/culture negative (+/-) samples also contain PGN (FIG. 21). In an example, the bacteriolytic enzyme can include lysostaphin (FIGS. **9A-9B**, 11A-11B, and 12A-12B). Lysostaphin is an endopeptidase that cleaves polyglycine cross-links of peptidoglycan in Gram-positive bacterial cell walls of *Staphylococci.* In one example, lysostaphin can be at a concentration of about 4 µg/mL to about 500 µg/mL in the mixture. In a preferred example, the lysostaphin is at a concentration of about 20 µg/mL to about 40 µg/mL in the mixture. In another example, the bacteriolytic enzyme can include lysozyme **(****FIGS. 14A-14C**. 15A-15C. 16A-16C, 17A-17C, and 18A-18C). Lysozyme is a glycoside hydrolase that catalyzes the hydrolysis of 1.4-beta-linkages between N-acetylmuramic acid and N-acetyl-D-glucosamine residues in peptidoglycan. In one example, the lysozyme can be at a concentration of about 10 µg/mL to about 5,000 µg/mL in the mixture In a preferred example, the lysozyme is at a concentration from about 100 µg/mL to about 500 µg/mL in the mixture In yet another example, the bacteriolytic enzyme can comprise lysostaphin and lysozyme at any combination of concentrations as provided above Without being bound by any particular theory, it is believed that use of one or more bacteriolytic enzymes can result in lysing of bacterial cells in the patient sample, allowing bacterial antigen to become more accessible and available for detection using a MID immunoassay.

In one example, the patient sample can be mixed with a protease capable of cleaving a bond in a native protein of the patient sample In one example, the protease in the mixture can be at a concentration from about 0.1µg/mL to about 5,000 µg/mL in the mixture. In another example, the protease can be at a concentration from about 10 µg/mL to about 1,000 µg/mL in the mixture In still another example, the protease can be at a concentration from about 20 µg/mL to about 500 µg/mL in the mixture In a preferred example, the patient sample is a synovial fluid sample. Synovial fluid includes glycoproteins, e.g., alpha-1-acid glycoprotein (AGP), alpha-1-antitrypsin (A1AT). lubricin, and hyaluronic acid. Hyaluronic acid is the primary component of synovial fluid (3-4 mg/ml) contributing to synovial fluid viscosity. In a preferred example, the protease comprises hyaluronidase. Hyaluronidase catalyses the hydrolysis of the 1-4 bond between N-acetyl-D-glucosamine and D-glucuronic acid in hyaluronic acid In one example, the hyaluronidase is at a concentration of about100 µg/mL to about 1000 µg/mL in the mixture. In a preferred example, the hyaluronidase is at a concentration of about 500 pg/mL in the mixture

In yet another example, the protease comprises trypsin (FIG. 21). Trypsin cleaves peptide chains primarily at the carboxyl side of the amino acids lysine or arginine, except when either is followed by proline. In still another example, the protease comprises hyaluronidase and trypsin. In a preferred example, the hyaluronidase or trypsin is at a concentration of about 200 µg/mL to about 500 µg/mL The use of one or more proteases capable of acting on a native protein of the patient sample can reduces viscosity of the patient sample, allows more accurate pipetting of the sample, improves sample homogeneity. and potentially frees-up microbial antigen in the patient sample. In a preferred example, the patient sample is mixed with a bacteriolytic enzyme and a protease to a native protein in the patient sample In another preferred example, the patient sample is mixed with lysostaphin or lysozyme, and hyaluronidase or trypsin.

In one example, a detergent can be added to the mixture of the patient sample and the bacteriolytic enzyme In another example, a detergent can be used to prepare the bacteriolytic enzyme before it is mixed with the patient sample. In still another example a detergent can be used to prepare the protease before it is mixed with the patient sample. In another example, the detergent can include a buffer such as phosphate buffered saline or Tris. In one example the detergent can be at a concentration from about 0.1% to about 10% in the mixture In a preferred example, the detergent can be at a concentration of about 0.5% to about 1.0% in the mixture **(****FIGS. 3A-3C****).** Detergent molecules allow the dispersion (miscibility) of water-insoluble, hydrophobic compounds into aqueous media, including the extraction and solubilisation of membrane proteins

In one example, detergent can comprise one or more non-ionic detergents, including, but not limited to, n-octyl-p-D-glucopyranside, n-octyl-p-D-maltoside. ZWITTERGENT 3 14, deoxycholate; n-Dodecanoylsucrose; n-Dodecyl-p-D-glucopyranoside; n-Dodecyl-p-D-maltoside; n-Octyl-p-D-glucopyranoside; n-Octyl-p-D-maltopyranoside: n-Octyl-(3-D-thioglucopyranoside; n-Decanoylsucrose; n-Decyl-p-D-maltopyranoside; n-Decyl-p-D-thiomaltoside: n-Heptyl-(3-D-glucopyranoside; n-Heptyl-(3-D-thioglucopyranoside; n-Hexyl-p-D-glucopyranoside; n-Nonyl-p-D-glucopyranoside; n-Octanoylsucrose; n-Octyl-p-D-5-glucopyranoside; n-Undecyl-p-D-maltoside: AP0-10, AP0-12; Big CHAP, Big CHAP. Deoxy: BRIJ^{®} 35; C12E5; Ci2E_{6:} C^Es, C12E9; Cyclohexyl-n-ethyl-p-D- maltoside, Cyclohexyl-n-hexyl-p-D-maltoside, Cyclohexyl-n-methyl-p-D-maltoside; Digitonin; ELUGENT^{™}; GENAPOL^{®} C-100; GENAPOL^{®} X-080; GENAPOL^{®} X-100; HECAMEG; MEGA-10; MEGA-8; MEGA-9; NOGA; NP-40; PLURONIC^{®} 10 F-127; TRITON^{®} X-100; TRITON^{®} X-1 14, TWEEN^{®} 20; or TWEEN^{®} 80. In a preferred example, the detergent comprises Triton^{®} X-100, Tween^{®} 20, or a combination thereof.

In another example, the detergent can comprise an ionic detergent, including, but not limited to BATC, Cetyltrimethylammonium Bromide, Chenodeoxycholic Acid, Cholic Acid, Deoxycholic Acid, Glycocholic Acid, Glycodeoxycholic Acid, Glycolithocholic Acid, Lauroylsarcosine, 15 Taurochenodeoxycholic Acid, Taurocholic Acid, Taurodehydrocholic Acid, Taurolithocholic Acid, Tauroursodeoxycholic Acid, and TOPPA. Zwitterionic detergents can also be used with the compositions and methods of the invention, including, but not limited to, amidosulfobetaines, CHAPS, CHAPSO, carboxybetaines, and methylbetaines. In still another example, the detergent can comprise an anionic detergent, including, but not limited to, e.g. SDS, N- lauryl sarcosine, sodium deoxycholate, alkyl-aryl sulphonates, long chain (fatty) alcohol sulphates, olefine sulphates and sulphonates, alpha olefine sulphates and sulphonates, sulphated monoglycerides, sulphated ethers, sulphosuccinates, alkane sulphonates, phosphate esters, alkyl isethionates, and sucrose esters.

The patient sample is mixed with the bacteriolytic enzyme, the detergent, and optionally the protease, and may be incubated for a period of time and at a temperature to permit the bacteriolytic enzyme, and/or the protease, and/or the detergent, or a combination of any of the foregoing, to act on the patient sample. In an example, the incubation can be at a temperature between about 4°C and about 37°C. In a preferred example. the incubation is at a temperature between about 25°C and about 37°C. In another preferred example, the incubation is at a temperature of about 37°C In some examples, the incubation can be for a period of time from about 1 minute to about 24 hours or longer. In other examples, the incubation can be for a period of time from about 5 minutes to about 12 hours. In still other examples the incubation can be for a period of time from about 5 minutes to about 2 hours. In a preferred example, the incubation can be for a period of time from about 5 minutes to about 60 minutes. In another preferred example, the incubation can be for period of time from about 5 minutes to about 30 minutes.

After mixing, and in certain examples after optionally incubating the mixture, the mixture is heated for period of time. The mixture is heated to a temperature greater than 70°C. In another preferred example, the mixture can be heated to a temperate of at least 95°C. In an example, the mixture can be heated for a period of time from about 30 seconds to about 30 minutes. In still other examples, the mixture can be heated for a period of time from about 1 minute to about 15 minutes. In still other examples, the mixture can be heated for a period of time from about 2 minutes to about 10 minutes In a preferred example, the mixture can be heated for a period of about 5 minutes. In a preferred example, the mixture can be heated to at least about 95°C for 5 minutes. In an example, the mixture can be heated in a water bath. Without being bound by any particular theory, it is believed that heating the mixture can lyse cells, can increase antigen signal in MID immunoassays compared to non-boiled samples and can inhibits non-specific interactions such as those from heterophiles (e.g., rheumatoid factor) that can cause false positive results.

In another example, after heating or boiling, the mixture can be centrifuged to separate aggregated and /or precipitated biological material from a supernatant containing microbial antigen. In one example, centrifugation can be at a force between 1,000 g and about 25,000 g. In another example, the centrifugation can be at a force between about 3,000 g and about 20,000 g. In yet another example, the centrifugation can be at a force between about 3,700 g and about 18,000 g. The centrifugation can be for a period of time between about 1 minute and about 60 minutes. In another example the centrifugation can be for a period of time between about 5 minutes and about 45 minutes. In still another example, the centrifugation can be for a period of time between about 10 minutes and about 40 minutes. In a preferred example, the sample can be centrifuged for a period of time of about 30 minutes. In a preferred example, the mixture can be centrifuged at a force of about 18.000 g for a period of about 10 minutes. In another preferred example, the mixture can be centrifuged at a force of about 3,700 g for a period of about 20 minutes to 30 minutes The supernatant resulting from centrifugation of the mixture can be used for further processing, including, without limitation, in an immunoassay.

### Immunoassays in General

In one example, the methods of present patent document can be performed using various immunoassay formats, which are well known in the art. Immunoassays are binding assays involving binding between antibodies and antigen. Many types and formats of immunoassays are well-known and all can be used for detection and identification of microbes in biological samples. Examples of immunoassays include enzyme linked immunosorbent assays ("ELISA"), enzyme linked immunospot assay ("ELISPOT"), radioimmunoassays ("RIA"), radioimmune precipitation assays ("RIPA"), immunobead capture assays, Western blotting, dot blotting, gel-shift assays, flow cytometry. protein arrays, antigen arrays, antibody arrays, multiplexed bead arrays, magnetic capture, in vivo imaging, fluorescence resonance energy transfer ("FRET"), fluorescence recovery/localization after photobleaching ("FRAP/FLAP"), a sandwich immunoassay. a competitive immunoassay, an immunoassay using a biosensor, an immunoprecipitation assay, an agglutination assay, a turbidity assay, a nephelometric assay, immunoPCR, Quanterix, Singulex, AlphaLISA, Siscapa, Luminex^{®}, Singulex Brenna^{®} immunoassay, TR-FRET, Meso-scale discovery (MSD). lateral flow immunochromatographic device, automated magnetic particle assay, fluorescent polarization, chemiluminescence, electrochemiluminescence, etc.

In general, immunoassays involve contacting a biological sample suspected of containing a molecule of interest (an analyte, such as the microbial antigens in the prepare patient sample) with an antibody to the analyte, or contacting an antibody to a analyte with a molecule that can be bound by the antibody, as the case may be, under conditions effective to allow the formation of immune complexes. Contacting a biological sample with the antibody to the analyte or with the molecule that can be bound by an antibody to the analyte under conditions for a period of time sufficient to allow the effective formation of immune complexes (primary immune complexes) is generally a matter of contacting the antibody and the sample and incubating the mixture for a period of time long enough for the antibodies to form immune complexes, i.e.. to bind to, any antigens present in the sample to which the antibodies can bind. In many forms of immunoassay, the sample-antibody composition can then be washed to remove any non-specifically bound antibodies or unbound proteins, allowing only those antibodies specifically bound in the primary immune complexes to be detected.

Immunoassays can include methods for detecting or quantifying the amount of analyte (such as the disclosed microbial antigens or their antibodies) in a biological sample, which methods generally involve the detection or quantification of immune complexes formed during the binding process. In general, the detection of immune complex formation is well known in the art and can be achieved through the application of numerous approaches. These methods are generally based upon the detection of a label or tag, such as any radioactive, coloured, chemiluminescent, fluorescent biological or enzymatic tag or any other known label. See, for example, U.S. Pat. Nos. 3,817,837; 3,850,752; 3,939,350; 3,996,345; 4.277,437; 4,275,149 and 4,366,241, for teachings regarding immunodetection methods and labels.

As used herein, a label can include a fluorescent dye, a member of a binding pair, such as biotin/streptavidin, a metal (e.g., gold), or an epitope tag that can specifically interact with a molecule that can be detected, such as by producing a colored substrate or fluorescence. Substances suitable for detectably labeling proteins include fluorescent dyes (also known herein as fluorochromes and fluorophores) and enzymes that react with colorimetric substrates (e.g., horseradish peroxidase). The use of fluorescent dyes is generally preferred in the practice of the methods disclosed herein.

There are two main types of immunoassays, homogeneous and heterogeneous, each of which may be used in the methods of detecting and identifying microbes described herein. In homogeneous immunoassays, both the immunological reaction between an antigen and an antibody and the detection are carried out simultaneously in a homogeneous reaction. Heterogeneous immunoassays include at least one separation step between bound and unbound label, which allows the differentiation of reaction products from unreacted reagents A variety of immunoassays can be used to detect one or more of the microorganisms disclosed herein

ELISA and Luminex^{®} bead-based array platforms are examples of sandwich immunoassays that can be used in the methods disclosed herein A "sandwich" immunoassay, in which the antigen being assayed is held between two antibodies, is a preferred format for the methods disclosed herein. In this method, a solid substrate is first coated with a solid phase antibody. The test sample, containing the antigen (e.g., a diagnostic protein), or a composition containing the antigen, such as a synovial fluid sample from a subject of interest, can then be added and the antigen can be allowed to react with the substrate-bound antibody. Any unbound antigen can be washed away. A known amount of enzyme-labeled detection antibody can then allowed to react with the bound antigen. Any excess, unbound enzyme-linked antibody can be washed away. The substrate specific for the enzyme used in the assay can then be added and the reaction between the substrate and the enzyme produces a color change. The amount of visual color change is a direct measurement of specific enzyme-conjugated bound antibody, and consequently directly proportional to the amount of antigen present in the sample tested.

In many immunoassays, as described elsewhere herein, detection of antigen is made with the use of antigen-specific antibodies as detection molecules However, immunoassays and the systems and methods of the present invention are not limited to the use of antibodies as detector molecules. Any substance that can bind or capture the antigen within a given sample may be used. Aside from antibodies, suitable substances that can also be used as detector molecules include but are not limited to enzymes, peptides, proteins, receptors, and nucleic acids Further, there are many detection methods known in the art in which the captured antigen may be detected In some assays, enzyme-linked antibodies produce a color change. In other assays, detection of the captured antigen is made through detecting fluorescent, luminescent, chemiluminescent, or radioactive signals. The system and methods of the current invention is not limited to the particular types of detectable signals produced in an immunoassay.

### MID Immunoassay

In one example, a patient sample, prepared as described above and in Experimental Example 1 through Experimental Example 5, can be assayed for the presence of one or more microorganisms In an example, the microorganism can include one or more of *Staphylococcus aureus, Staphylococcus epidermidis, Enterococcus faecalis, Enterococcus faecium, Pseudomonas aeruginosa, Staphylococcus lugdunensis, Staphylococcus warneri, Staphylococcus capitis, Staphylococcus caprae. Streptococcus mitis, Streptococcus oralis, Streptococcus agalactiae, Streptococcus anginosus, Streptococcus gordonii, Eschericia coli, Propionibacterium acnes, Proteus mirabilis, Granulicatella adjacens, Acinetobacter baumannii, Abiotrophia defective, Corynebacterium striatum, Coynebacterium minutissimum, Parvimonas micra, Candida parapsilosis, Candida tropicalis. Candida glabrata,* and *Candida albicans* In an example, the assay is an immunoassay configured to detect the presence of one or more microorganisms in the prepared patient sample

In a preferred example, the microorganism can include, and the immunoassay is configured to detect, the presence of one or more of *Staphylococcus aureus, Staphylococcus epidermidis, Enterococcus faecalis, Enterococcus faecium, Pseudomonas aeruginosa, Staphylococcus lugdunensis, Staphylococcus warneri, Staphylococcus capitis, Staphylococcus caprae, Streptococcus mitis, Streptococcus oralis, Streptococcus agalactiae, Eschericia coli, Propionibacterium acnes, Candida parapsilosis, Candida tropicalis. Candida glabrata,* and *Candida albicans,* microorganisms in the prepared patient sample.

Antibodies to a microorganism of interest, for example, antibody directed against whole bacterial cells, are also commercially available [Meridian Life Sciences]. In one example, the commercially-available antibody can be labeled with biotin and used in an ELISA sandwich immunoassay to detect microorganisms in a synovial fluid sample (FIGS. 20A-20B). In another example, commercially-available antibody can be conjugated to fluorescent beads or microspheres, e g. Luminex^{®} beads, and used in a sandwich immunoassay to detect the presence of microorganisms in a patient sample

In an example, isolated strains of one or more microorganisms of interest, e.g. as those microorganisms identified above as possible infectious agents in native joint infection and PJI, can be obtained from commercial sources (e.g., from ATTC). Alternatively, or in addition, to commercially available microorganisms, one of ordinary skill in the art can readily prepare isolated microbial cultures from a patient sample, such as a sample from a patient having a confirmed infection. The patient sample can be grown in culture to isolate one or more microorganism from the patient sample. In an example, a synovial fluid sample testing positive for microbes by automated microbial culture methods (Becton-Dickenson) can be streaked, a single colony-isolated, and submitted to species identification via automated biochemical methods (Vitek, Biomerieux) In another example, glycerol-stocks of each identified microbial culture can be stored as bacterial stocks for use in the development of immunoreagents and MID immunoassay development. In one example, the bacterial stock can be used for immunogen preparation for animal immunizations In another example, bacterial stock can be used as standard/calibrator/QC for antibody screening in MID immunoassays. In another example, bacterial stock can be used for large scale preparation of antigen to cross-link to resin for affinity purification purposes.

In an example, bacterial stock of one or more microorganisms of interest, such as those identified above as being potential infectious agents in native joint infection or PJI (whether isolated from a patient sample or obtained from commercial source) can be used to immunize an animal for the production of polyclonal antisera In an example, the microorganism can be grown in liquid culture media to saturation or log growth phase, and can then be used as the source of immunogen. In an example, an immunogen can be heat-killed prior to animal immunization. An example of a general immunization protocol can include a collection of a single pre-bleed sample prior to administering the target immunogen intravenously (IV). After a 21-day rest, animals can receive six single additional IV boosts 3-4 days apart A test bleed can be collected and evaluated to identify the level of immunological response to the target immunogen prior to entering the production scale-up phase. Thereafter, responding animals transition into the scale-up phase, where two production bleeds intercalated by a single IV boosts are collected and further evaluated in a direct bind assay (antigen as a capture) against the pre- and test bleeds. Serum from each production bleed from each animal immunized can be evaluated Consideration to pool or exclude production bleeds can include not only antibody titer, but also cross-reactivity of the production bleed to non-target pathogens. Serum master pools of qualified production bleeds (based on titer and cross-reactivity) can be prepared and then Protein A purified. In some examples, Protein A purified antibody can be subjected to a second purification round via affinity chromatography (purification against the immunogen coupled onto the resin). In another example, the Protein A purified antibody can be subjected to affinity purification with pH gradient fractionated elution of the antibody **(****FIG. 23A****).**

An example of an immunization schedule is in Table 1 below.

**Table 1.**

| **D a y** | **Days since last Immunization** | **Procedure** |
|---|---|---|
| **0** | 0 | Prcbleed |
| **0** | 0 | Initial inoculation with 0.25nd cells given IV |
| **2** | | |
| **1** | 21 | Boost with 0.5ml cells given IV |
| **2** | | |
| **4** | 3 | Boost with 1.0ml cells given IV |
| 2 | | |
| 8 | 4 | Boost with 1.5ml cells given IV |
| 3 | | |
| 1 | 3 | Boost with 2.0ml cells given IV |
| 3 | | |
| 5 | 4 | Boost with 2.5ml cells given IV |
| 3 | | |
| 8 | 3 | Boost with 3.0ml cells given IV |
| 4 | | |
| 2 | 4 | Test Bleed |
| 5 | | |
| 6 | 14 | Boost with 3.0 ml cells given IV |

| The production bleed cycle consists of: Initial boost followed by collection of two weekly production bleeds followed by a second boost. This cycle repeats every 21 days. | | |
|---|---|---|
| | 0 | Boost |
| | 7 | Production Bleed |
| | 14 | Production Bleed |
| | 21 | Boost |

In an example, purified anti-microorganism polyclonal antibodies can be used as reagents (e.g, capture reagents, detection reagents, or both) in MID immunoassays **(****FIG. 23B****)** for detection of microorganisms

In one example of a MID immunoassay, the assay can be an ELISA assay. In one example of an ELISA MID immunoassay, the purified antibody can be bound to a solid substrate In an example, the antibody can be bound to a solid substrate comprising a plate well or a tube. In another example of an ELISA MID immunoassay, the detector reagent can be Streptavidin-HRP

In one example, the MID immunoassay can be in a multiplex platform. In a preferred example, the multiplex assay can employ a solid substrate comprising carboxylated, optionally magnetic, microparticles (beads) which have been internally doped with different proportions of fluorescent dyes to create an array of multiple beads. Each bead is characterized by a unique spectrum signature, permitting simultaneous testing of multiple analytes in a single 96-microtiter plate well. The covalent coupling of antibody (capture reagents) to beads is a well-known process utilizing a standard 2-step NHS-EDC chemistry. Detailed instructions for forming antibody-bead complexes can be found in the xMAP Cookbook (3rd. Ed., Luminex)

In one example of a multiplex MID immunoassay, a patient sample and controls/standards can be mixed with the antibody-coated magnetic beads and incubated for a period of time. In an example, the sample and control/standard can be incubated with the antibody-coated beads for a period of between about 15 minutes and about 2 hours. In another example, the patient sample and controls/standards can be mixed with the antibody-coated magnetic beads and incubated for a period of time between about 30 minutes and 60 minutes. Incubation of the sample and controls/standards together with the antibody-beads can be at a temperature from about 18°C to about 38°C In a preferred example, incubation is at room temperature or about 20°C to about 25°C.

MID immunoassays can be designed in either a heterogeneous format, where washing steps can be performed either manually or automated, or alternatively or additionally in a homogeneous (no wash) protocol In an example, washing can be performed from 1 to 5 times. In a preferred example, washing is performed 3 times at each wash step. In an example, a cocktail of biotinylated detection antibodies to one or more of the microorganism (such as those referred to above as potential infectious agents in native joint infection or PJI) can be added to the patient sample and control/standard and antibody-coated magnetic beads after washing (if using a heterogeneous protocol), to complete the sandwich immune complex In another example, the MID immunoassay method can include one or more washes (heterogeneous protocol)/or no wash (homogenous protocol), followed by incubation with Streptavidin-Phycoerythrin (SAPE) as the reporter molecule. After a washing step (which can include between 1 and 5 washes) to remove unbound reporter, the beads can be resuspended, e.g., in a Sheath Fluid (Biorad) and the beads are read on a device capable of detecting fluorescence, e.g. Luminex^{®} 100/200 Each magnetic bead is characterized by a unique spectrum signature, thereby permitting identification of a microorganism associated with a particular signature. Association of phycoerythrin fluorescence with the fluorescence characteristic of the specific antibody-bead complex can indicate the identity of a particular microorganism in the patient sample Fluorescent intensity of the reporter dye is directly proportional to the amount of a particular microorganism in the sample.

### Immunoassay Compositions

The present document further provides immunoassay compositions for direct detection of microorganisms in biological samples, including patient samples In one example, a first portion of a purified polyclonal antibody can be coupled to a solid substrate, such as a plate well, a tube, or a fluorescent bead or microsphere. e g.. a Luminex^{®} bead. In a preferred example the bead or microsphere can be a magnetic bead or microsphere. For preparing capture reagents, each anti-microorganism antibody of the first portion of polyclonal antisera can be coupled to a Luminex^{®} bead having a different fluorescence. In an example, the primary antibody (bound to a solid substrate, e.g., a plate well, tube or a bead) can be directly labeled In another example, the antibodies can be provided in an amount of from about 0.001 µg to 100 µg, and more preferably about 0 01 µg to 10 µg In an example, for conjugating antibody to the beads, the concentration of antibody can range from about 1 µg/1 million beads to about 10 µg/1 million beads In another example, the concentration of detection antibody can range from about 0 1 µg/mL to about 10 µg/mL.

In an example, the antibody detection reagent is preferably a polyclonal antibody. A second portion of the purified polyclonal anti-microorganism antibody can be labelled with a biotin to provide a detection reagent. The fluorescent antibody-bead and biotinylated antibody comprise a pair of immunoassay reagents, respectively capture reagents and detection reagents, specific for the microorganism to which the polyclonal antibody was made A unique pair of capture reagent and detection reagent can be prepared for each microorganism to be detected by a sandwich immunoassay, such as a MID immunoassay. A unique panel of anti-microorgani sm reagents can be joined in a system for multiplex MID immunoassay. In an example, a panel can comprise capture reagents and/or detection reagents for at least two, pre-selected microorganisms. In another example, capture reagents for at least two, pre-selected microorganisms can be mixed together to form a mixture or cocktail of capture reagents for performing a multiplex MID immunoassay. In another example, detection reagents for at least two, pre-selected microorganisms can be mixed together to form a mixture or cocktail of detection reagents for performing a multiplex MID immunoassay.

In an example, the at least two microorganisms can be selected from the group consisting of *Staphylococcus aureus, Staphylococcus epidermidis, Enterococcus faecalis, Enterococcus faecium, Pseudomonas aeruginosa, Staphylococcus lugdunensis, Staphylococcus warneri. Staphylococcus capitis, Staphylococcus caprae. Streptococcus mitis, Streptococcus oralis, Streptococcus agalactiae, Streptococcus anginosus, Streptococcus gordonii, Eschericia coli, Propionibacterium acnes, Proteus mirabilis, Granulicatella adjacens, Acinetobacter baumannii, Abiotrophia defective, Corynebacterium striatum, Corynebacterium minutissimum, Parvimonas micra, Candida parapsilosis, Candida tropicalis, Candida glabrata,* and *Candida albicans.*

### Immunoassay Systems

Immunoassay systems are also disclosed herein. These systems can include, in separate containers polyclonal antibodies and/or monoclonal antibodies having binding specificity for the microorganisms suspected of infection in a biological sample. In one example, the system can include instructions for use and/or for interpretation of results. In another example, the instructions can include an algorithm for interpreting results of any immunoassay performed using the antibodies of the system.

In one example, the system can comprise polyclonal and/or monoclonal antibodies have binding specificity for a microorganism selected from the group consisting of *Staphylococcus aureus, Staphylococcus epidermidis, Enterococcus faecalis, Enterococcus faecium, Pseudomonas aeruginosa, Staphylococcus lugdunensis, Staphylococcus warneri, Staphylococcus capitis, Staphylococcus caprae, Streptococcus mitis, Streptococcus oralis, Streptococcus agalactiae. Streptococcus anginosus, Streptococcus gordonnii, Eschericia coli, Propionibacterium acnes, Proteus mirabilis, Gramulicatella adjacens, Acinetobacter baumannii, Abiotrophia defective, Corynebacterium striatum, Corynebacterium minutissimum, Parvimonas micra, Candida parapsilosis, Candida tropicalis, Candida glabrata,* and *Candida albicans.*

In one example, a system can include a set of preferred antibodies, either labeled or unlabeled, that are useful for the detection and identification of at least two microorganisms. In general, a system can comprise a capture reagent and a detection reagent. each of which is suitable for detecting the presence of a microorganism of interest.

In one example, system can be provided with instructions for use In an example, the instructions can include directions to prepare a patient tissue sample in accordance with any one of the examples described herein, including, without limitation. Experimental Examples 1-5. In certain examples, the system can include instructions for performing an immunoassay according to any of the examples discloses herein, including, without limitation, experimental Examples 5 and 6. In still another example, the system can include instructions directing a user to apply an algorithm to immunoassay data according to any of the examples disclose herein, including, without limitation, one or more of the algorithms of Experimental Examples 5 and 6. In another example, the system can comprise instructions wheiein the system can comprise polyclonal antibody capable of binding to at least one of *Staphylococcus aureus* and *Staphylococcus epidermidis* In another example, the system can be useful for providing indication of presence or absence of infection in a synovial fluid sample In still other examples, the system can be useful for detecting and identifying microorganisms in biological sample that has previously tested culture negative ("Cul -" ), i.e., when cultured the biological sample did not evidence the presence of any microorganism. In another example, the system can be useful for detecting and identifying microorganisms in a biological sample that has previously tested α-defensin positive ("AD+") and culture negative (Cul -). In yet other examples, the system is useful for providing a recommendation of whether or not revision surgery would be relevant for a patient

In some examples, the system can include a panel or a set of antibodies In some examples, the kit is an immunoassay kit as described previously herein. In some examples, panel or set of antibodies are configured to detect the presence of at least two microorganisms selected from the group consisting of *Staphylococcus aureus, Staphylococcus epidermidis, Enterococcus faecalis, Enterococcus faecium, Pseudomonas aeruginosa, Staphylococcus lugdunensis, Staphylococcus warneri, Staphylococcus capitis, Staphylococcus caprae. Streptococcus mitis, Streptococcus oralis, Streptococcus agalactiae. Streptococcus anginosus, Streptococcus gordonii, Escherichia coli, Propionibacterium acnes, Proteus mirabilis, Granulicatella adjacens, Acinetobacter baumannii, Abiotrophia defective, Corynebacterium striatum, Corynebacterium minutissimum, Parvimonas micra, Candida parapsilosis, Candida tropicalis, Candida glabrata.* and *Candida albicans*

In an example, the panel or set of antibodies can be configured to detect the presence of *Staphylococcus aureus* and *Staphylococcus epidermidis,* In other examples, the panel or sets of antibodies are configured to detect the presence of *Staphylococcus aureus, Staphylococcus epidermidis,* and at least one microorganisms selected from the group consisting of *Enterococcus faecalis, Enterococcus faecium, Pseudomonas aeruginosa, Staphylococcus lugdunensis, Staphylococcus warneri, Staphylococcus capitis, Staphylococcus caprae, Streptococcus mitis, Streptococcus oralis, Streptococcus agalactiae. Streptococcus anginosus, Streptococcus gordonii, Escherichia coli, Propionibacterium acnes, Proteus mirabilis, Granulicatalla adjacens, Acinetobacter baumannii, Abiotrophia defective, Corynebacterium striatum, Corynebacterium minutissimum, Parvimonas micra, Candida parapsilosis, Candida tropicalis, Candida glabrata,* and *Candida albicans*

In still other examples, the panel or sets of antibodies can be configured to detect the presence of *Staphylococcus aureus* and *Staphylococcus epidermidis,* and at least two microorganisms selected from the group consisting of *Enterococcus faecalis, Enterococcusfaecium, Pseudomonas aeruginosa, Staphylococcus lugdunensis, Staphylococcus warneri, Staphylococcus capitis, Staphylococcus caprae, Streptococcus mitis. Streptococcus oralis. Streptococcus agalactiae, Streptococcus anginosus, Streptococcus gordonii, Escherichia coli, Propionibacterium acnes, Proteus mirabilis, Granulicatella adjacens, Acinetobacter baumannii, Abiotrophia defective, Corynebacterium striatum, Corynebacterium minutissimum, Parvimonas micro. Candida parapsilosis, Candida tropicalis, Candida glabrata,* and *Candida albicans.*

In yet another example, the panel or sets of antibodies can be configured to detect the presence of *Staphylococcus aureus* and *Staphylococcus epidermidis,* and at least three microorganisms selected from the group consisting of *Enterococcus faecalis. Enterococcus faecium, Pseudomonas aeruginosa, Staphylococcus lugdunensis, Staphylococcus wameri, Staphylococcus capitis, Staphylococcus caprae. Streptococcus mitis. Streptococcus oralis, Streptococcus agalactiae, Streptococcus anginosus,* Streptococcus gordonii, *Eschencia coli, Propionibacterium acnes, Proteus mirabilis, Granulicatella adjacens, Acinetobacter baumannii, Abiotrophia defective, Corynebacterium striatum, Corynebacterium minutissimum, Parvimonas micra, Candida parapsilosis, Candida tropicalis, Candida glabrata,* and *Candida albicans.*

In a preferred example, the panel or set of antibodies can be configured to detect at least for microorganism selected from the group consisting of Staphylococcus *aureus, Staphylococcus epidermidis, Enterococcus faecalis, Pseudomonas aeruginosa, Staphylococcus lugdunensis, Streptococcus mitis, Streptococcus oralis, Streptococcus agalactiae, Eschericia coli. Propionibacterium acnes, Candida parapsilosis, Candida tropicalis,* and *Candida albicans.*

In the exemplary systems, the panel or sets of antibodies are targeted to the detection of microbial infection, particularly microbial infection in PJI, and that can be informative about microbial infection. The panels or sets may also comprise a large or small number of antibodies that detect antigens that are not informative about microbial infection; such panels and sets can be useful as controls and for normalization (e.g, spiked-in samples). Panels and sets may be a dry mixture or a mixture in solution. In some examples, panels and sets can be affixed to a solid substrate to form an array of antibody panels or sets. A device for measurement of the microbes detected and identified by the kits and systems of the present invention may also be included in the kits and systems.

### Definitions

### "Microorganism" means a bacteria, fungi, virus, and protozoa

"Antibody," as used herein, refers to an immunoglobulin molecule which specifically binds with an antigen. Antibodies can be intact immunoglobulins derived from natural sources or from recombinant sources and can be immunoreactive portions of intact immunoglobulins. Antibodies are typically tetramers of immunoglobulin molecules. The antibodies in the present invention may exist in a variety of forms including, for example, polyclonal antibodies, monoclonal antibodies, Fv, Fab and F(ab)2, as well as single chain antibodies (Harlow et al., 1999, In: Using Antibodies: A Laboratory Manual, Cold Spring Harbor Laboratory Press, NY; Harlow et al., 1989, In: Antibodies: A Laboratory Manual. Cold Spring Harbor, New York; Houston et al, 1988, Proc. Natl. Acad. Sci. USA, 85:5879-5883, Bird et al., 1988, Science, 242:423-426).

"Specifically bind(s)," as used herein with respect to an antibody. is meant an antibody which recognizes a specific antigen, but does not substantially recognize or bind other molecules in a sample For example, an antibody that specifically binds to an antigen from one species may also bind to that antigen from one or more species. But, such cross-species reactivity does not itself alter the classification of an antibody as specific In another example, an antibody that specifically binds to an antigen may also bind to different allelic forms of the antigen. However, such cross reactivity does not itself alter the classification of an antibody as specific.

"Antigen" is a molecule that binds to a receptor of the immune system and provokes an immune response. This immune response may involve either antibody production, or the activation of specific immunologically-competent cells, or both. The skilled artisan will understand that any macromolecule. including virtually all proteins or peptides, can serve as an antigen. It is readily apparent that an antigen can be generated synthesized or can be derived from a biological sample. Such a biological sample can include, but is not limited to a tissue sample, a tumor sample, a cell or a biological fluid.

"Biological sample" or "tissue sample" or "sample" means a biological material isolated from an individual The biological sample may comprise cellular and/or non-cellular material obtained from the individual. A biological sample may be of any biological tissue or fluid. Frequently the sample will be a "clinical sample" which is a sample derived from a patient. Typical clinical samples include, but are not limited to, bodily fluid samples such as articular or synovial fluid, sputum, blood, urine, blood plasma, blood serum, plasma, sweat, mucous, saliva, lymph, bronchial aspirates, peritoneal fluid. cerebrospinal fluid. and pleural fluid, and tissues samples such as blood-cells (e.g., white cells), tissue or fine needle biopsy samples and abscesses or cells therefrom, pleural fluid, or cells therefrom. Biological samples may also include sections of tissues, such as frozen sections or formalin fixed sections taken for histological purposes. Periprosthetic tissues are often analyzed for evidence of infection.

"Immunoassay" refers to a biochemical test that measures the presence or concentration of a substance in a sample, such as a biological sample, using the reaction of an antibody to its cognate antigen, for example the specific binding of an antibody to a protein Both the presence of the antigen and/or the amount of the antigen present can be measured

"Instructional material" includes a publication, a recording, a diagram, or any other medium of expression which can be used to communicate the usefulness of a component of the invention in a kit or a system for detecting microorganisms in a biological samples as disclosed herein. The instructional material of the kit or the system of the invention can. for example, be affixed to a container which contains the component of the invention or be shipped together with a container which contains the component. Alternatively, the instructional material can be shipped separately from the container with the intention that the instructional material and the component be used cooperatively by the recipient.

"Patient," "subject," "individual," and the like are used interchangeably herein, and refer to any animal, or cells thereof whether in vitro or in situ, amenable to the methods described herein. In certain non-limiting embodiments, the patient, subject or individual is a human.

"Solid support," "support," and "substrate" as used herein are used interchangeably and refer to a material or group of materials having a rigid or semi-rigid surface or surfaces. In one embodiment, at least one surface of the solid support will be substantially flat, although in some embodiments it may be desirable to physically separate synthesis regions for different compounds with, for example, wells, raised regions, pins, etched trenches, or the like. According to other embodiments, the solid support(s) will take the form of beads, resins, gels, microspheres, or other geometric configurations. See U.S. Pat. No. 5,744,305 for exemplary substrates.

### EXPERMENTAL EXAMPLES

The invention is further described in detail by reference to the following experimental examples. These examples are provided for purposes of illustration only, and are not intended to be limiting unless otherwise specified. Thus, the invention should in no way be construed as being limited to the following examples.

Without further description, it is believed that one of ordinary skill in the art can, using the preceding description and the following illustrative examples, make and utilize the compounds of the present invention and practice the claimed methods.

The materials and methods employed in the experiments disclosed herein are now described.

### Example 1. Biological Sample Preparation - I

As shown in FIG.1, a biological sample taken from a subject can be prepared according to one example of the invention disclosed herein, as follows:
1 Aliquot desired volume (250 µL) of patient biological sample (e.g. synovial fluid) into 96 well plate or individual sample tubes (500 µL to 1 mL capacity) (100)
2. Add 1 mL Tris Buffer (0 2M, pH 8.0) (110).
3. Centrifuge at 18,000 g for 10 minutes (120)
4 Separate pellet (140) and supernatant (130) (remove supernatant to a different sample tube or well)
5. Add 270 µL Tris Buffer to pellet (150).
6 Add 30 µL lysostaphin (200 µg/mL) to re-suspended pellet and vortex briefly (150).
7. Incubate enzyme-sample mixture at 37°C for 10 minutes (160).
8. Boil enzyme-sample mixture for 5 minutes (170).
9 Briefly centrifuge to remove condensation from lid.
10. Use supernatant from step #9 for MID immunoassay (220). Results of a MID immunoassay using this preparation method, are illustrated in the figures, such as at **FIGS. 2A-2D****,** **FIG. 8 through FIGS. 11A****-11B,** **FIGS. 13A-13E****.** Supernatant (130) may also be immunoassayed.

### Example 2 Biological Sample Preparation - II

As shown in FIG.1, a biological sample taken from a subject can be prepared according to one example of the invention disclosed herein, as follows:
1 Aliquot desired volume (125-250 µL) of patient biological sample (e.g., synovial fluid) into 96 well plate or individual sample tubes (500 µL to 1 mL capacity) (100).
2. Add equal volume of 2x Buffer (180).
3 Vortex briefly.
4 Incubate at 37°C for 30 minutes (190).
5. Boil for 5 minutes (200).
6 Centrifuge (210) at 18,000g for 10 minutes (sample tubes) or 3,700 g for 30 minutes (plates)
7. Use supernatant for Luminex assay or ELISA assay (220) Results of a MID immunoassay using this preparation method, are illustrated in the figures, such as at **FIGS. 3A-3D** through **FIGS.** 7A-7C, and FIGS. 22A-22B and FIG. 24.

2x Buffer comprises Tris Buffer, 1% detergent (made up in Tris Buffer), 200 µg/ml hyaluronidase, 40 µg/ml lysostaphin (final [Tris] = 156 mM - 200 mM (final Tris concentration can vary slightly).

### Example 3. Biological Sample Preparation - III

As shown in **FIG.1****,** a biological sample taken from a subject can be also be prepared according to another example of the invention disclosed herein, as follows:
1 Aliquot desired volume (100-500µL) of patient biological sample (e.g., synovial fluid) into 96 well plate or individual sample tubes (500 mL to 1 mL capacity) (100)
2. Add equal volume of 2x Sample Preparation Buffer (180).
3 Vortex briefly.
4 Incubate for 30 minutes at 37°C in incubator (190)
5. Boil for 5 minutes in water bath (200).
6. Centrifuge for 10-20 minutes at 3,700 g (210)
7. Use 50 µl supernatant for MID immunoassay (220).

2x Sample Preparation Buffer comprises phosphate buffered saline ("PBS"), 0.5% to 1% Tween-20 (prepared with PBS) or 0.5% to 1% Triton-X 100 (prepared with PBS), 200 µg/mL hyaluronidase, and 40 µg/mL lysostaphin or 500 µg/mL lysozyme, or both lysostaphin and lysozyme.

### Example 4 Biological Sample Preparation - IV

As shown in **FIG.1****,** a biological sample taken from a subject can be prepared according to one example of the invention disclosed herein, as follows:
1. An aliquot (100) of 200 µL of patient biological sample, e.g., synovial fluid ("SF") or QC control is dispensed into a 96-well Eppendorf deep well plate (500 µL/well capacity). Individual sample tubes can be substituted for a deep well plate.
2. Add an equal volume of 2x Sample Preparation Buffer (180) 2x Sample Preparation Buffer comprises phosphate buffered saline (PBS), 1% detergent (Triton-X 100 or Tween-20 (10% Tween-20 or Triton-X 100 prepared in PBS), 200 µg/mL hyaluronidase, and 40 µg/mL lysostaphin. 500 µg/mL lysozyme, or both.
3. Cover the plate (or tubes) with adhesive film and place on a plate shaker (Titer Plate Shaker) at speed 3 for 1 minute, or pipette up and down with a pipettor 5 times to mix.
4. Incubate plate/sample tubes (190) for 30 minutes at 37 °C in an incubator
5. Boil the plate/sample tubes (200) for 5 minutes in a water bath.
6. Centrifuge (210) for 30 minutes at 3,700g (plates: using SH-3000 rotor with microplate attachments in Sorvall RC6 centrifuge), or 18,000 g for 10 minutes (sample tubes).
7. Use 50 µL of the supernatant for MID immunoassay (220).

2x Sample Preparation Buffer (1mL) is prepared by combining 872 µL of 1X PBS (20 mM PO₄, 150 mM NaCl), 100 µL Tween-20 (10% in PBS), 8 µL lysostaphin (5 mg/mL) and 20 µL Hyaluronidase (10 mg/mL).

### Example 5. Biological Sample Preparation -V

As shown in **FIG.1****,** a biological sample taken from a subject can be prepared according to one example of the invention disclosed herein, as follows:
1. Dispense an aliquot (100) of 200 µl of patient SF or QC control into a 96-well Eppendorf deep well plate (500 µL/well capacity) Individual sample tubes can be substituted for a deep well plate
2. Add equal volume of 2x Sample Preparation Buffer (1800). 2x Sample Preparation Buffer. PBS, 1% Tween-20 prepared by spiking with 10% Tween-20 in PBS, 200 µg/ml hyaluronidase, 500 µg/ml lysozyme or lysostaphin). Mix up and down with a pipettor 5 times to mix the 2x Sample Preparation Buffer with the sample
3. Incubate plate/sample tubes (190) for 30 minutes at 37 °C in incubator.
4. Boil plate/sample tubes (200) for 5 minutes in water bath.
5. Centrifuge plate/sample tubes (700) for 30 minutes at 3,700g (using SH-3000 rotor with microplate attachments in Sorvall RC6 centrifuge)
6. Use 50 µl of supernatant for MID immunoassay (220).

2x Sample Preparation Buffer (1 mL) comprises 870 µL 1X PBS (20 mM PO4, 150 mM NaCl); 100 µL Tween-20 (10% in PBS), 10 µL. lysostaphin or lysozyme (50 mg/mL); 20 µL hyaluronidase (10 mg/mL).

### Example 6- MID ImmunoAssay of SF Samples-!

The following describes a study validating a bead-based (e g., Luminex^{®} ) multiplex assay for microorganism detection and identification by testing patient synovial fluid ("SF") clinical samples with known microbial antigen, no microbial antigen, and unknown microbial antigen These SF samples are segregated by α-defensin (sometimes referred to herein as "AD"), C-reactive protein ("CRP"), and relative white blood cell ("WBC") levels, along with culture positivity, i.e., sample tested positive for microbial culture This protocol was used to determine the sensitivity and specificity of the multiplex assay for microorganism identification as well as the percent of α-defensin positive/culture negative ("+/-") samples which can be identified.

In this example, the microbial identification multiplexed assay can identify four groups of organisms commonly determined to be the cause of infection in periprosthetic joints. *Staphylococcus* panel A; *Staphylococcus* panel B; *E. faecalis,* and *Candida* panel (*Candida albicans* and *Candida parapsilosis*). The microbial identification multiplexed assay (220) **of** **FIG. 1** includes polyclonal antibodies specific for each microorganism coupled to magnetic beads (Luminex^{®}) are generally referred to herein as "AB-bead complex). AB-bead complexes are incubated with processed synovial fluid samples (e.g., according to an example in **FIG. 1**) suspected of infection, in 96-well microplates Following incubation, plates are washed, the sample is incubated with biotinylated polyclonal detection antibodies specific for each microorganism coupled to magnetic beads, followed by another wash, addition of Streptavidin-PE (phycoerythrin), and read on the fluorescence detection device (e g. Luminex^{®} 100/200). Target sensitivity and specificity for each panel is ≥ 85%.

**Table 2 Patient SF samples targeted to be tested in the assay**

| **Sample type** | **α-defensin** | **CRP** | **WBC** | **% neutrophils** | **Culture result (organism)** | **Sample No.** |
|---|---|---|---|---|---|---|
| A | + | NA | NA | NA | *Staphylococcus sp.* | >50 |
| B | + | NA | NA | NA | *Candida sp.* | >10 |
| C | + | NA | NA | NA | *E. faecalis* | >10 |
| D | + | + | >3000 | >80% | Negative | >100 |
| E | + | - | >3000 | >80% | Negative | >15 |
| F | + | - | NA | <60% | Negative | >5 |
| G | - | - | <3000 | NA | Negative | >100 |

| | | | | | | |
|---|---|---|---|---|---|---|
| Sample number dependent on availability | | | | | | |

The sample set was tested over multiple days and each sample type was sufficiently randomized between days. Data was analyzed relative to controls as well as a pre-established cut-off ("C/O") as defined as either 1 5 times (*Staphylococcus panels*) or two times (*Candida* panel and *E. faecalis* assay) the mean value for QC-1. Sensitivity was calculated using specific organism samples (sample type A-C). Specificity was calculated using confirmed negative samples (sample type G). Sample characteristics such as CRP levels, protein levels, etc. were considered in the determination of positivity frequency in the α-defensin positive/culture negative ("+/-") samples. Assay(s) were performed according to the following Sample Preparation and Assay Procedures. All matrices were subjected to this sample preparation method.

**Table 3. Plate Map**

| | **1** | **2** | **3** | **4** | **5** | **6** | **7** | **8** | **9** | **10** | **11** | **12** |
|---|---|---|---|---|---|---|---|---|---|---|---|---|
| **A** | QC1 | Patient SF Samples | | | | | | | | | | |
| **B** | QC1 | | | | | | | | | | | |
| **C** | QC1 | | | | | | | | | | | |
| **D** | QC1 | | | | | | | | | | | |
| **E** | QC2 | | | | | | | | | | | |
| **F** | QC3 | | | | | | | | | | | |
| | | **G** | | | | | | QC4 | | | | |
| | | **H** | | | | | | QC5 | | | | |

**Table 4. Controls**

| **Name** | **QC** # |
|---|---|
| Negative Control | QC1 |
| *Candida Panel A* | QC2 |
| *Enterococcus faecalis* | QC3 |
| *Staphylococcus Panel A* | QC 4 |
| *Staphylococcus Panel B* | QC5 |

Synovial Fluid ("SF") sample preparation is according to Example 4 - Biological Sample Preparation IV

### MID immunoassay (magnetic bead, multiplex) procedure:

1. Block plate/test sample with 250 µL Assay Diluent (Luminex^{®} = 1xPBST with Proclin950, 0.5% bovine serum albumin ("BSA")) and incubate at room temperature for a minimum of 10 minutes.
2. Remove blocking solution by inversion and blot plate dry.
3. Dilute the microsphere cocktail in Assay Diluent to 1x, and aliquot 50 µL per well. Dilution to 1x is based on concentration of stock preparation (e.g, 60x stock is diluted 1:60; 240x stock is diluted 1:240)
4. Add 50 µL processed patient SF sample or control sample per well and seal plate with adhesive film. The control sample ("QC") is either Assay Diluent or Assay Diluent spiked with a concentration of the appropriate bacterium or fungus which gives a low- to mid-positive response. These samples are prepared and ready for the extraction procedure.
5. Incubate while shaking at room temperature for 60 minutes at speed 4 5. Keep microliter plate in the dark by either sealing it with aluminum-adhesive film or covering it with aluminum foil.
6. Ensure that the plate washer is equipped with the appropriate magnet and that the customized wash program has been selected prior to executing the washing step.
7. Position the microtiter plate on the magnet and wait for 1-minute prior to starting the washing step. Wash 3x with PBST using 200 µL, with a minute incubation between washes to allow the beads to settle on the bottom, leaving about 25 µL of volume between washes as not to aspirate the beads out of the plate. Do not invert or blot microtiter plate onto paper towels.
8. Dilute the Detection Antibody to the appropriate concentration in Assay Diluent. Add 50 µL of the detection antibody cocktail (includes detection antibody for analytes). The final concentration of each detection antibody is listed below. The detection antibody may be supplied individually or as a cocktail containing all four antibodies at 60X the working concentration.

**Table 5.**

| **Antibody to (Cognate Antigen):** | **Antibody Concentration (µg/mL)** |
|---|---|
| *Staphylococcus epidermidis* | 0.25 |
| *Staphylococcus aureus* | 4.0 |
| *Enterococcus faecalis* | 4.0 |
| *Candida albicans* | 0.31 |

9. Incubate in the dark as described in step #5 while shaking at room temperature for 60 minutes
10. Wash 3x as described in steps #6 and #7.
11. Prepare a 1.2000 dilution (0.5 mg/mL) of SA-PE and add 50 Ml per well. Seal plate with adhesive film.
12. Incubate in the dark as described in step #5 while shaking at room temperature for 30 minutes
13. Wash 3x as described in steps #6 and #7
14. Add 100 µL Sheath Fluid (Biorad) per well. Seal plate with adhesive film.
15. Incubate in the dark while shaking at room temperature for 5 minutes.
16. Read fluorescence on instrument (e g Luminex^{®} 100/200)

### Algorithm for interpreting reactivity in the MID immunoassay:

The following algorithm was used to interpret the data in the validation study. These rules represent all contingencies that have been identified in this study and other clinical testing Abbreviations used in the rules *Staphylococcus* Panel (SP); *Staphylococcus aureus* Panel A (SPA); *Staphylococcus epidermidis* Panel B (SPB), *Candida* Panel (CP); *Enterococcus faecalis* (EF); Cut-off (CIO) = 1. A summary of the rules is as follows:
1. If all assays < C/O then all are reported as negative.
2. If only one assay is ≥ C/O then only that assay is reported as positive. If both SPA and SPB are ≥ C/O, then only SP is reported as positive.
3. If CP and EF are ≥ C/O and SPA and SPB < C/O then CP and EF are both reported as positive.
4. If SPA ≥ C/O and CP and/or EF ≥ C/O but < SPA, then SP is reported as positive and CP and/or EF are reported as indeterminate.
5. If SPA > C/O and CP and/or EF ≥ C/O and > SPA. then SP and CP and/or EF are reported as positive.
6. If SPA < C/O, SPB ≥ C/O and < 6, EF ≥ C/O and EF > SPB then SP is reported as indeterminate and EF is reported as positive, but if EF < SPB then SP and EF are reported as positive
7. If SPA < C/O, SPB ≥ 6 and CA and/or EF ≥ C/O. then SP is reported as positive and CP and/or EF are reported as positive.

**Table 6. Algorithm Rules**

| **Rule** # | **Assay Results S:C/O** | **SP** | **CP** | **EF** |
|---|---|---|---|---|
| 1 | If SPA and SPB < 1.0 and CP and EF < 1.0 | - | - | - |
| 2 | If SPA and/or SPB ≥ 1.0 and CP and EF < 1.0 | + | - | - |
| 3 | If SPA and SPB < 1.0, CP < 1.0 and EF ≥ 1.0 | - | - | + |
| 4 | If SPA and SPB < 1.0, CP ≥ 1.0 and EF < 1.0 | - | + | - |
| 5 | If SPA and SPB < 1.0, CP and EF ≥ 1.0 **^{E1}** | | | |
| | | - | + | + |
| 6 | If SPA ≥ 1.0, CP and EF ≥ 1.0 and CP and EF ≤ SPA **^{E2}** | + | I | I |
| 7 | If SPA > 1.0, CP and EF > 1.0 and CP and EF > SPA **^{E3}** | + | + | + |
| 8 | If SPA ≥ 1.0, CP and EF ≥ 1.0 but CP < SPA and EF > SPA | + | I | + |
| 9 | If SPA ≥ 1.0, CP and EF ≥ 1.0 but CP > SPA and EF ≤ SPA | + | + | I |
| 10 | If SPA ≥ 1.0, CP < 1 0 and EF ≥ 1.0 but EF ≤ SPA | + | - | I |
| 11 | If SPA ≥ 10, EF < 10 and CA ≥ 1.0 but CA ≤ SPA | + | I | - |
| 12 | lf SPA ≥ 1.0, CP < 1.0 but EF ≥ 1.0 and EF > SPA | + | - | + |
| 13 | If SPA ≥ 1.0, EF < 1 0 but CA ≥ 1.0 and CA > SPA | + | + | - |
| 14 | If SPA < 1.0, SPB ≥ 1.0 but < 6.0, CA < 1.0. EF ≥ 1.0 and EF > SPB **^{E4}** | I | - | + |
| 15 | If SPA < 1.0. SPB ≥ 1.0 but < 6.0, CA < 10, EF ≥ 1.0 but EF ≤ SPB | + | - | + |
| 16 | If SPA < 1.0, SPB ≥ 1.0 but < 6.0, CA ≥ 1.0, EF ≥ 1.0 and EF > SPB | I | + | + |
| 17 | If SPA < 1.0, SPB ≥ 1.0 but < 6.0, CA ≥ 1.0, EF ≥ 1.0 but EF ≤ SPB | + | + | + |
| 18 | If SPA < 1.0, SPB ≥ 6.0, CA < 1.0, EF ≥ 1.0 | + | - | + |
| 19 | If SPA < 1.0, SPB ≥ 6.0, CA ≥ 1.0, EF ≥ 1.0 | + | + | + |
| 20 | If SPA < 1.0. SPB ≥ 1.0, EF < 1.0, CA ≥ 1.0 | + | + | - |

Reported results are designated as positive (+), negative (-), or indeterminate (I). In R6-R13, the SPB result is insignificant to the reported results E1 - *Candida* and *Enterococcus* common organisms associated together in poly-microbial infections. E2 - Protein A, found on cell surface of *Staphylococcus aureus,* can result in low-level cross-reactivity in *Candida* and *Enterococcus faecalis* assays. E3 - Cross-reactivity experiments do not indicate that high levels of *Candida* or *Enterococcus faecalis* result in positivity in *Staphylococcus* Panel A. E4 - High levels of *Enterococcus faecalis* are known to result in low-level cross-reactivity in the *Staphylococcus* Panel B assay.

### Acceptance and determination criteria:

An assay is considered valid if the Median Fluorescence Intensity signal ("MFI") of each positive control (QC2-QC5) is > cut-off ("C/O") of the respective analytes. The C/O = 1.5 times the mean MFI of negative control replicates (QC1) for each of the two *Staphylococcus* Panels, and 2 times the mean MFI of negative control replicates (QC1) for *Candida* Panel and *Enterococcus faaecalis* assay. A sample result will be considered positive if the MIF (signal) of the sample is > C/O. A sample result will be considered negative if the MIF of the sample is <C/O.

All samples with a S/ µQC1 of > *1.5* (*Staphylococcuspanels*) or *>2.0 (Candida* panel and *Enterococcus faecalis)* were considered positive in the assay Values are normalized to a signal to cutoff ("S:C/O" or "S/CO") which is determined for the assays as follows: S:C/O = S/ pQC1/1.5 for both *Staphylococcus* Panels; S:C/O = S/ µQC1/2.0 for *Candida* Panel and *Enterococcus faecalis* assay.

### Results:

Raw data (MFI) and S:C/O for each plate as illustrated in **FIGS. 25A-25H****.** Well contents represent the controls or patient sample tested. Note that the controls for plate 1 were erroneous due to a technical error therefore the controls from plate 2 (run at the same time) were used for the assay. As shown in **FIGS. 26A-26R****,** **FIGS. 27A-27H****,** **FIGS. 28A-28D****,** and **FIGS. 29A-29D****,** individual sample cell and culture data listed in sample order, S/CO and assay interpretation: α-defensin >1 is positive; C-reactive protein >3 is positive; total nucleated cells ("TNC") >3000 is positive. Organism is identified through culture at a maximum of 7 days (most samples (>95%) cultured positive within 2 days.

**FIGS. 30A-30D** present scatter plots of signal:noise (signal:QC1) in each assay for samples separated by group (abbreviations. +/+ = α-defensin positive/culture positive samples, SA = *Staphylococcus aureus,* SE = *Staphylococcus epidermidis.* SL = *Staphylococcus. lugdunensis,* EF = *Enterococcus faecalis,* Can sp = *Candida species,* St Ag. = *Streptococcus agalactiae.* PA = *Pseudomonas aeruginosa,* AD = α-defensin, Cul = culture, CRP = C-reactive protein). The dotted line represents the cutoff in the assay. Data are summarized in each graph.

FIGS. 31A-31D represent Scatter plots of signal noise (signal:QC1) in each assay for α-defensin positive/culture negative samples separated by group (abbreviations: AD = α-defensin, Cul = culture, CRP = C-reactive protein). Dotted line represents cutoff in the assay This data identifies microorganisms in 19% of α-defensin positive/culture negative (+/-) patient SF samples **FIGS. 32A-32D** summarize the assay results by species tested. Reactivity is determined by the algorithm mentioned above.

### Conclusion.

The results for this test for the controls ("QC") were within the acceptance criteria for the assay. There is expected cross reactivity in the controls between the *Staphylococcus* Panels Additionally, the *Staphylococcus* Panel A control (QC4) is detected in the *Enterococcus faecalis* assay This is not unexpected as the protein A on the cell surface of *Staphylococcus aureus* will cross react non-specifically with multiple antibodies.

The multiplex (4-plex) assay performed as expected using clinical SF samples. A sample was considered positive in either *Staphylococcus* panel if the signal.noise (MFI in sample mean MFI in QC1) is >1.5. A result is positive in the *Enterococcus faecalis* assay or *Candida* panel if signal:noise >2.0. These values were calculated to two significant digits. Signal Cutoff ("S/CO") is determined by dividing the signal noise value by the cutoff Data are presented as S/CO in the figures. In culture-positive samples tested in the *Staphylococcus* Panel, 71 % of *S*. *epidermidis,* 89% of *S*. *aureus* and 62% of *S. lugdunensis* samples were detected Of the *non-Staphylococcus* culture-positive samples tested, 9% of *E*. *faecalis* and 50% of *S. agalactiae* were also detected. The overall specificity was >98% in those panels. Results are summarized in **FIGS. 30A-30D**, 31A-31B and 32A-32D.

Of the multiple *Candida* species tested in the *Candida* Panel, 80% were detected Additionally, 13% of the *S*. *aureus* culture-positive samples also reacted in the assay. *S*. *aureus* cross reactivity can be mediated by Protein A on the bacteria and is not unexpected. As these were detected in the *Staphylococcus* panel, they would be classified as indeterminate (positive in assay but organism identity not determined) in the *Candida* assay. The overall specificity was 100% in the panel.

Of the multiple *Enterococcus faecalis* samples tested in the *Enterococcus faecalis* assay. 100% were detected Additionally, 21% of the *S*. *aureus* also reacted in the assay. *S. aureus* cross reactivity is mediated by Protein A on the bacteria and is not unexpected. As these were detected in the *Staphylococcus* panel and the signal was higher in the *Staph* Panel, they would be classified as indeterminate. 100% of *Streptococcus agalactiae* were also detected. *Streptococcus agalactiae* expresses protein G on its cell surface which is known to non-specifically react with antibodies Furthermore. *Enterococcus faecalis* was formally grouped in the *Streptococcus* genera so there may be some common epitopes. A small percentage (15%) of the *Candida* culture positive samples were also detected in the assay As these samples were negative in either *Staphylococcus* Panel these are considered positive in the assay as well. This may be the result of a co-infection as both *Candida* and *Enterococcus faecalis* are organisms that are occasionally found to co-infect. The overall specificity was >96% in the assay.

One Hundred eleven (111) α-defensin positive/culture negative samples were tested in the assay and 19% unique samples tested positive in the assay and could be identified as either *Staphylococcus, Candida* and/or *Enterococcus faecalis* as per the algorithm mentioned above. One Hundred and three (103) α-defensin negative/culture negative samples were tested in the assay and only 3 tested positive in the assays (2 *Staphylococcus* Panel, 1 *Enterococcus faecalis* assay)

The target sensitivity of 85% for *Staphylococcus* Panel B (sensitivity = 71%) and the *Candida* Panel (sensitivity = 80%) were not achieved This was primarily a result of setting a more stringent cutoff in order to minimize false positive results in the assays and achieving a specificity of >95%. This is not a result of assay performance as lower cut-offs could result -85% sensitivity and specificity in the assays. The clinical cutoffs may be reevaluated over time as the assay is put into practice For example, raising the cutoff in the *Staphylococcus* Panel A would have no effect on sensitivity but could improve on specificity.

### Example 6 -M ID Immunoassay Of SF Samples - II

The purpose of this study was to validate the MID multiplex immunoassay for microbial identification by testing patient synovial fluid ("SF") clinical samples and antigen-spiked SF samples, which can serve as a surrogate for culture positive samples of rare organisms detected in joint infection. These samples were separated by α-defensin ("AD"), C- reactive protein ("CRP"), and relative white blood cell ("WBC") levels along with culture positivity. This data determines the analytical sensitivity and specificity of the assay, as well as the percent of α-defensin positive/culture negative (+/-) samples which can be identified using the MID immunoassay

In this example, the multiplexed assay is designed to identify four organisms that are a possible cause of infection in periprosthetic joints_{:} *Pseudomonas aeruginosa; Escherichia coli; Propionibacterium acnes;* and *Streptococcus mitis* The assay consists of polyclonal antibodies specific for each organism coupled to Luminex^{®} magnetic beads, which are incubated with processed synovial fluid samples suspected of infection, in 96-well microplates Following incubation, plates were washed, and antibody-bead complex were incubated with biotinylated polyclonal detection antibodies, followed by another wash, addition of Streptavidin-phycoerythrin, and read on the Luminex^{®} 100/200. The biological samples are prepared according to Example 5 - Sample Preparation V

The microbes in this panel have a relatively low prevalence in periprosthetic joint infection ("PJI"). *Pseudomonas aeruginosa* -3.3%; *Escherichia colt* -1.9%; *Propionibacterium acnes* (unknown-the organism is difficult to culture from synovial fluid), and *Streptococcus mitts* -2.5%. As such, there are minimal patient samples available for testing clinical sensitivity In this study, analytical sensitivity was determined by spiking microbial isolates of each species into α-defensin negative/culture negative/CRP negative synovial fluid samples at different isolate concentrations.

The target specificity for *Pseudomonas aeruginosa, Escherichia coli,* and *Streptococcus mitis* was 85% and the results for *Propionibacterium acnes* will be as reported Since there is published evidence suggesting that a *Propionibacterium acnes* infection does not always have an immune and inflammatory response consistent with most microbial infections and *Propionibacterium acnes* is difficult to culture, as it is unstable in synovial fluid at room temperature, an understanding of the distribution of *Propionibacterium acnes* in synovial fluid samples will occur prospectively The number of+/- samples identified in this assay are reported. The patient SF sample types, target sample and actual number of samples tested in the assay are listed in the table below:

**Table 7.**

| **Sample type** | **α-defensin** | **CRP** | **WBC** | **% neut roph its** | **Culture result (organism)** | **Sample No.** | **No. Tested** |
|---|---|---|---|---|---|---|---|
| A | - | - | <3000 | NA | Negative SF spiked with *Pseudomonas aeruginosa* | 1-8 isolates (at multiple concentrations) | 8 |
| B | - | - | <3000 | NA | Negative SF spiked with *Escherichia coli* | 1-8 isolates (at multiple concentrations) | 8 |
| C | - | - | <3000 | NA | Negative SF spiked with *Propionibacterium acnes* | 1-8 isolates (at multiple concentrations) | 4 |
| D | - | - | <3000 | NA | Negative SF spiked with *Streptococcus mitis* | 1-8 isolates (multiple concentrations) | 1 |
| E | + | + | >3000 | >80 | Negative | >100 | 48 |
| F | + | - | >3000 | >80 | Negative | >15 | 11 |
| G | + | - | NA | <60 | Negative | > 5 | 8 |
| H | - | - | <3000 | NA | Negative | >100 | 89 |
| F | + | Mixed | | | Negative | | 25 |

Note that in some categories the actual number tested is lower than the projected number to be tested due to minimal sample availability in the synovial fluid clinical sample blinded inventory.

The sample sets were randomized and tested over multiple days. Data was analyzed relative to controls as well as pre-established cut-offs as defined in the Acceptance and Determination Criteria below. Analytical sensitivity was calculated using specific organism samples (AD-/Cul-/CRP- synovial fluid that has been spiked with boiled bacterial antigen) (sample type A-D). Specificity was calculated using confirmed negative samples (sample type H). Positivity frequency in the AD+/Cul- samples was also determined.

### Microbial assay controls:

**Table 8: Plate Map.**

| | **1** | **2** | **3** | **4** | **5** | **6** | **7** | **8** | 9 | **10** | **11** | **12** |
|---|---|---|---|---|---|---|---|---|---|---|---|---|
| **A** | QC1 | Patient SF Samples | | | | | | | | | | |
| **B** | QC1 | | | | | | | | | | | |
| **C** | QC1 | | | | | | | | | | | |
| **D** | QC1 | | | | | | | | | | | |
| **E** | QC6 | | | | | | | | | | | |
| **F** | QC7 | | | | | | | | | | | |
| **G** | QC8 | | | | | | | | | | | |
| **H** | QC9 | | | | | | | | | | | |

**Table 9: Controls.**

| **Name** | | **QC #** |
|---|---|---|
| Negative Control | | QC1 |
| | *Escherichia coli* | QC6 |
| | *Pseudomonas aeruginosa* | QC7 |
| | *Streptococcus mitis* | QC8 |
| | *Propionibacterium acnes* | QC9 |

Synovial Fluid ("SF") samples were prepared according to Example 5- Biological Sample Preparation - V.

### Microbial identification assay procedure:

1. Block plate with 250 µl Assay Diluent (1X PBST with Proclin950, 0.5% BSA) and incubate at room temperature for a minimum of 10 min.
2. Remove blocking solution by inversion and blot plate dry.
3. Dilute the microsphere cocktail by 60-fold in Assay Diluent (Luminex^{®}) and aliquot 50 µl per well. Individual bead preparations may be used and combined.
4. Add 50 µl processed patient SF sample or control sample per well and seal plate with adhesive film. The control sample is either assay diluent or Assay Diluent spiked with a concentration of the appropriate microbe which gives a low to mid positive response. These samples are prepared and ready for the extraction procedure
5. Incubate while shaking at room temperature for 60 minutes at speed 4.5 on a Labline Shaker Model 4625 or equivalent. Keep microtiter plate in the dark by either sealing it with aluminum-adhesive film or covering it with aluminum foil
6. Ensure that the plate washer is equipped with the appropriate magnet and that the customized wash program has been selected prior to executing the washing step.
7. Position the microtiter plate on the magnet and wait for 1-minute prior to starting the washing step. Wash 3x with PBST using 200 µL, with a minute incubation between washes to allow the beads to settle on the bottom, leaving about 25 µL of volume between washes as not to aspirate the beads out of the plate. Do not invert or blot microtiter plate onto paper towels.
8. Dilute the detection antibody to the appropriate concentration in Assay Diluent. Add 50 µl of the detection antibody cocktail (includes detection antibody for analytes) per well. The final concentration of each detection antibody is listed below. The detection antibody may be supplied individually or as a cocktail containing all four antibodies at 60X the working concentration.

**Table 10.**

| **Antibody to (Cognate Antigen):** | **Antibody Concentration (µg/mL)** |
|---|---|
| *Propionibacterium acnes* | 0.25 |
| *Streptococcus mitis* | 4 |
| *Escherichia coli* | 0.5 |
| *Pseudomonas aeruginosa* | 0.5 |

9. Incubate in the dark as described in step #5 while shaking at room temperature for 60 minutes.
10. Wash 3x as described in steps #6 and #7
11. Prepare a 1:2000 dilution (0 5 mg/mL) of SA-PE (Moss) and add 50 µl per well. Seal plate with adhesive film
12. Incubate in the dark as described in step #5 while shaking at RT for 30 minutes.
13. Wash 3x as described in steps #6 and #7
14. Add 100 µl sheath fluid per well. Seal plate with adhesive film.
15. Incubate in the dark while shaking at RT for 5 minutes.
16. Read on fluorescence instrument (e.g, Luminex 100/200).

### Acceptance and determination criteria:

An assay will be considered valid if MFI (Median Fluorescence Intensity) (signal) of each positive control (QC6-QC9) is > cut-off ("C/O" or "CO") of the respective analytes. The C/O = 2 times the mean MFI of negative control replicates (QC1) for *Escherichia coli. Pseudomonas aeruginosa,* and *Streptococcus oral is* assays, and 10 times the mean MFI of negative control replicates (QC1) for *Propionibacterium acnes* assay. A sample result will be considered positive if the MIF (signal) of the sample is > C/O. A sample result will be considered negative if. the MIF (signal) of die sample is < C/O

The plate maps for each plate are shown in **FIGS. 33A-33B** The -/-/- synovial fluid pool was created with SF samples from the blinded inventory as shown in **FIG. 33C****.**

The following method was followed for each validation plate:
1 Microbial antigen stocks,-/-/- SF pool and the appropriate # of other SF samples (-/-/-, +/ +/-, etc. were removed from the freezer and allowed to thaw.
2. Microbial antigen dilutions were prepared according to the tables shown in **FIGS. 34A-34C**
3 200 µL of each QC was added to the Sample Prep plate following the plate map above.
4. 190 µL of -/-/- SF pool was added to the Sample Prep plate according to the plate map above
5 10 µL of the Desired Concentration Solution was added into each well following the plate map above and mixed well.
6. 200 µL of each additional SF sample type was added into each well following the plate map above.
7 Sample preparation and assay execution were carried out as described above.

The quality controls on each plate met the criteria set forth in the Acceptance and determination criteria are shown in **FIG. 35****.** The raw MFI and calculated signal: cutoff (S:C/O) are summarized for each plate in **FIGS. 36A-36C** (EC =*Escherichia coli.* PAR= *Pseudomonas aeruginosa,* SM= *Streptococcus mitis,* PAC= *Propionibacterium acnes*). The results for all antigen spiked synovial fluid samples are summarized in **FIGS. 37A-****37D.** Positive results are represented by S. C/O≥1.

Multiple, but not all *Pseudomonas aeruginosa* strains were detected in the *Pseudomonas aeruginosa* assay at 1×10⁴ - 1×10⁶ CFU/mL (note: the three strains detected all belong to the E (PVIII) serogroup). In the *Escherichia coli* assay. 5 out of the 8 *Escherichia coli* spiked strains were detected at 1×10⁶ CFU/mL Two spiked *Propionibacterium acnes* strains were detected at 1×10⁵ -1×10⁶ CFU/mL The sole *Streptococcus mitis* strain in our inventory was detected at 1×10⁶ CFU/mL.

Note that all microbial antigens were spiked into -/-/- synovial fluid to mimic clinical samples as minimal culture positive samples exist for these organisms The recovery of microbial antigens from -/ -/ - synovial fluid tested in this experiment range from 30-100% (data not shown). Matrix effects on recovery prevent quantitative comparisons between studies where antigens are extracted from different matrices (i.e assay diluent).

The S:C/O values of synovial fluid samples tested are organized by three types (AD+/CRP+/Culture-, AD+/CRP-/Culture- and AD-/CRP-/Culture-) as shown in **FIGS. 38A-38D**

### Rules:

The following rules to determine the qualitative result (positive, negative or indeterminate) of each assay were developed based on findings from the cross-reactivity studies

**Table 11.**

| **Rule** # | **Assay Results S:C/O** | **EC** | **PAR** | **SM** | **PAC** |
|---|---|---|---|---|---|
| 1 | If EC, PAR, SM and PAC < 1.0 | - | - | - | - |
| 2 | If EC ≥ 1.0 and PAR, SM and PAC < 1.0 | + | - | - | - |
| 3 | If PAR ≥ 1.0 and EC, SM and PAC < 1.0 and EF ≥ 1.0 | - | + | - | - |
| 4 | If SM ≥ 1.0, and EC, PAR and PAC < 10 | - | - | + | - |
| 5 | If PAC ≥ 1.0 and EC. PAR and SM < 1.0 | - | - | - | + |
| 6 | If EC and PAR ≥ 1.0, but< 3.0 and SM and PAC < 1.0 | + | + | - | - |
| 7 | If EC and PAR ≥ 1.0, EC> PAR and PAR <3 and SM and PAC < 1.0 | + | I | - | - |
| 8 | If EC and PAR ≥ 1.0. PAR> EC and EC <3 and SM and PAC < 1.0 | I | + | - | - |
| 9 | If EC and PAR ≥ 3.0 and SM and PAC < 1.0 | + | + | - | - |
| 10 | If PAR and SM ≥ 1.0, but< 3.0 and EC and PAC < 1.0 | - | + | + | - |
| 11 | If PAR and SM ≥ 1.0, SM > PAR and PAR <3 and EC and PAC < 1.0 | - | I | + | - |
| 12 | If PAR and SM ≥ 1.0, PAR > SM and SM <3 and EC and PAC < 1.0 | - | + | I | - |
| 13 | If PAR and SM ≥ 3.0 and EC and PAC < 1.0 | - | + | + | - |

**Table 12 Rules for interpreting data together with Staphylococcus species test results.**

| Rule # | **Assay Results S:C/O** | **EC** | **PAR** | **SM** | **PAC** | **Notes** |
|---|---|---|---|---|---|---|
| 1 | If EC, PAR, SM and PAC < 1.0 | - | - | - | - | |
| 2A | If SPA <10.0, PAC ≥ 1.0 and EC, PAR and SM < 1.0 | - | - | - | + | |
| 2B | If SPA ≥10.0, PAC ≥ 1.0 and EC, PAR and SM < 1.0 | - | - | - | | At high levels, Protein A found on cell surface of *S. aureus* can result in cross-reactivity |
| 3A | If SPA <10.0, SM ≥ 1.0 and EC, PAR and PAC < 1.0 | - | - | + | - | |
| 3B | If SPA ≥10.0, SM ≥ 1.0 and EC, PAR and PAC < 1.0 | - | - | I | - | At high levels. Protein A found on cell surface of *S. aureus* can result in cross-reactivity |
| 4A | If SPA 10.0, PAC and SM ≥1 and EC and PAR <1 | - | - | + | + | |
| 4B | If SPA ≥10.0, PAC and SM ≥1 and EC and PAR <1 | - | - | I | I | At high levels, Protein A found on cell surface of *S. aureus* can result in cross-reactivity |
| 5A | If SPA <10.0, PAR ≥ 1.0 and EC, SM and PAC < 1.0 | - | + | - | - | |
| 5B | If SPA ≥10.0, PAR ≥ 1.0 and EC, SM and PAC < 1.0 | - | I | - | - | At high levels. Protein A found on cell surface of *S. aureus* can result in cross-reactivity |
| 6A | If SPA <10.0, PAR and PAC > 1.0 and EC and SM < 1.0 | - | + | - | + | |
| 6B | If SPA ≥10.0, PAR and PAC ≥ 1.0 and EC and SM < 1.0 | - | I | - | I | At high levels, Protein A found on cell surface of *S*. *aureus* can result in cross-reactivity |
| 7A | If SPA <10.0, PAR and SM ≥ 3.0 and EC and PAC < 1.0 | - | + | + | - | |
| 7B | If SPA >10.0, PAR and SM ≥ 3.0 and EC and PAC < 1.0 | - | I | I | - | At high levels, Protein A found on cell surface of *S*. *aureus* can result in cross-reactivitv |
| 8A | If SPA <10.0, PAR and SM > 1.0 but < 3.0 and EC and PAC <1 | - | + | + | - | |
| 8B | If SPA ≥10.0, PAR and SM ≥ 1.0 but < 3.0 and EC and PAC <1 | - | I | I | - | At high levels, Protein A found on cell surface of *S*. *aureus* can result in cross-reactivity |
| 9A | If SPA <10.0, PAR and SM ≥ 1.0 but < 3.0, and PAC ≥1.0 and EC and <1.0 | - | + | + | + | |
| 9B | If SPA ≥10.0, PAR and SM > 1.0 but < 3.0, and PAC ≥1.0 and EC and <1.0 | - | I | I | I | At high levels, Protein A found on cell surface of *S*. *aureus* can result in cross-reactivity |
| 10A | If SPA <10.0, PAR and SM ≥ 3.0 and PAC ≥ 1.0 and EC < 1.0 | - | + | + | + | |
| 10B | If SPA ≥10.0, PAR and SM ≥ 3.0 and PAC ≥ 1.0 and EC < 1.0 | - | I | I | I | At high levels, Protein A found on cell surface of *S*. *aureus* can result in cross-reactivity |
| 11A | If SPA <10, SM ≥ 3.0. and PAR ≥ 1.0 but < 3.0 and EC and PAC < 1.0 | - | I | + | - | High levels of *S.mitis* can lead to low level positivity in the *P*. *aeruginosa* assay |
| 11B | If SPA ≥10, SM ≥ 3.0, and PAR ≥ 1.0 but < 3.0 and EC and PAC < 1.0 | - | I | I | - | At high levels, Protein A found on cell surface of *S*. *aureus* can result in cross-reactivity |
| 12A | If SPA <10, SM ≥ 3.0, and PAR ≥ 1.0 but < 3.0 and EC <1 and PAC ≥ 1.0 | - | I | + | + | High levels of *S.mitiscan* lead to low level positivity in the *P*. *aeruginosa* assay |
| 12B | If SPA ≥ 10,SM ≥ 3.0, and PAR ≥ 1.0 but < 3.0 and EC <1 and PAC ≥ 1.0 | - | I | I | I | At high levels, Protein A found on cell surface of *S*. *aureus* can result in cross-reactivity |
| 13A | If SPA <10.0, EC ≥ 1.0 and PAR, SM and PAC < 1.0 | + | - | - | - | |
| 13B | If SPA ≥10.0, EC ≥ 1.0 and PAR, SM and PAC < 1.0 | I | - | - | - | At high levels, Protein A found on cell surface of *S*. *aureus* can result in cross-reactivity |
| 14A | If SPA <10.0, EC and PAC ≥ 1.0 and PAR and SM < 1.0 | + | - | - | + | |
| 14B | If SPA ≥10.0, EC and PAC ≥ 1.0 and PAR and SM < 1.0 | I | - | - | I | At high levels, Protein A found on cell surface of *S*. *aureus* can result in cross-reactivity |
| 15A | If SPA <10.0, EC and SM ≥ 1.0 and PAR and PAC < 1.0 | + | - | + | - | |
| 15B | If SPA ≥10.0, EC and SM ≥ 1.0 and PAR and PAC < 1.0 | I | - | I | - | At high levels, Protein A found on cell surface of *S*. *aureus* can result in cross-reactivity |
| 16A | If SPA <10.0, EC PAC and SM ≥ 1.0 and PAR < 1.0 | + | - | + | + | |
| 16B | If SPA ≥10.0, EC PAC and SM ≥ 1.0 and PAR < 1.0 | I | - | I | I | At high levels, Protein A found on cell surface of *S*. *aureus* can result in cross-reactivity |
| 17A | If SPA <10.0, EC and PAR ≥ 1.0, but < 3.0 and SM and PAC < 1.0 | + | + | - | - | |
| 17B | If SPA ≥ 10.0, EC and PAR ≥ 1.0, but < 3.0 and SM and PAC < 1.0 | I | I | - | - | At high levels, Protein A found on cell surface of *S. aureus* can result in cross-reactivity |
| 18A | If SPA <10.0, EC and PAR > 3.0 and SM and PAC < 1.0 | + | + | - | - | |
| 18B | If SPA ≥ 10.0, EC and PAR ≥ 3.0 and SM and PAC < 1.0 | I | I | - | - | At high levels. Protein A found on cell surface of *S. aureus* can result in cross-reactivity |
| 19A | If SPA <10.0, EC and PAR ≥ 1.0, but < 3.0 and SM <1 and PAC ≥ 1.0 | + | + | - | + | |
| 19B | If SPA ≥10.0, EC and PAR ≥ 1.0, but < 3.0 and SM <1 and PAC ≥ 1.0 | I | I | - | I | At high levels, Protein A found on cell surface of *S. aureus* can result in cross-reactivity |
| 20A | If SPA <10.0, EC and PAR ≥ 3.0 and PAC ≥ 1.0 and SM < 1.0 | + | + | - | + | |
| 20B | If SPA ≥ 10.0, EC and PAR ≥ 3.0 and PAC ≥ 1.0 and SM < 1.0 | I | I | - | I | At high levels, Protein A found on cell surface of *S*. *aureus* can result in cross-reactivity |
| 21A | If SPA <10.0, EC, PAR and SM ≥ 3.0 and PAC < 1.0 | + | + | + | - | |
| 21B | If SPA ≥10.0, EC, PAR and SM ≥ 3.0 and PAC < 1.0 | I | I | I | - | At high levels, Protein A found on cell surface of *S*. *aureus* can result in cross-reactivitv |
| 22A | If SPA<10.0, EC, PAR and SM ≥ 1.0 but <3.0 and PAC < 1.0 | + | + | + | - | |
| 22B | If SPA ≥10.0, EC, PAR and SM ≥ 1.0 but <3.0 and PAC < 1.0 | I | I | I | - | At high levels, Protein A found on cell surface of *S*. *aureus* can result in cross-reactivity |
| 23A | If SPA<10.0, EC, PAR and SM ≥ 3.0 and PAC ≥ 1.0 | + | + | + | + | |
| 23B | If SPA ≥10.0, EC, PAR and SM ≥ 3.0 and PAC ≥ 1.0 | I | I | I | I | At high levels, Protein A found on cell surface of *S*. *aureus* can result in cross-reactivitv |
| 24A | If SPA<10.0, EC, PAR and SM ≥ 1.0 but <3.0 and PAC ≥ 1.0 | + | + | + | + | |
| 24B | If SPA ≥10.0, EC, PAR and SM ≥ 1.0 but <3.0 and PAC ≥ 1.0 | I | I | I | I | At high levels, Protein A found on cell surface of *S*. *aureus* can result in cross-reactivity |
| 25A | If SPA <10.0, EC and PAR ≥ 3.0, and SM ≥ 1.0 but < 3.0 and PAC < 1.0 | + | + | I | - | High levels of *P. aeruginosa* can lead to low level cross reactivity in the *S*. *mitis* assay |
| 25B | If SPA ≥10.0, EC and PAR ≥ 3.0, and SM ≥ 1.0 but < 3.0 and PAC < 1.0 | I | I | I | - | At high levels, Protein A found on cell surface of *S*. *aureus* can result in cross-reactivity |
| 26A | If SPA <10.0, EC and PAR ≥ 3.0, and SM ≥ 1.0 but < 3.0 and PAC ≥ 1.0 | + | + | I | + | High levels of *P. aeruginosa* can lead to low level cross reactivity in the *S*. *mitis* assay |
| 26B | If SPA ≥10.0, EC and PAR ≥ 3.0, and SM ≥ 1.0 but < 3.0 and PAC ≥ 1.0 | I | I | I | 1 | At high levels, Protein A found on cell surface of *S. aureus* can result in cross-reactivity |
| 27A | If SPA <10.0, EC and PAR ≥ 1.0, but PAR < 3.0 and EC ≥3.0 and SM <1 and PAC ≥ 1.0 | + | I | - | + | High levels of *E.coli* can lead to low level cross reactivity in the *P*. *aeruginosa* assay |
| 27B | If SPA ≥10.0, EC and PAR ≥ 1.0, but PAR < 3.0 and EC ≥3.0 and SM <1 and PAC≥ 1.0 | I | I | - | I | At high levels, Protein A found on cell surface of *S. aureus* can result in cross-reactivity |
| 28A | If SPA <10.0, EC and PAR ≥ 1.0, but PAR ≥ 3.0 and EC <3.0 and SM <1 and PAC < 1.0 | I | + | - | - | High levels of *P. aeruginosa* can lead to low level cross reactivity in the *E*. *coli* assay |
| 28B | If SPA ≥10.0, EC and PAR ≥ 1.0, but PAR ≥ 3.0 and EC <3.0 and SM <1 and PAC < 1.0 | I | I | - | - | At high levels, Protein A found on cell surface of *S*. *aureus* can result in cross-reactivity |
| 29A | If SPA <10.0, EC and PAR > 1.0, but EC < 3.0 and PAR ≥3.0 and SM <1 and PAC ≥ 1.0 | I | + | - | + | High levels of *P. aeruginosa* can lead to low level cross reactivity in the *E*. *coli* assay |
| 29B | If SPA ≥10.0, EC and PAR ≥ 1.0, but EC < 3.0 and PAR ≥3.0 and SM <1 and PAC ≥ 1.0 | I | I | - | I | At high levels, Protein A found on cell surface of *S. aureus* can result in cross-reactivity |
| 30A | If SPA <10, EC and SM ≥ 1.0, but < 3.0, PAR ≥3.0 and PAC <1.0 | I | + | I | - | High levels of *P. aeruginosa* can lead to low level cross reactivity in the *E*. *coli* and *S. mitis* assays |
| 31B | If SPA <10, EC and SM ≥ 1.0, but < 3.0, PAR ≥3.0 and PAC ≥1.0 | I | + | I | + | High levels of *P. aeruginosa* can lead to low level cross reactivity in the *E*. *coli* and *S. mitis* assays |
| 31A | If SPA ≥10, EC and SM ≥ 1.0, but < 3.0, PAR ≥3.0 and PAC <1.0 | I | I | I | - | At high levels, Protein A found on cell surface of *S*. *aureus* can result in cross-reactivity |
| 30B | If SPA ≥10, EC and SM ≥ 1.0, but < 3.0, PAR ≥3.0 and PAC ≥1.0 | I | I | I | I | At high levels, Protein A found on cell surface of *S. aureus* can result in cross-reactivity |
| 32A | If SPA <10, EC ≥3.0. PAR ≥1 but <3. SM and PAC <1.0 | + | I | - | - | High levels of E.coli can lead to low level cross reactivity in the P. *aeruginosa* assay |
| 32B | If SPA ≥10, EC ≥3.0, PAR ≥1 but <3. SM and PAC <1.0 | I | I | - | - | At high levels, Protein A found on cell surface of *S. aureus* can result in cross-reactivity |
| 33A | If SPA<10, PAR ≥3.0, SM ≥1.0 but <3.0. EC and PAC <1.0 | - | + | I | - | High levels of *P. aeruginosa* can lead to low level cross reactivity in the *S*. *mitis* assay |
| 33B | If SPA≥10, PAR ≥3.0, SM ≥1.0 but <3.0. EC and PAC <1.0 | - | I | I | - | At high levels, Protein A found on cell surface of *S. aureus* can result in cross-reactivity |
| 34A | If SPA<10, PAR ≥3.0, SM ≥ 1.0 but <3.0, PAC ≥1.0 and EC <1.0 | - | + | I | + | High levels of *P. aeruginosa* can lead to low level cross reactivity in the *S*. *mitis* assay |
| 34B | If SPA≥10, PAR ≥3.0, SM ≥1.0 but <3.0, PAC ≥1.0 and EC <1.0 | - | I | I | I | At high levels, Protein A found on cell surface of *S. aureus* can result in cross-reactivity |
| 35A | If SPA <10.0, EC ≥1 but <3, PAR and SM ≥3 and PAC <1 | I | + | + | - | High levels of *P. aeruginosa* can lead to low level cross reactivity in the *E*. *coli* assay |
| 35B | If SPA <10.0, EC ≥1 but <3, PAR and SM ≥3 and PAC ≥1 | I | + | + | + | High levels of *P. aeruginosa* can lead to low level cross reactivity in the *E*. *coli* assay |
| 36A | If SPA ≥10.0, EC ≥1 but <3. PAR and SM ≥3 and PAC <1 | I | I | I | - | At high levels, Protein A found on cell surface of *S*. *aureus* can result in cross-reactivity |
| 36B | If SPA ≥10.0, EC ≥1 but <3, PAR and SM ≥3 and PAC ≥1 | I | I | I | I | At high levels, Protein A found on cell surface of *S*. *aureus* can result in cross-reactivity |
| 37A | If SPA <10.0, EC and SM ≥3.0, PAR ≥1.0 but <3. PAC<1.0 | + | I | + | - | High levels of *S.mitis* can lead to low level positivity in the *P*. *aeruginosa* assay |
| 37B | If SPA ≥ 10.0, EC and SM ≥3.0, PAR ≥1.0 but <3. PAC<1.0 | I | I | I | - | At high levels, Protein A found on cell surface of *S. aureus* can result in cross-reactivity |
| 38A | If SPA <10.0, EC and SM ≥3.0, PAR ≥1.0 but <3. PAC ≥1.0 | + | I | + | + | High levels of *E.coli* and *S*. *mitis* can lead to low level cross reactivity in the *P*. *aeruginosa* assay |
| 38B | If SPA ≥10.0, EC and SM ≥3.0, PAR ≥1.0 but <3. PAC ≥1.0 | I | I | I | I | At high levels, Protein A found on cell surface of *S. aureus* can result in cross-reactivity |

Reported results are positive (+), negative (-), and indeterminate (I). Table 11, R6 - R9: Cross-reactivity studies indicate low level reactivity (S.C/O < 2.0) of *Pseudomonas aeruginosa* in the *Escherichia coli* assay as well as low level reactivity (S:C/O < 2 0) of *Escherichia coli* in the *Pseudomonas aeruginosa* assay, when tested at high concentrations (2×10⁷ CFU/mL) R10 - R13: Cross-reactivity studies indicate low level reactivity (S:C/O < 2.0) of *Streptococcus mitis* in the *Pseudomonas aeruginosa* assay as well as low level reactivity (S:C/O < 2.0) of *Streptococcus mitis* in the *Escherichiacoli* assay, when tested at high concentrations (2×10⁷ CFU/mL). The *Propionibacterium acnes* assay did not react with any non-target microbial strain at any concentration and thus an S/C>1 will always be positive

The presence of *Staphylococcus aureus* resulted in low level reactivity (S.C/O < 3.0) in the *Escherichia coli, Pseudomonas aeruginosa* and *Streptococcus mitis* assays. If the *Staphylococcus aureus* assay is positive, an indeterminate (I) result would be reported Note that none of *Escherichia coli, Pseudomonas aeruginosa, Staphylococcus mitis* and *Propionibacterium acnes* reacted in the *Staphylococcus aureus* assay (data not shown)

All clinical samples tested here had a S:C/O < 1 except for C4237 on plate 3, and thus all assays would be reported as negative according to the Example 2 assay rules listed above Sample C4237 would be considered positive in the *Propionibacterium acnes* assay

The results of the 180 synovial fluid samples tested are summarized in the table below

**Table 13.**

| **Sample type** | **α-defensin** | **CRP** | **WBC** | **% neu trap hils** | **Culture result** | **No. Tested** | **EC +** | **PAR +** | **SM +** | **PAC +** |
|---|---|---|---|---|---|---|---|---|---|---|
| E | + | + | >3000 | >80 | Negative | 48 | 0 | 0 | 0 | 1 |
| F | + | - | >3000 | >80 | Negative | 11 | 0 | 0 | 0 | 0 |
| G | + | - | NA | <60 | Negative | 8 | 0 | 0 | 0 | 0 |
| H | - | - | <3000 | NA | Negative | 89 | 0 | 0 | 0 | 0 |
| F | + | Mixed | | | Negative | 25 | 0 | 0 | 0 | 0 |

Given the lower prevalence of the target organisms, it is not surprising that only 1 out of the 92 AD positive/culture negative samples (1.1%) resulted in a positive result. Additionally, this assay was designed to have extremely high specificity.

### Conclusions.

The microbial targets of the Example 2 assays were identified from PJI suspected synovial fluid with a relatively low prevalence *(Pseudomonas aeruginosa* -3.3%, *Escherichia coli* -1.9%, *Propionibacterium acnes* <1%, and *Streptococcus mitis* -2.5%), and thus a minimal number of samples that cultured positive for these organisms exist. Therefore, the analytical sensitivity of each Example 2 assay was determined by spiking microbial antigen into a negative synovial fluid pool as described above. The table below summarizes the analytical sensitivity of each assay:

**Table 14**

| **Assay** | **Analytical Sensitivity (CFU/ml)** |
|---|---|
| *Pseudomonas aeruginosa* | 1 ×10 ⁴ - 1 ×10 ⁶ |
| *Escherichia coli* | 1 ×10 ⁶ |
| *Propionibacterium acnes* | 1 ×10 ⁵ - 1 ×10⁶ |
| *Streptococcus mitis* | 1 ×10 ⁶ |

Additionally, clinical samples were also tested to determine assay specificities as well as positivity rates in AD+/CRP+/Culture- samples as these are the most probable true positives (CRP negatives would generally be expected to have a low positivity rate). The results for each assay are summarized in the tables below Each assay demonstrated 100% specificity. Given the low number of+/- samples that were available for this study, detection in these categories were as expected. The assay detection rates in the +/- samples will be adjusted, if need be, as more clinical samples are tested

**Table 15.**

| ***Pseudomonas aeruginosa* Assay** | | | | |
|---|---|---|---|---|
| **Sample Type** | **# of Samples Tested** | **# of Samples Tested Positive** | **Specificity** | **% detection in +/-** |
| AD-/CRP-/Culture- | **89** | 0 | 100% | - |
| AD+/CRP+/Culture- | 71 | 0 | - | 0.00% |

**Table 16.**

| ***Escherichia coli* Assay** | | | | |
|---|---|---|---|---|
| **Sample Type** | **# of Samples Tested** | **# of Samples Tested Positive** | **Specificity** | **% detection in +/-** |
| AD-/CRP-/Culture- | 89 | 0 | 100% | - |
| AD+/CRP+/Culture- | 71 | 0 | - | 0.00% |

**Table 17.**

| ***Propionibacterium acnes* Assay** | | | | |
|---|---|---|---|---|
| **Sample Type** | **# of Samples Tested** | **# of Samples Tested Positive** | **Specificity** | **% detection in +/-** |
| A D-/CRP-/Culture- | 89 | 0 | 100% | -- |
| AD+/CRP+/Culture- | 71 | 1 | -- | 1.40% |

**Table 18.**

| ***Streptococcus mitis* Assay** | | | | |
|---|---|---|---|---|
| **Sample Type** | **# of Samples Tested** | **# of Samples Tested Positive** | **Specificity** | **% detection in +/-** |
| AD-/CRP-/Culture- | 89 | 0 | 100% | -- |
| AD+/CRP+/Culture- | 71 | 0 | -- | 0.00% |

Each of the non-limiting elements, features, and examples described in this patent document can stand on its own, or can be combined in various permutations or combinations with one or more of the other elements, features, and/or examples

The above detailed description includes references to the accompanying drawings, which form a part of the detailed description. The drawings show, by way of illustration, specific embodiments in which the invention can be practiced. These embodiments are also referred to herein as "examples."

Method examples described herein can be machine or computer-implemented at least in part. Some examples can include a computer-readable medium or machine-readable medium encoded with instructions operable to configure an electronic device to perform methods as described in the above examples, including one or more of the algorithms described in Experimental Examples 5 and 6. An implementation of such methods can include code, such as microcode, assembly language code, a higher-level language code, or the like. Such code can include computer readable instructions for performing various methods. The code may form portions of computer program products Further, in an example, the code can be tangibly stored on one or more volatile, non-transitory, or non-volatile tangible computer-readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.

The foregoing description of the examples and embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described.

Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims.

The Abstract is provided to allow the reader to quickly ascertain the nature of the technical disclosure. It is submitted with the understanding that it will not be used to interpret or limit the scope or meaning of the claims.

All numeric values are herein assumed to be modified by the term "about", whether or not explicitly indicated The term "about", when referring to numeric values, generally refers to a range of numbers that one of skill in the art would consider equivalent to the recited value (i e., having the same function or result). In many instances, the term "about" may be indicative as including numbers that are rounded to the nearest significant figure. "About", when referring to a measurable value such as an amount, a temporal duration, and the like, may encompass variations of ±20% or ±10%. more preferably ±5%, even more preferably ±1%, and still more preferably ±0.1% from the specified value, as such variations are appropriate to perform the disclosed methods.

Throughout this disclosure. various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range For example, description of a range such as from 1 to 6 should be considered to have specifically disclosed subranges such as from 1 to 3, from 1 to 4, from 1 to 5, from 2 to 4, from 2 to 6, from 3 to 6 etc.. as well as individual numbers within that range, for example, 1, 2, 2.7, 3, 4, 5, 5.3, and 6. This applies regardless of the breadth of the range.

Although some suitable dimensions, ranges and/or values pertaining to various components, features and/or specifications are disclosed, one of skill in the an, incited by the present disclosure, would understand desired dimensions, ranges and/or values may deviate from those expressly disclosed.

In this document, the terms "a" or "an" are used, as is common in patent documents, to include one or more than one, independent of any other instances or usages of "at least one" or "one or more " In this document the term "or" is used to refer to a nonexclusive or, such that "A or B" includes "A but not B," "B but not A." and "A and B," unless otherwise indicated. In this document, the terms "including" and "in which" are used as the plain-English equivalents of the respective terms "comprising" and "wherein " Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section Terms such as "first," "second," and other numerical terms when used herein do not imply a sequence or order unless clearly indicated by the context Thus, a first element, component, region, layer or section discussed below could be termed a second element, component, region, layer or section without departing from the teachings of the example embodiments.

The terminology used herein is for the purpose of describing particular example embodiments only and is not intended to be limiting. As used herein, the singular forms "a," "an," and "the" may be intended to include the plural forms as well. unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

## Claims

1. A method of manipulating a patient sample, comprising;
mixing a portion of a patient sample with a buffer solution comprising a detergent and an enzyme configured to cleave a microbial cell wall protein; and
heating the mixture to a temperature greater than 70°C.

2. The method of claim 1, wherein the enzyme comprises:
(a) a glycosidase, an endopeptidase, an amidase, or a combination thereof; or
(b) lysostaphin, lysozyme, or a combination thereof.

3. The method of claim 1, wherein the buffer solution comprises hyaluronidase, trypsin, or a combination thereof.

4. The method of claim 1, wherein the heating is to a temperature greater than about 95°C.

5. The method of claim 1, further comprising centrifuging the patient sample at a force of between about 3,000 g and about 18,000 g to form a pellet, and wherein mixing includes mixing the pellet with the buffer solution.

6. The method of claim 1, further comprising centrifuging the mixture at a force between about 3,000 g and about 18,000 g, optionally wherein the centrifuging is for a period of time from about 5 minutes to about 60 minutes.

7. The method of claim 1, wherein the patient sample has previously tested negative for the presence of a microorganism.

8. The method of claim 1, further comprising, after the heating step, performing an immunoassay of the mixture, the immunoassay configured to detect a microorganism in the mixture.

9. The method of claim 8, further comprising preparing an immunoassay capture reagent, or an immunoassay detection reagent, or both, configured to specifically bind a microorganism isolated from a subject having a joint infection.

10. The method of claim 8, wherein the immunoassay is configured to detect:
(a) at least two different genus of microorganisms; or
(b) at least two microorganisms selected from the group consisting of Enterococcus faecalis, Enterococcus faecium, Pseudomonas aeruginosa, Staphylococcus aureus, Staphylococcus epidermidis, Staphylococcus lugdunensis, Staphylococcus wameri, Staphylococcus capitis, Staphylococcus caprae, Streptococcus mitis, Streptococcus oralis, Streptococcus agalactiae, Streptococcus anginosus, Streptococcus gordonii, Escherichia coli, Propionibacterium acnes, Proteus mirabilis, Granulicatella adjacens, Acinetobacter baumannii, Abiotrophia defective, Corynebacterium striatum, Corynebacterium minutissimum, Parvimonas micra, Candida parapsilosis, Candida glabrata, Candida tropicalis, and Candida albicans.

11. The method of claim 8, wherein the immunoassay comprises a capture reagent, a detection reagent, or both, configured to specifically bind a microorganism isolated from a biological sample obtained from a subject having a joint infection.

12. The method of claim 8, wherein the immunoassay comprises isolated polyclonal antibodies to at least two microorganisms selected from:
(a) Enterococcus faecalis;
(b) Staphylococcus aureus;
(c) Staphylococcus epidermidis;
(d) Propionibacterium acnes; and
(e) A Candida species selected from Candida parapsilosis, Candida glabrata, Candida tropicalis, and Candida albicans.

13. The method of claim 12, wherein the immunoassay comprises isolated polyclonal antibodies to:
(a) a Staphylococcus species, and further comprises isolated polyclonal antibodies to a Streptococcus species, and at least one of Pseudomonas aeruginosa and Enterococcus faecalis; and
(b) a Candida species, Propionibacterium acnes, and Escherichia coli.

14. Use of a kit in the method of claim 1, the kit comprising:
an enzyme configured to cleave a microbial cell wall protein;
a detergent; and
instructions directing a user to prepare a patient sample by making a solution with the enzyme and the detergent, adding the patient sample to the solution, and heating the patient sample in the solution to a temperature of at least 70°C.

15. Use according to claim 14, further comprising
(a) hyaluronidase, wherein the instructions direct a user to prepare a patient sample by adding hyaluronidase to the patient sample; and
(b) isolated polyclonal antibodies, bound to a solid substrate, isolated polyclonal antibodies configured to bind at least one microorganism selected from the group consisting of Enterococcus faecalis, Enterococcus faecium, Pseudomonas aeruginosa, Staphylococcus aureus, Staphylococcus epidermidis, Staphylococcus lugdunensis, Staphylococcus warneri, Staphylococcus capitis, Staphylococcus caprae, Streptococcus mitis, Streptococcus oralis, Streptococcus agalactiae, Streptococcus anginosus, Streptococcus gordonii, Escherichia coli, Propionibacterium acnes, Proteus mirabilis, Granulicatella adjacens, Acinetobacter baumannii, Abiotrophia defective, Corynebacterium striatum, Corynebacterium minutissimum, Parvimonas micra, Candida parapsilosis, Candida tropicalis, Candida glabrata, and Candida albicans.

## Patentansprüche

1. Verfahren zum Manipulieren einer Patientenprobe, umfassend:
Mischen eines Teils einer Patientenprobe mit einer Pufferlösung, die ein Detergens und ein Enzym umfasst, das konfiguriert ist, um ein mikrobielles Zellwandprotein zu spalten; und
Erhitzen der Mischung auf eine Temperatur von mehr als 70 °C.

2. Verfahren nach Anspruch 1, wobei das Enzym Folgendes umfasst:
(a) eine Glycosidase, eine Endopeptidase, eine Amidase oder eine Kombination davon; oder
(b) Lysostaphin, Lysozym oder eine Kombination davon.

3. Verfahren nach Anspruch 1, wobei die Pufferlösung Hyaluronidase, Trypsin oder eine Kombination davon umfasst.

4. Verfahren nach Anspruch 1, wobei das Erhitzen auf eine Temperatur von mehr als etwa 95 °C ist.

5. Verfahren nach Anspruch 1, ferner umfassend Zentrifugieren der Patientenprobe mit einer Kraft zwischen etwa 3.000 g und etwa 18.000 g, um ein Pellet zu bilden, und wobei das Mischen Mischen des Pellets mit der Pufferlösung beinhaltet.

6. Verfahren nach Anspruch 1, ferner umfassend Zentrifugieren der Mischung mit einer Kraft zwischen etwa 3.000 g und etwa 18.000 g, wobei optional das Zentrifugieren für einen Zeitraum von etwa 5 Minuten bis etwa 60 Minuten ist.

7. Verfahren nach Anspruch 1, wobei die Patientenprobe zuvor negativ auf das Vorhandensein eines Mikroorganismus getestet worden ist.

8. Verfahren nach Anspruch 1, ferner umfassend nach dem Erhitzungsschritt Durchführen eines Immuntests der Mischung, wobei der Immuntest konfiguriert ist, um einen Mikroorganismus in der Mischung zu detektieren.

9. Verfahren nach Anspruch 8, ferner umfassend Herstellen eines Immuntest-Einfangreagens oder eines Immuntest-Detektionsreagens oder von beidem, die konfiguriert sind, um spezifisch einen Mikroorganismus zu binden, der aus einem Subjekt mit einer Gelenkinfektion isoliert ist.

10. Verfahren nach Anspruch 8, wobei der Immuntest konfiguriert ist, um Folgendes zu detektieren:
(a) zumindest zwei verschiedene Gattungen von Mikroorganismen; oder
(b) zumindest zwei Mikroorganismen ausgewählt aus der Gruppe bestehend aus Enterococcus faecalis, Enterococcus faecium, Pseudomonas aeruginosa, Staphylococcus aureus, Staphylococcus epidermidis, Staphylococcus lugdunensis, Staphylococcus warneri, Staphylococcus capitis, Staphylococcus caprae, Streptococcus mitis, Streptococcus oralis, Streptococcus agalactiae, Streptococcus anginosus, Streptococcus gordonii, Escherichia coli, Propionibacterium acnes, Proteus mirabilis, Granulicatella adjacens, Acinetobacter baumannii, Abiotrophia defective, Corynebacterium striatum, Corynebacterium minutissimum, Parvimonas micra, Candida parapsilosis, Candida glabrata, Candida tropicalis und Candida albicans.

11. Verfahren nach Anspruch 8, wobei der Immuntest ein Einfangreagens, ein Detektionsreagens oder beides umfasst, die konfiguriert sind, um spezifisch einen Mikroorganismus zu binden, der aus einer biologischen Probe isoliert ist, die von einem Subjekt mit einer Gelenkinfektion erhalten wird.

12. Verfahren nach Anspruch 8, wobei der Immuntest isolierte polyklonale Antikörper gegen zumindest zwei Mikroorganismen umfasst, ausgewählt aus:
(a) Enterococcus faecalis;
(b) Staphylococcus aureus;
(c) Staphylococcus epidermidis;
(d) Propionibacterium acnes; und
(e) einer Candida-Art ausgewählt aus Candida parapsilosis, Candida glabrata, Candida tropicalis und Candida albicans.

13. Verfahren nach Anspruch 12, wobei der Immuntest isolierte polyklonale Antikörper gegen Folgendes umfasst:
(a) eine Staphylococcus-Art, und ferner isolierte polyklonale Antikörper gegen eine Streptococcus-Art und zumindest eines von Pseudomonas aeruginosa und Enterococcus faecalis umfasst; und
(b) eine Candida-Art, Propionibacterium acnes und Escherichia coli.

14. Verwendung eines Kits in dem Verfahren nach Anspruch 1, wobei das Kit Folgendes umfasst:
ein Enzym, das konfiguriert ist, um ein mikrobielles Zellwandprotein zu spalten;
ein Detergens; und
Anweisungen, die einen Benutzer anweisen, eine Patientenprobe herzustellen, indem eine Lösung mit dem Enzym und dem Detergens gemacht, die Patientenprobe der Lösung hinzufügt und die Patientenprobe in der Lösung auf eine Temperatur von zumindest 70 °C erhitzt wird.

15. Verwendung nach Anspruch 14, ferner umfassend
(a) Hyaluronidase, wobei die Anweisungen einen Benutzer anweisen, eine Patientenprobe herzustellen, indem der Patientenprobe Hyaluronidase hinzugefügt wird; und
(b) isolierte polyklonale Antikörper, gebunden an ein festes Substrat, isolierte polyklonale Antikörper, die konfiguriert sind, um zumindest einen Mikroorganismus zu binden, ausgewählt aus der Gruppe bestehend aus Enterococcus faecalis, Enterococcus faecium, Pseudomonas aeruginosa, Staphylococcus aureus, Staphylococcus epidermidis, Staphylococcus lugdunensis, Staphylococcus warneri, Staphylococcus capitis, Staphylococcus caprae, Streptococcus mitis, Streptococcus oralis, Streptococcus agalactiae, Streptococcus anginosus, Streptococcus gordonii, Escherichia coli, Propionibacterium acnes, Proteus mirabilis, Granulicatella adjacens, Acinetobacter baumannii, Abiotrophia defective, Corynebacterium striatum, Corynebacterium minutissimum, Parvimonas micra, Candida parapsilosis, Candida tropicalis, Candida glabrata und Candida albicans.

## Revendications

1. Méthode de manipulation d'un échantillon de patient, comprenant :
le mélange d'une portion d'un échantillon de patient avec une solution tampon comprenant un détergent et une enzyme conçue pour cliver une protéine de paroi cellulaire microbienne ; et
le chauffage du mélange à une température supérieure à 70°C.

2. Méthode selon la revendication 1, ladite enzyme comprenant :
(a) une glycosidase, une endopeptidase, une amidase ou une combinaison de celles-ci ; ou
(b) la lysostaphine, le lysozyme ou une combinaison de ceux-ci.

3. Méthode selon la revendication 1, ladite solution tampon comprenant de la hyaluronidase, de la trypsine ou une combinaison de celles-ci.

4. Méthode selon la revendication 1, ledit chauffage s'effectuant à une température supérieure à environ 95°C.

5. Méthode selon la revendication 1, comprenant en outre la centrifugation de l'échantillon de patient à une force comprise entre environ 3 000 g et environ 18 000 g pour former un culot, et ledit mélange comprenant le mélange du culot avec la solution tampon.

6. Méthode selon la revendication 1, comprenant en outre la centrifugation du mélange à une force comprise entre environ 3 000 g et environ 18 000 g, éventuellement ladite centrifugation s'effectuant pendant une période de temps d'environ 5 minutes à environ 60 minutes.

7. Méthode selon la revendication 1, ledit échantillon de patient ayant préalablement été testé négatif pour la présence d'un micro-organisme.

8. Méthode selon la revendication 1, comprenant en outre, après l'étape de chauffage, la réalisation d'un dosage immunologique du mélange, le dosage immunologique étant conçu pour détecter un micro-organisme dans le mélange.

9. Méthode selon la revendication 8, comprenant en outre la préparation d'un réactif de capture pour dosage immunologique, ou d'un réactif de détection pour dosage immunologique, ou les deux, conçu pour se lier spécifiquement à un micro-organisme isolé d'un sujet comportant une infection articulaire.

10. Méthode selon la revendication 8, ledit test immunologique étant conçu pour détecter :
(a) au moins deux genres différents de micro-organismes ; ou
(b) au moins deux micro-organismes choisis dans le groupe constitué par Enterococcus faecalis, Enterococcus faecium, Pseudomonas aeruginosa, Staphylococcus aureus, Staphylococcus epidermidis, Staphylococcus lugdunensis, Staphylococcus wameri, Staphylococcus capitis, Staphylococcus caprae, Streptococcus mitis, Streptococcus oralis, Streptococcus agalactiae, Streptococcus anginosus, Streptococcus gordonii, Escherichia coli, Propionibacterium acnes, Proteus mirabilis, Granulicatella adjacens, Acinetobacter baumannii, Abiotrophia defectiva, Corynebacterium striatum, Corynebacterium minutissimum, Parvimonas micra, Candida parapsilosis, Candida glabrata, Candida tropicalis et Candida albicans.

11. Méthode selon la revendication 8, ledit test immunologique comprenant un réactif de capture, un réactif de détection, ou les deux, conçu pour se lier spécifiquement à un micro-organisme isolé à partir d'un échantillon biologique prélevé chez un sujet comportant une infection articulaire.

12. Méthode selon la revendication 8, ledit test immunologique comprenant des anticorps polyclonaux isolés dirigés contre au moins deux micro-organismes choisis parmi :
(a) Enterococcus faecalis ;
(b) Staphylococcus aureus ;
(c) Staphylococcus epidermidis ;
(d) Propionibacterium acnes ; et
(e) une espèce de Candida choisie parmi Candida parapsilosis, Candida glabrata, Candida tropicalis et Candida albicans.

13. Méthode selon la revendication 12, ledit test immunologique comprenant des anticorps polyclonaux isolés contre :
(a) une espèce de Staphylococcus, et comprenant en outre des anticorps polyclonaux isolés contre une espèce de Streptococcus, et au moins un micro-organisme parmi Pseudomonas aeruginosa et Enterococcus faecalis ; et
(b) une espèce de Candida, Propionibacterium acnes et Escherichia coli.

14. Utilisation d'un kit dans la méthode selon la revendication 1, le kit comprenant :
une enzyme conçue pour cliver une protéine de paroi cellulaire microbienne ;
un détergent ; et
des instructions indiquant à un utilisateur de préparer un échantillon de patient en préparant une solution avec l'enzyme et le détergent, en ajoutant l'échantillon de patient à la solution et en chauffant l'échantillon de patient dans la solution à une température d'au moins 70°C.

15. Utilisation selon la revendication 14, comprenant en outre :
(a) l'hyaluronidase, les instructions demandant à un utilisateur de préparer un échantillon de patient en ajoutant de l'hyaluronidase à l'échantillon de patient ; et
(b) des anticorps polyclonaux isolés, liés à un substrat solide, les anticorps polyclonaux isolés étant conçus pour se lier à au moins un micro-organisme choisi dans le groupe constitué par Enterococcus faecalis, Enterococcus faecium, Pseudomonas aeruginosa, Staphylococcus aureus, Staphylococcus epidermidis, Staphylococcus lugdunensis, Staphylococcus wameri, Staphylococcus capitis, Staphylococcus caprae, Streptococcus mitis, Streptococcus oralis, Streptococcus agalactiae, Streptococcus anginosus, Streptococcus gordonii, Escherichia coli, Propionibacterium acnes, Proteus mirabilis, Granulicatella adjacens, Acinetobacter baumannii, Abiotrophia défectueux, Corynebacterium striatum, Corynebacterium minutissimum, Parvimonas micra, Candida parapsilosis, Candida tropicalis, Candida glabrata et Candida albicans.
